# EUROPÄISCHE PATENTSCHRIFT

(11) **EP 2 121 583 B1**
(45) Veröffentlichungstag und Bekanntmachung des Hinweises auf die Patenterteilung: **22.09.2010**
(21) Anmeldenummer: 08701381.9
(22) Anmeldetag: 10.01.2008
(51) Int. Cl.: C07C 237/00, C07D 231/14, C07D 277/56, C07D 307/68, C07D 333/38, C07D 401/12, C07D 403/12, C07D 405/12, C07D 409/12, C07F 7/18, A01N 43/56, A01N 47/12, A01N 47/16, A01N 47/38, A01N 55/00

(54) **HETEROAROYLSUBSTITUIERTE SERIN-AMIDE**
HETEROARYL-SUBSTITUTED SERINE AMIDES
SÉRINE-AMIDES À SUBSITUTION HÉTÉROAROYLE

(30) Priorität: 11.01.2007 EP 07100427
(43) Veröffentlichungstag der Anmeldung: 25.11.2009
(73) Patentinhaber: BASF SE, 67056 Ludwigshafen (DE)
(72) Erfinder: WITSCHEL, Matthias, 67098 Bad Duerkheim (DE); SONG, Dschun, 68167 Mannheim (DE); HUPE, Eike, 67067 Ludwigshafen (DE); NEWTON, Trevor William, 67435 Neustadt (DE); MOBERG, William Karl, 67454 Hassloch (DE); PARRA RAPADO, Liliana, 77654 Offenburg (DE); STELZER, Frank, 68309 Mannheim (DE); VESCOVI, Andrea, 68167 Mannheim (DE); REINHARD, Robert, 67117 Limburgerhof (DE); SIEVERNICH, Bernd, 67454 Hassloch (DE); GROSSMANN, Klaus, 67141 Neuhofen (DE); EHRHARDT, Thomas, 67346 Speyer (DE)
(86) Internationale Anmeldenummer: PCT/EP2008/050228
(87) Internationale Veröffentlichungsnummer: WO 2008/084073

(56) Entgegenhaltungen:
- WO-A-2005/061443
- WO-A-2006/029828
- WO-A-2006/029829
- WO-A-2006/125687
- WO-A-2007/134984

## Beschreibung

Die Erfindung betrifft heteroaroylsubstituierte Serin-Amide der Formel (I), 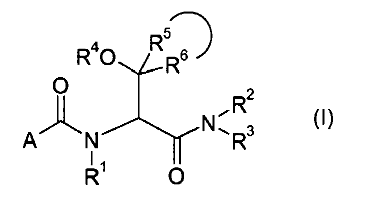 in der die Variablen die folgenden Bedeutungen haben:
- A: 5- oder 6-gliedriges Heteroaryl mit ein bis vier Stickstoffatomen, oder mit ein bis drei Stickstoffatomen und einem Sauerstoff- oder Schwefelatom, oder mit einem Sauerstoff- oder Schwefelatom, welches partiell oder vollständig halogeniert sein kann und/oder 1 bis 3 Reste aus der Gruppe Cyano, C₁-C₆-Alkyl, C₃-C₆- Cycloalkyl, C₁-C₆-Halogenalkyl, C₁-C₆-Alkoxy, C₁-C₆-Halogenalkoxy und C₁-C₆- Alkoxy-C₁-C₄-alkyl tragen kann;
- R¹, R²: Wasserstoff, Hydroxy oder C₁-C₆-Alkoxy;
- R³: C₁-C₆-Alkyl, C₁-C₄-Cyanoalkyl oder C₁-C₆-Halogenalkyl;
- R⁴: Wasserstoff, C₁-C₆-Alkyl, C₃-C₆-Cycloalkyl, C₃-C₆-Alkenyl, C₃-C₆-Alkinyl, C₃-C₆- Halogenalkenyl, C₃-C₆-Halogenalkinyl, Formyl, C₁-C₆-Alkylcarbonyl, C₃-C₆-Cyclo- alkylcarbonyl, C₂-C₆-Alkenylcarbonyl, C₂-C₆-Alkinylcarbonyl, C₁-C₆-Alkoxycarbo- nyl, C₃-C₆-Alkenyloxycarbonyl, C₃-C₆-Alkinyloxycarbonyl, Aminocarbonyl, C₁-C₆- Alkylaminocarbonyl, C₃-C₆-Alkenylaminocarbonyl, C₃-C₆-Alkinylaminocarbonyl, C₁-C₆-Alkylsulfonylaminocarbonyl, Di-(C₁-C₆-alkyl)-aminocarbonyl, N-(C₃-C₆- Alkenyl)-N-(C₁-C₆-alkyl)-aminocarbonyl, N-(C₃-C₆-Alkinyl)-N-(C₁-C₆-alkyl)- aminocarbonyl, N-(C₁-C₆-Alkoxy)-N-(C₁-C₆-alkyl)-amino-carbonyl, N-(C₃-C₆- Alkenyl)-N-(C₁-C₆-alkoxy)-aminocarbonyl, N-(C₃-C₆-Alkinyl)-N-(C₁-C₆-alkoxy)- aminocarbonyl, (C₁-C₆)Alkylaminothiocarbonyl, Di-(C₁-C₆-alkyl)- aminothiocarbonyl, (C₁-C₆-Alkyl)cyanoimino, (Amino)cyanoimino, [(C₁-C₆- Alkyl)amino]cyanoimino, [Di(C₁-C₆-Alkyl)amino]cyanoimino, C₁-C₆-Alkylcarbonyl- C₁-C₆-alkyl, C₁-C₆-Alkoxyimino-C₁-C₆-alkyl, N-(C₁-C₆-Alkylamino)-imino-C₁-C₆- alkyl, N-(Di-C₁-C₆-alkylamino)-imino-C₁-C₆-alkyl oder Tri-C₁-C₄-alkylsilyl, wobei die genannten Alkyl-, Cycloalkyl- und Alkoxyreste partiell oder voll- ständig halogeniert sein können und/oder eine bis drei der folgenden Grup- pen tragen können: Cyano, Hydroxy, C₃-C₆-Cycloalkyl, C₁-C₆-Alkoxy-C₁-C₄- alkyl, C₁-C₄-Alkoxy-C₁-C₄-alkoxy-C₁-C₄-alkyl, C₁-C₄-Alkoxy, C₁-C₄-Alkylthio, Di-(C₁-C₄-alkyl)-amino, C₁-C₄-Alkyl-C₁-C₄-alkoxycarbonyl-amino, C₁-C₄- Alkylcarbonyl, Hydroxycarbonyl, C₁-C₄-Alkoxycarbonyl, Aminocarbonyl, C₁- C₄-Alkylamino-carbonyl, Di-(C₁-C₄-alkyl)-aminocarbonyl oder C₁-C₄- Alkylcarbonyloxy; Phenyl, Phenyl-C₁-C₆-alkyl, Phenylcarbonyl, Phenylcarbonyl-C₁-C₆-alkyl, Pheno- xycarbonyl, Phenylaminocarbonyl, Phenylsulfonylaminocarbonyl, N-(C₁-C₆- Alkyl)-N-(phenyl)-aminocarbonyl, Phenyl-C₁-C₆-alkylcarbonyl, wobei der Phenylrest partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy; oder SO₂R⁷;
- R⁵ und R⁶: zusammen mit dem Kohlenstoffatom, an das sie gebunden sind, einen 3- 12-gliedrigen, gesättigten oder partiell ungesättigten Ring, der carbocyclisch ist oder 1 bis 3 N-Atome, 0 bis 3 N-Atome und 1 O- oder S-Atom, 0 bis 2 N-Atome und 2 O- oder S-Atome, 0 oder 1 N-Atom und 1 O- und 1 S-Atom, 3 O- oder S- Atome, 2 O-Atome und 1 S-Atom, oder 1 O-Atom und 2 S-Atome enthält, wobei der Ring unsubstituiert ist oder substituiert durch 1 bis 3, im Fall von Halogen auch bis zur maximal möglichen Anzahl, Substituenten aus der Gruppe Halogen, Cyano, Nitro, C₁-C₆-Alkyl, C₃-C₆-Cycloalkyl, C₃-C₆- Alkenyl, C₃-C₆-Alkinyl, C₃-C₆-Halogenalkenyl, C₃-C₆-Halogenalkinyl, Hydroxy, C₁-C₆-Alkoxy, C₃-C₆-Alkenyloxy, C₃-C₆-Alkinyloxy, Trialkylsilyloxy, Formyl, C₁-C₆-Alkyl-carbonyl, C₃-C₆-Cycloalkylcarbonyl, C₂-C₆- Alkenylcarbonyl, C₂-C₆-Alkinylcarbonyl, C₁-C₆-Alkoxycarbonyl, C₃-C₆- Alkenyloxycarbonyl, C₃-C₆-Alkinyloxycarbonyl, Aminocarbonyl, C₁-C₆- Alkylaminocarbonyl, C₃-C₆-Alkenylamino-carbonyl, C₃-C₆- Alkinylaminocarbonyl, Di-(C₁-C₆-alkyl)-aminocarbonyl, N-(C₃-C₆-Alkenyl)- N-(C₁-C₆-alkyl)-aminocarbonyl, N-(C₃-C₆-Alkinyl)-N-(C₁-C₆-alkyl)- aminocarbonyl, N-(C₁-C₆-Alkoxy)-N-(C₁-C₆-alkyl)-amino-carbonyl, N-(C₃- C₆-A)kenyl)-N-(C₁-C₆-alkoxy)-aminocarbonyl, N-(C₃-C₆-Alkinyl)-N-(C₁-C₆- alkoxy)-aminocarbonyl, Di-(C₁-C₆-alkyl)-aminothiocarbonyl, C₁-C₆- Alkoxyimino-C₁-C₆-alkyl, N-(C₁-C₆-Alkylamino)-imino-C₁-C₆-alkyl oder N- (Di-C₁-C₆-alkylamino)-imino-C₁-C₆-alkyl, Amino, Formylamino, C₁-C₆- Al- kylcarbonylamino, C₁-C₆-Alkoxycarbonylamino, C₁-C₆-Alkylamino, For- myl-C₁-C₆-Alkylamino, C₁-C₆-Alkylcarbonyl-C₁-C₆-Alkylamino, C₁-C₆- Alkoxycarbonyl-C₁-C₆-Alkylamino, Di-(C₁-C₆-alkyl)amino, Amino- Carbonylamino, C₁-C₆-Alkylamino-Carbonylamino, Di(C₁-C₆-)alkylamino- Carbonylamino, C₁-C₆-Alkylthio, C₁-C₆-Alkylsulfonyl, Alkylsolfonyloxy, Al- kylsulfonylamino, C₁-C₆-Alkylsulfinyl, C₁-C₆-Alkylsulfimino, C₁-C₆-Alkyl- C₁-C₆-Alkyisulfimino, Carbonyl, Thiocarbonyl, Imino, Alkylimino, Hydroxy- imino, Alkoxyimino, Aminoimino, Alkylaminoimino, Di-(Alkyl)aminoimino, Alkylcarbonylaminoimino, Alkylsulfonylaminoimino, C₁-C₆-Vinylidenyl, C₁- C₆-Alkoxyvinyliden, Di-C₁-C₆-Alkylaminovinyliden, wobei die genannten Alkyl-, Cycloalkyl- und Alkoxyreste partiell oder vollständig halogeniert sein können und/oder eine bis drei der folgen- den Gruppen tragen können: Cyano, Hydroxy, C₃-C₆-Cycloalkyl, C₁- C₄-Alkoxy, C₁-C₄-Alkylthio, Di-(C₁-C₄-alkyl)-amino, C₁-C₄- Alkylcarbonyl, Hydroxycarbonyl, C₁-C₄-Alkoxycarbonyl, Aminocarbo- nyl, C₁-C₄-Alkylaminocarbonyl, Di-(C₁-C₄-alkyl)haminocarbonyl oder C₁-C₄-Alkylcarbonyloxy, Phenyl, Phenyl-C₁-C₆-alkyl, Phenylcarbonyl, Phenylcarbonyl-C₁-C₆-alkyl, Phenoxycarbonyl, Phenylaminocarbonyl, Phenylsulfonylaminocarbonyl, N- (C₁-C₆-Alkyl)-N-(phenyl)-aminocarbonyl, Phenyl-C₁-C₆-alkylcarbonyl, Hete- rocyclyl, Heterocyclyl-C₁-C₆-alkyl, Heterocyclylcarbonyl, Heterocyclylsulfo- nylaminocarbonyl, Heterocyclylcarbonyl-C₁-C₆-alkyl, Heterocyclyloxycar- bonyl, Heterocyclylaminocarbonyl, N-(C₁-C₆-Alkyl)-N-(heterocyclyl)- aminocarbonyl oder Heterocyclyl-C₁-C₆-alkylcarbonyl, wobei der Phenyl- und der Heterocyclyl-Rest der 17 letztgenannten Substituenten partiell oder vollständig halogeniert sein kann und/oder eine bis drei der folgenden Gruppen tragen kann: Nitro, Cyano, C₁- C₄-Alkyl, C₁-C₄-Halogenalkyl, C₁-C₄-Alkoxy oder C₁-C₄- Halogenalkoxy; und wobei der Ring monocyclisch ist oder anelliert ist mit einem weiteren 3 bis 7- gliedrigen gesättigen, partiell ungesättigten oder vollständig ungesättigten Ring, der carbocyclisch ist oder 1 bis 3 N-Atome, 0 bis 2 N-Atome und 1 O-Atom oder S-Atom, 0 oder 1 N-Atom und 2 O-Atome oder S-Atome, 0 oder 1 N-Atom und 1 O-Atom und 1 S-Atom, 2 O-Atome und 1 S-Atom, oder 1 O-Atom und 2 S-Atome enthält, wobei der anellierte Ring unsubstituiert ist oder substituiert ist durch 1 bis 3, im Fall von Halogen auch bis zur maximal möglichen Anzahl, Substituenten aus der Gruppe Halogen, Cyano, Nitro, C₁-C₆-Alkyl, C₃-C₆-Alkenyl, C₁-C₆- Halogenalkyl, C₃-C₆-Halogenalkenyl, C₃-C₆-Halogenalkinyl, Hydroxy, C₁-C₆- Alkoxy, C₁-C₆-Halogenalkoxy und C₁-C₆-Alkylsulfonyl, und wobei der Ring unverbrückt ist oder überbrückt mit einer 1 bis 4-zähligen gesättigten oder ungesättigten Kette, die keine Heteroatome enthält oder 1 bis 2 N-Atome, 0 oder 1 N-Atom und 1 O-Atom oder 1 S-Atom, 0 oder 1 N-Atom und 2 O-Atome oder 2 S-Atome, oder 0 oder 1 N-Atom und 1 O-Atom und 1 S-Atom enthält, wobei die Brücke unsubstituiert ist oder substituiert mit 1 bis 3, im Fall von Halogen auch bis zur maximal möglichen Anzahl, Substituenten aus Grup- pe Halogen, Cyano, Nitro, C₁-C₆-Alkyl, C₃-C₆-Alkenyl, C₁-C₆-Halogenalkyl, C₃-C₆-Halogenalkenyl, C₃-C₆-Halogenalkinyl, Hydroxy, C₁-C₆-Alkoxy, C₁-C₆- Halogenalkoxy und C₁-C₆-Alkylsulfonyl;
- R⁷: C₁-C₆-Alkyl, C₁-C₆-Halogenalkyl oder Phenyl, wobei der Phenylrest partiell oder vollständig halogeniert sein kann und/oder eine bis drei der folgenden Gruppen tragen kann: C₁-C₆-Alkyl, C₁- C₆-Halogenalkyl oder C₁-C₆-Alkoxy;
sowie deren landwirtschaftlich brauchbaren Salze.

Außerdem betrifft die Erfindung Verfahren und Zwischenprodukte zur Herstellung von Verbindungen der Formel (I), Mittel, welche diese enthalten, sowie die Verwendung dieser Verbindungen oder diese enthaltende Mittel zur Schadpflanzenbekämpfung.

Fungizid wirksame thienylsubstituierte Aminosäurederivate, die in α-Position einen Alkylrest tragen, welcher gegebenenfalls durch Hydroxy oder Alkoxy substituiert sein kann, werden u.a. in EP 450 355 beschrieben.

Weiterhin sind aus der Literatur, beispielsweise aus US 5,346,907, WO 96/012499 sowie WO 02/069905 Serinderivate mit pharmazeutischer Wirksamkeit bekannt, welche in α-Position unter anderem einen Alkylrest tragen können, der gegebenenfalls durch Hydroxy oder Alkoxy substituiert sein kann.

Herbizid wirksame Serinderivate sind beispielsweise aus der WO 03/45878, WO 03/66576, WO 05/061464, WO 05/061443, WO 06/29829 und WO 06/29828 bekannt. Desweiteren beschreibt WO 2006/125687 heteroarylsubstituierte Serinderivate zur Bekämpfung uherwünschter Pflanzer.

Die bekannten Verbindungen sind jedoch in vielen Fällen nicht völlig zufriedenstlellend, beispielsweise was Aufwandmenge, Wirkspektrum, Wirkdauer, Verträglichkeit gegenüber Kulturpflanzen, Neigung zu Resistenzbildung oder ökonomische Aspekte des Herstellungsverfahrens angeht.

Es lag daher dieser Erfindung die Aufgabe zugrunde, neue, insbesondere herbizid wirksame Verbindungen mit verbesserten Eigenschaften zu finden.

Demgemäß wurden die heteroaroylsubstituierten Serin-Amide der Formel (I) sowie deren herbizide Wirkung gefunden.

Ferner wurden herbizide Mittel gefunden, welche die Verbindungen (I) enthalten und eine sehr gute herbizide Wirkung besitzen. Außerdem wurden Verfahren zur Herstellung dieser Mittel und Verfahren zur Bekämpfung von unerwünschtem Pflanzenwuchs mit den Verbindungen (I) gefunden.

Die Verbindungen der Formel (I) enthalten je nach Substitutionsmuster ein oder mehr Chiralitätszentren und liegen dann als Enantiomeren- oder Diastereomerengemische vor. Gegenstand der Erfindung sind sowohl die reinen Enantiomeren oder Diastereomeren als auch deren Gemische.

Die Verbindungen der Formel (I) können auch in Form ihrer landwirtschaftlich brauchbaren Salze vorliegen, wobei es auf die Art des Salzes in der Regel nicht ankommt. Im Allgemeinen kommen die Salze derjenigen Kationen oder die Säureadditionssalze derjenigen Säuren in Betracht, deren Kationen, beziehungsweise Anionen, die herbizide Wirkung der Verbindungen (I) nicht negativ beeinträchtigen.

Es kommen als Kationen insbesondere Ionen der Alkalimetalle, vorzugsweise Lithium, Natrium und Kalium, der Erdalkalimetalle, vorzugsweise Calcium und Magnesium, und der Übergangsmetalle, vorzugsweise Mangan, Kupfer, Zink und Eisen, sowie Ammonium, wobei hier gewünschtenfalls ein bis vier Wasserstoffatome durch C₁-C₄-Alkyl, Hydroxy-C₁-C₄-alkyl, C₁-C₄-Alkoxy-C₁-C₄-alkyl, Hydroxy-C₁-C₄-alkoxy-C₁-C₄-alkyl, Phenyl oder Benzyl ersetzt sein können, vorzugsweise Ammonium, Dimethylammonium, Diisopropylammonium, Tetramethylammonium, Tetrabutylammonium, 2-(2-Hydroxyeth-1-oxy)eth-1-ylammonium, Di-(2-hydroxyeth-1-yl)-ammonium, Trimethylbenzylammonium, des weiteren Phosphoniumionen, Sulfoniumionen, vorzugsweise Tri(C₁-C₄-alkyl)sulfonium und Sulfoxoniumionen, vorzugsweise Tri(C₁-C₄-alkyl)-sulfoxonium, in Betracht.

Anionen von brauchbaren Säureadditionsalzen sind in erster Linie Chlorid, Bromid, Fluorid, Hydrogensulfat, Sulfat, Dihydrogenphosphat, Hydrogenphosphat, Nitrat, Hydrogencarbonat, Carbonat, Hexafluorosilikat, Hexafluorophosphat, Benzoat sowie die Anionen von C₁-C₄-Alkansäuren, vorzugsweise Formiat, Acetat, Propionat und Butyrat.

"Anelliert" im Sinne der Erfindung bedeutet, dass zwei Ringen mindestens ein Atom gemeinsam ist. Damit umfasst die Definition neben kondensierten Systemen auch spirocyclisch verknüpfte Systeme.

Die für die Substituenten R¹-R⁷ oder als Reste an Phenyl-, Aryl-, Heteroaryl- oder Hetrocyclylringen genannten organischen Molekülteile stellen Sammelbegriffe für individuelle Aufzählungen der einzelnen Gruppenmitglieder dar. Sämtliche Kohlenwasserstoffketten, also alle Alkyl-, Alkylsilyl-, Alkenyl-, Alkinyl-, Cyanoalkyl-, Halogenalkyl-, Halogenalkenyl-, Halogenalkinyl-, Alkoxy-, Halogenalkoxy-, Alkoxyalkyl-, Alkoxyalkoxyalkyl, Alkylcarbonyl-, Alkenylcarbonyl-, Alkinylcarbonyl-, Alkoxycarbonyl-, Alkenyloxycarbonyl-, Alkinyloxycarbonyl-, Alkylamino-, Alkylsulfonylamino-, Halogenalkylsulfonylamino-, Alkylalkoxycarbonylamino-, Alkylaminocarbonyl-, Alkenylaminocarbonyl-, Alkinylaminocarbonyl-, Alkylsulfonylaminocarbonyl, Dialkylaminocarbonyl-, N-Alkenyl-N-alkylaminocarbonyl-, N-Alkinyl-N-alkylamino-carbonyl-, N-Alkoxy-N-alkylaminocarbonyl-, N-Alkenyl-N-alkoxyaminocarbonyl-, N-Alkinyl-N-alkoxyaminocarbonyl-, Dialkylaminothiocarbonyl-, Alkylcarbonylalkyl-, Alkoximinoalkyl-, N-(Alkylamino)-iminoalkyl, N-(Dialkylamino)-iminoalkyl, Alkylcyanoimino-, Alkylaminocyanoimino-, Dialkylaminocyanoimino-, Formylaminoalkyl-, Alkoxycarbonylaminoalkyl-, (Alkylamino)carbonyloxyalkyl-, (Alkylamino)carbonylaminoalkyl-, (Dialkylamino)carbonylaminoalkyl-, Phenylcarbonylaminoalkyl-, Phenylalkyl-, Phenylcarbonylalkyl-, N-Alkyl-N-phenylaminocarbonyl-, Phenylalkylcarbonyl-, Arylalkyl-, Heterocyclylalkyl, Heterocyclylcarbonylalkyl-, N-Alkyl-N-heterocyclylaminocarbonyl-, Heterocyclylalkylcarbonyl-, Alkylthio- und Alkylcarbonyloxy-Teile können geradkettig oder verzweigt sein.

Sofern nicht anders angegeben tragen halogenierte Substituenten vorzugsweise ein bis fünf gleiche oder verschiedene Halogenatome. Die Bedeutung Halogen steht jeweils für Fluor, Chlor, Brom oder Iod.

Ferner bedeuten beispielsweise:
- C₁-C₄-Alkyl sowie die Alkylteile von Tri-C₁-C₄-alkylsilyl, C₁-C₄-Alkylcarbonyloxy, C₁-C₄-alkyl-C₁-C₄-alkoxycarbonylamino, C₁-C₆-Alkyliminooxy-C₁-C₄-alkyl, C₁-C₄-Alkoxy-C₁-C₄-alkoxy-C₁-C₄-alkyl, C₁-C₆-Alkoxy-C₁-C₄-alkyl, C₂-C₆-Alkenyloxy-C₁-C₄-alkyl, C₂-C₆-Alkinyloxy-C₁-C₄-alkyl, C₁-C₆-Halogenalkoxy-C₁-C₄-alkyl, C₂-C₆-Halogenalkenyloxy-C₁-C₄-alkyl, C₂-C₆-Halogenalkinyloxy-C₁-C₄-alkyl, C₁-C₆-Alkoxy-C₁-C₄-alkoxy-C₁-C₄-alkyl, C₁-C₆-Alkylthio-C₁-C₄-alkyl, C₂-C₆-Alkenylthio-C₁-C₄-alkyl, C₂-C₆-Alkinylthio-C₁-C₄-alkyl, C₁-C₆-Alkylsulfinyl-C₁-C₄-alkyl, C₁-C₆-Halogenalkylsulfinyl-C₁-C₄-alkyl, - C₁-C₆-Alkylsulfonyl-C₁-C₄-alkyl , C₁-C₆-Halogenalkylsulfonyl-C₁-C₄-alkyl, Amino-C₁-C₄-alkyl, C₁-C₆-Alkylamino-C₁-C₄-alkyl, Di(C₁-C₆-Alkyl)amino-C₁-C₄-alkyl, Formylamino-C₁-C₄-alkyl, C₁-C₆-Alkoxycarbonylamino-C₁-C₄-alkyl, C₁-C₆-Alkylsulfonylamino-C₁-C₄-alkyl, C₁-C₆-Alkylsulfonyl-(C₁-C₆-alkylamino)-C₁-C₄-alkyl, Hydroxycarbonyl-C₁-C₄-alkyl, C₁-C₆-Alkoxycarbonyl-C₁-C₄-alkyl, C₁-C₆-Halogenalkoxycarbonyl-C₁-C₄-alkyl, C₁-C₆-Alkylcarbonyloxy-C₁-C₄-alkyl, Aminocarbonyl-C₁-C₄-alkyl, C₁-C₆-Alkylaminocarbonyl-C₁-C₄-alkyl, Di(C₁-C₆-Alkyl)aminocarbonyl-C₁-C₄-alkyl, [(C₁-C₆-alkyl)aminocarbonylamino]-C₁-C₄-alkyl, [Di(C₁-C₆-alkyl)aminocarbonylamino]-C₁-C₄-alkyl, C₁-C₆-Alkylcarbonylamino-C₁-C₄-alkyl, C₁-C₆-Alkylcarbonyl-(C₁-C₆-alkylamino)-C₁-C₄-alkyl, [(C₁-C₆-Alkyl)aminocarbonyloxy]C₁-C₄-alkyl, [Di(C₁-C₆-alkyl)aminocarbonyloxy]C₁-C₄-alkyl, {Di[di(C₁-C₆-alkyl)amino]carbonyloxy}C₁-C₄-alkyl, Heterocylyl-C₁-C₄-alkyl, Phenyl-C₁-C₄-alkyl, Phenylcarbonylamino-C₁-C₄-alkyl, Phenyl-C₁-C₄-alkyl, Phenylcarbonyl-C₁-C₄-alkyl, Heteroarylcarbonyl-C₁-C₄-alkyl, Heteroarylcarbonyloxy-C₁-C₄-alkyl, Heteroaryloxycarbonyl-C₁-C₄-alkyl, Heteroaryloxy-C₁-C₄-alkyl, Heteroarylthio-C₁-C₄-alkyl, Heteroarylsulfinyl-C₁-C₄-alkyl, Heteroarylsulfonyl-C₁-C₄-alkyl, und Aryl(C₁-C₄-alkyl): z.B. Methyl, Ethyl, n-Propyl, 1-Methylethyl, n-Butyl, 1-Methylpropyl, 2-Methylpropyl und 1,1-Dimethylethyl;
- C₁-C₆-Alkyl sowie die Alkylteile von C₁-C₆-Cyanoalkyl, C₁-C₆-Alkoxycarbonyl-C₁-C₆-alkyl, C₁-C₆-Alkylsulfonylamino, C₁-C₆-Alkylsulfonylaminocarbonyl, N-(C₃-C₆-Alkenyl)-N-(C₁-C₆-alkyl)-aminocarbonyl, (C₃-C₆-Alkinyl)-N-(C₁-C₆-alkyl)-aminocarbonyl, N-(C₁-C₆-Alkoxy)-N-(C₁-C₆-alkyl)-aminocarbonyl , C₁-C₆-Alkylcarbonyl-C₁-C₆-alkyl, C₁-C₆-Alkoxyimino-C₁-C₆-alkyl, N-(C₁-C₆-Alkylamino)-imino-C₁-C₆-alkyl , N-(Di-C₁-C₆-alkyl-amino)-imino-C₁-C₆-alkyl, (C₁-C₆-Alkyl)-cyanoimino, Phenyl-C₁-C₆-alkyl, Phenylcarbonyl-C₁-C₆-alkyl, N-(C₁-C₆-Alkyl)-N-phenylaminocarbonyl, Heterocyclyl-C₁-C₆-alkyl, Hetrocyclyl-carbonyl-C₁-C₆-alkyl und N-(C₁-C₆-Alkyl)-N-heterocyclylaminocarbonyl und (C₁-C₆)-Alkylam inothiocarbonyl:
   C₁-C₄-Alkyl, wie voranstehend genannt, sowie z.B. n-Pentyl, 1-Methyl-butyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethyl-butyl, 2-Ethylbutyl, 1,1,2-Trimethylpropyl, 1-Ethyl-1-methylpropyl und 1-Ethyl-3-methylpropyl;
- C₁-C₄-Alkylcarbonyl: z.B. Methylcarbonyl, Ethylcarbonyl, Propylcarbonyl, 1-Methylethylcarbonyl, Butylcarbonyl, 1-Methylpropylcarbonyl, 2-Methylpropylcärbonyl oder 1,1-Dimethylethylcarbonyl;
- C₁-C₆-Alkylcarbonyl sowie die Alkylcarbonylreste von C₁-C₆-Alkylcarbonyl-C₁-C₆-alkyl, C₁-C₆-Alkylcarbonyloxy-C₁-C₆-alkyl, C₁-C₆-Alkylcarbonylamino-C₁-C₄-alkyl, Phenyl-C₁-C₆-alkylcarbonyl und Heterocyclyl-C₁-C₆-alkylcarbonyl, C₁-C₆-Alkylcarbonyl-(C₁-C₆-alkylamino)-C₁-C₄-alkyl :
   C₁-C₄-Alkylcarbonyl, wie voranstehend genannt, sowie z.B. Pentylcarbonyl, 1-Methylbutylcarbonyl, 2-Methylbutylcarbonyl, 3-Methylbutylcarbonyl, 2,2-Dimethylpropylcarbonyl, 1-Ethylpropylcarbonyl, Hexylcarbonyl, 1,1-Dimethylpropylcarbonyl, 1,2-Dimethylpropylcarbonyl, 1-Methylpentylcarbonyl, 2-Methylpentylcarbonyl, 3-Methylpentylcarbonyl, 4-Methylpentylcarbonyl, 1,1-Dimethylbutylcarbonyl, 1,2-Dimethylbutylcarbonyl, 1,3-Dimethylbutylcarbonyl, 2,2-Dimethylbutylcarbonyl, .2,3-Dimethylbutylcarbonyl, 3,3-Dimethylbutylcarbonyl, 1-Ethylbutylcarbonyl, 2-Ethylbutylcarbonyl, 1,1,2-Trimethylpropylcarbonyl, 1,2,2-Trimethylpropylcarbonyl, 1-Ethyl-1-methylpropylcarbonyl oder 1-Ethyl-2-methylpropylcarbonyl;
- C₃-C₆-Cycloalkyl sowie die Cycloalkylteile von C₃-C₆-Cycloalkylcarbonyl: monocyclischer, gesättigter Kohlenwasserstoff mit 3 bis 6 Ringgliedern, wie Cyclopropyl, Cyclobutyl, Cyclopentyl und Cyclohexyl;
- C₃-C₆-Cycloalkenyl: z.B. 1-Cyclopropenyl, 2-Cyclopropenyl, 1-Cyclobutenyl, 2-Cyclobutenyl, 1-Cyclopentenyl, 2-Cyclopentenyl, 1,3-Cyclopentadienyl, 1,4-Cyclopentadienyl, 2,4-Cyclopentadienyl, 1-Cyclohexenyl, 2-Cyclohexenyl, 3-Cyclohexenyl, 1,3-Cyclohexadienyl, 1,4-Cyclohexadienyl, 2,5-Cyclohexadienyl;
- C₃-C₆-Alkenyl sowie die Alkenylteile von C₃-C₆-Alkenyloxycarbonyl, C₃-C₆-Alkenylaminocarbonyl, N-(C₃-C₆-Alkenyl)-N-(C₁-C₆-alkyl)aminocarbonyl und N-(C₃-C₆-Alkenyl)-N-(C₁-C₆-alkoxy)aminocarbonyl: z.B. 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl und 1-Ethyl-2-methyl-2-propenyl;
- C₂-C₆-Alkenyl sowie die Alkenylteile von C₂-C₆-Alkenylcarbonyl, C₂-C₆-Alkenyloxy-C₁-C₄-alkyl, C₂-C₆-Alkenylthio-C₁-C₄-alkyl, Phenyl-C₂-C₄-alkenyl, Heteroary-C₂-C₄-alkenyl: C₃-C₆-Alkenyl wie voranstehend genannt sowie Ethenyl;
- C₃-C₆-Alkinyl sowie die Alkinylteile von C₃-C₆-Alkinyloxycarbonyl, C₃-C₆-Alkinylaminocarbonyl, N-(C₃-C₆-Alkinyl)-N-(C₁-C₆-alkyl)-aminocarbonyl, N-(C₃-C₆-Alkinyl)-N-(C₁-C₆-alkoxyaminocarbonyl: z.B. 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl und 1-Ethyl-1-methyl-2-propinyl;
- C₂-C₆-Alkinyl sowie die Alkinylteile von C₂-C₆-Alkinylcarbonyl, C₂-C₂-Alkinyloxy-C₁-C₄-alkyl, C₂-C₆-Alkinylthio-C₁-C₄-alkyl, Phenyl-C₂-C₄-alkinyl, Heteroaryl-C₂-C₄-alkinyl: C₃-C₆-Alkinyl wie voranstehend genannt sowie Ethinyl;
- C₁-C₄-Cyanoalkyl: z.B. Cyanomethyl, 1-Cyanoeth-1-yl, 2-Cyanoeth-1-yl, 1-Cyano-prop-1-yl, 2-Cyanoprop-1-yl, 3-Cyanoprop-1-yl, 1-Cyanoprop-2-yl, 2-Cyanoprop-2-yl, 1-Cyanobut-1-yl, 2-Cyanobut-1-yl, 3-Cyanobut-1-yl, 4-Cyanobut-1-yl, 1-Cyano-but-2-yl, 2-Cyanobut-2-yl, 1-Cyanobut-3-yl, 2-Cyanobut-3-yl, 1-Cyano-2-methyl-prop-3-yl, 2-Cyano-2-methyl-prop-3-yl, 3-Cyano-2-methyl-prop-3-yl und 2-Cyanomethyl-prop-2-yl;
- C₁-C₄-Hydroxyalkyl sowie die C₁-C₄-Hydroxyalkyl-Teile von Phenyl-C₁-C₄-hydroxyalkyl, Heteroaryl-C₁-C₄-hydroxyalkyl: z.B. Hydroxymethyl, 1-Hydroxyeth-1-yl, 2-Hydroxyeth-1-yl, 1-Hydroxyprop-1-yl, 2-Hydroxyprop-1-yl, 3-Hydroxyprop-1-yl, 1-Hydroxyprop-2-yl, 2-Hydroxyprop-2-yl, 1-Hydroxybut-1-yl, 2-Hydroxybut-1-yl, 3-Hydroxybut-1-yl, 4-Hydroxybut-1-yl, 1-Hydroxybut-2-yl, 2-Hydroxybut-2-yl, 1-Hydroxybut-3-yl, 2-Hydroxybut-3-yl, 1-Hydroxy -2-methyl-prop-3-yl, 2-Hydroxy -2-methyl-prop-3-yl, 3-Hydroxy -2-methyl-prop-3-yl und 2-Hydroxymethyl-prop-2-yl, 1,2-Diydroxyethyl, 1,2-Dihydroxyprop-3-yl, 2,3-Dihydroxyprop-3-yl, 1,2-Ditiydroxy-prop-2-yl, 1,2-Diydroxybut-4-yl, 2,3-Diydroxybut-4-yl, 3,4-Diydroxybut-4-yl, 1,2-Diydroxybut-2-yl, 1,2-Diydroxybut-3-yl, 2,3-Diydroxybut-3-yl, 1,2-Dihydroxy-2-methyl-prop-3-yl, 2,3-Dihydroxy-2-methyl-prop-3-yl;
- C₁-C₆-Hydroxyalkyl: C₁-C₄-Hydroxyalkyl wie voranstehend genannt, sowie z.B. 1-Hydroxy-pent-5-yl, 2-Hydroxy-pent-5-yl, 3-Hydroxy-pent-5-yl, 4-Hydroxy-pent-5-yl, 5-Hydroxy-pent-5-yl, 1-Hydroxypent-4-yl, 2-Hydroxypen-4-tyl, 3-Hydroxypent-4-yl, 4-Hydroxypent-4-yl, 1-Hydroxy-pent-3-yl, 2-Hydroxy-pent-3-yl, 3-Hydroxy-pent-3-yl, 1-Hydroxy-2-methyl-but-3-yl, 2-Hydroxy-2-methyl-but-3-yl, 3-Hydroxy-2-methyl-but-3-yl, 1-Hydroxy-2-methyl-but-4-yl, 2-Hydroxy-2-methyl-but-4-yl, 3-Hydroxy-2-methyl-but-4-yl, 4-Hydroxy-2-methyl-but-4-yl, 1-Hydroxy-3-methyl-but-4-yl, 2-Hydroxy-3-methyl-but-4-yl, 3-Hydroxy-3-methyl-but-4-yl, 4-Hydroxy-3-methyl-but-4-yl, 1-Hydroxy-hex-6-yl, 2-Hydroxy-hex-6-yl, 3-Hydroxy-hex-6-yl, 4-Hydroxy-hex-6-yl, 5-Hydroxy-hex-6-yl, 6-Hydroxy-hex-6-yl, 1-Hydroxy-2-methyl-pent-5-yl, 2-Hydroxy-2-methyl-pent-5-yl, 3-Hydroxy-2-methyl-pent-5-yl, 4-Hydroicy-2-methyl-pent-5-yl, 5-Hydroxy-2-methyl-pent-5-yl, 1-Hydroxy-3-methyl-pent-5-yl, 2-Hydroxy-3-methyl-pent-5-yl, 3-Hydroxy-3-methyl-pent-5-yl, 4-Hydroxy-3-methyl-pent-5-yl, 5-Hydroxy-3-methyl-pent-5-yl, 1-Hydroxy-4-methyl-pent-5-yl, 2-Hydroxy-4-methyl-pent-5-yl, 3-Hydroxy-4-methyl-pent-5-yl, 4-Hydroxy-4-methyl-pent-5-yl, 5-Hydroxy-4-methyl-pent-5-yl, 1-Hydroxy-5-methyl-pent-5-yl, 2-Hydroxy-5-methyl-pent-5-yl, 3-Hydroxy-5-methyl-pent-5-yl, 4-Hydroxy-5-methyl-pent-5-yl, 5-Hydroxy-5-methyl-pent-5-yl, 1-Hydroxy-2,3-dimethyl-but-4-yl, 2-Hydroxy-2,3-dimethyl-but-4-yl, 3-Hydroxy-2,3-dimethyl-but-4-yl, 4-Hydroxy-2,3-dimethyl-but-4-yl, 1,2-Dihydroxy-pent-5-yl, 2,3-Dihydroxy-pent-5-yl, 3,4-Dihydroxy-pent-5-yl, 4,5-Dihydroxy-pent-5-yl, 1,2-Diydroxypent-4-yl, 2,3-Diydroxypent-4-yl, 3,4-Diydroxypent-4-yl, 4,5-Diydroxypent-4-yl, 1,2-Dihydroxy-pent-3-yl, 2,3-Dihydroxy-pent-3-yl, 1,2-Dihydroxy-2-methyl-but-3-yl, 2,3-Dihydroxy-2-methyl-but-3-yl, 3,4-Dihydroxy-2-methyl-but-3-yl, 2-Hydroxy-2-hdroxymethyl-but-3-yl, 1,2-Dihydroxy-2-methyl-but-4-yl, 2,3-Dihydroxy-2-methyl-but-4-yl, 3,4-Dihydroxy-2-methyl-but-4-yl, 1,2-Dihydroxy-3-methyl-but-4-yl, 2,3-Dihydroxy-3-methyl-but-4-yl, 3,4-Dihydroxy-3-methyl-but-4-yl, 3-Hydroxy-3-hydroxymethyl-but-4-yl, 1,2-Diydroxy-hex-6-yl, 2,3-Diydroxy-hex-6-yl, 3,4-Diydroxy-hex-6-yl, 4,5-Diydroxy-hex-6-yl, 5,6-Diydroxy-hex-6-yl, 1,2-Dihydroxy-2-methyl-pent-5-yl, 2,3-Dihydroxy-2-methyl-pent-5-yl, 3,4-Dihydroxy-2-methyl-pent-5-yl, 4,5-Dihydroxy-2-methyl-pent-5-yl, 2-Hydroxy-2-hdroxymethyl-pent-5-yl, 1,2-Dihydroxy-3-methyl-pent-5-yl, 2,3-Dihydroxy-3-methyl-pent-5-yl, 3,4-Dihydroxy-3-methyl-pent-5-yl, 4,5-Dihydroxy-3-methyl-pent-5-yl, 3-Hydroxy-3-hdroxymethyl-pent-5-yl, 1,2-Dihydroxy-4-methyl-pent-5-yl, 2,3-Dihydroxy-4-methyl-pent-5-yl, 3,4-Dihydroxy-4-methyl-pent-5-yl, 4,5-Dihydroxy-4-methyl-pent-5-yl, 4-Hydroxy-4-hdroxymethyl-pent-5-yl, 1,2-Dihydroxy-5-methyl-pent-5-yl, 2,3-Dihydroxy-5-methyl-pent-5-yl, 3,4-Dihydroxy-5-methyl-pent-5-yl, 4,5-Dihydroxy-5-methyl-pent-5-yl, 5-Hydroxy-5-hdroxymethyl-pent-5-yl, 1,2-Diydroxy-2,3-dimethyl-but-4-yl, 2,3-Diydroxy-2,3-dimethyl-but-4-yl, 3,4-Dihydroxy-2,3-dimethyl-but-4-yl, 2-Hydroxy-2-hydroxymethyl-3-methyl-but-4-yl, 3-Hydroxy-3-hydroxymethyl-2-methyl-but-4-yl;
- C₁-C₄-Halogenalkyl sowie die Halogenalkylteile von Phenyl-C₁-C₄-halogenalkyl, Heteroaryl-C₁-C₄-halogenalkyl: ein C₁-C₄-Alkylrest wie vorstehend genannt, der partiell oder vollständig durch Fluor, Chlor, Brom und/oder Iod substituiert ist, also z.B. Chlormethyl, Dichlormethyl, Trichlormethyl, Fluormethyl, Difluormethyl, Trifluormethyl, Chlorfluormethyl, Dichlorfluormethyl, Chlordifluormethyl, Brommethyl, lodmethyl, 2-Fluorethyl, 2-Chlorethyl, 2-Bromethyl, 2-lodethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl, Pentafluorethyl, 2-Fluorpropyl, 3-Fluorpropyl, 2,2-Difluorpropyl, 2,3-Difluorpropyl, 2-Chlorpropyl, 3-Chlorpropyl, 2,3-Dichlorpropyl, 2-Brompropyl, 3-Brompropyl, 3,3,3-Trifluorpropyl, 3,3,3-Trichlorpropyl, 2,2,3,3,3-Pentafluorpropyl, Heptafluorpropyl, 1-(Fluormethyl)-2-fluorethyl, 1-(Chlormethyl)-2-chlorethyl, 1-(Brommethyl)-2-bromethyl, 4-Fluorbutyl, 4-Chlorbutyl, 4-Brombutyl, Nonafluorbutyl, 1,1,2,2-Tetrafluorethyl und 1-Trifluormethyl-1,2,2,2-tetrafluorethyl;
- C₁-C₆-Halogenalkyl sowie die Halogenalkylteile von C₁-C₆-Halogenalkylsulfonylamino, C₁-C₆-Halogenalkyl-C₁-C₄-thioalkyl,: C₁-C₄-Halogenalkyl wie voranstehend genannt, sowie z.B. 5-Fluorpentyl, 5-Chlorpentyl, 5-Brompentyl, 5-lodpentyl, Undecafluorpentyl, 6-Fluorhexyl, 6-Chlorhexyl, 6-Bromhexyl, 6-lodhexyl und Tridecafluorhexyl;
- C₃-C₆-Halogenalkenyl: ein C₃-C₆-Alkenylrest, wie voranstehend genannt, der partiell oder vollständig durch Fluor, Chlor, Brom und/oder Iod substituiert ist, z.B. 2-Chlor-prop-2-en-1-yl, 3-Chlorprop-2-en-1-yl, 2,3-Dichlorprop-2-en-1-yl, 3,3-Dichlorprop-2-en-1-yl, 2,3,3-Trichlor-2-en-1-yl, 2,3-Dichlorbut-2-en-1-yl, 2-Bromprop-2-en-1-yl, 3-Bromprop-2-en-1-yl, 2,3-Dibromprop-2-en-1-yl, 3,3-Dibromprop-2-en-1-yl, 2,3,3-Tribrom-2-en-1-yl oder 2,3-Dibrombut-2-en-1-yl;

- C₂-C₆-Halogenalkenyl sowie die C₂-C₆-Halogenalkenyl-Teile von C₂-C₆-Halogenalkenyloxy-C₁-C₄-alkyl, C₂-C₆-Halogenalkenyl-C₁-C₄-thioalkyl, Phenyl-C₂-C₄-halogenalkenyl, Heteroaryl-C₂-C₄-halogenalkenyl: ein C₂-C₆-Alkenylrest, wie voranstehend genannt, der partiell oder vollständig durch Fluor, Chlor, Brom und/oder Iod substituiert ist, z.B. 2-Chlorvinyl, 2-Chlorallyl, 3-Chlorallyl, 2,3-Dichlorallyl, 3,3-Dichlorallyl, 2,3,3-Trichlorallyl, 2,3-Dichlorbut-2-enyl, 2-Bromvinyl, 2-Bromallyl, 3-Bromallyl, 2,3-Dibromallyl, 3,3-Dibromallyl, 2,3,3-Tribromallyl oder 2,3-Dibrombut-2-enyl;
- C₂-C₆-Cyanoalkenyl: z.B. 2-Cyanovinyl, 2-Cyanoallyl, 3-Cyanoallyl, 2,3-Dicyanoallyl, 3,3-Dicyanoallyl, 2,3,3-Tricyanoallyl, 2,3-Dicyanobut-2-enyl;
- C₂-C₆-Hydroxyalkenyl sowie die Hydroxy-Teile von Phenyl-C₁-C₄-hydroxyalkenyl, Heteroaryl-C₁-C₄-hydroxyalkenyl: z.B. 2-Hydroxyvinyl, 2-Hydroxyallyl, 3-Hydroxyallyl, 2,3-Dihydroxyallyl, 3,3-Dihydroxyallyl, 2,3,3-Trihydroxyallyl, 2,3-Dihydroxybut-2-enyl;
- C₃-C₆-Halogenalkinyl: ein C₃-C₆-Alkinylrest, wie voranstehend genannt, der partiell oder vollständig durch Fluor, Chlor, Brom und/oder Iod substituiert ist, z.B. 1,1-Difluor-prop-2-in-1-yl, 3-lod-prop-2-in-1-yl, 4-Fluorbut-2-in-1-yl, 4-Chlorbut-2in-1-yl, 1,1-Difluorbut-2-in-1-yl, 4-lodbut-3-in-1-yl, 5-Fluorpent-3-in-1-yl, 5-lodpent-4-in-1-yl, 6-Fluorhex-4-in-1-yl oder 6-lodhex-5-in-1-yl;
- C₂-C₆-Halogenalkinyl sowie die C₂-C₆-Halogenalkinyl-Teile von C₂-C₆-Halogenalkinyloxy-C₁-C₄-alkyl, C₂-C₆-Halogenalkinyl-C₁-C₄-thioalkyl, Phenyl-C₂-C₄-halogenalkinyl, Heteroaryl-C₂-C₄-halogenalkinyl: ein C₂-C₆-Alkinylrest, wie voranstehend genannt, der partiell oder vollständig durch Fluor, Chlor, Brom und/oder Iod substituiert ist, z.B. 1,1-Difluor-prop-2-in-1-yl, 3-lod-prop-2-in-1-yl, 4-Fluorbut-2-in-1-yl, 4-Chlorbut-2-in-1-yl, 1,1-Difluorbut-2-in-1-yl, 4-lodbut-3-in-1-yl, 5-Fluorpent-3-in-1-yl, 5-lodpent-4-in-1-yl, 6-Fluorhex-4-in-1-yl oder 6-lodhex-5-in-1-yl;
- C₂-C₆-Cyanoalkinyl: z.B. 1,1-Dicyano-prop-2-in-1-yl, 3-Cyano-prop-2-in-1-yl, 4-Cyano-but-2-in-1-yl, 1,1-Dicyanobut-2-in-1-yl, 4-Cyanobut-3-in-1-yl, 5-Cyanopent-3-in-1-yl, 5-Cyanopent-4-in-1-yl, 6-Cyanohex-4-in-1-yl oder 6-Cyanohex-5-in-1-yl;
- C₂-C₆-Hydroxyalkinyl sowie die Hydroxy-Teile von Phenyl-C₂-C₄-hydroxyalkinyl: z.B. 1,1-Dihydroxy-prop-2-in-1-yl, 3-Hydroxy-prop-2-in-1-yl, 4-Hydroxy-but-2-in-1-yl, 1,1-Dihydroxybut-2-in-1-yl, 4-Hydroxybut-3-in-1-yl, 5-Hydroxypent-3-in-1-yl, 5-Hydroxypent-4-in-1-yl, 6-Hydroxyhex-4-in-1-yl oder 6-Hydroxyhex-5-in-1-yl;
- C₁-C₆-Alkylsulfinyl (C₁-C₆-Alkyl-S(=O)-) sowie die C₁-C₆-Alkylsulfinyl-Teile von C₁-C₆-Alkylsulfinyl-C₁-C₄-alkyl: z.B. Methylsulfinyl, Ethylsulfinyl, Propylsulfinyl, 1-Methylethylsulfinyl, Butylsulfinyl, 1-Methylpropylsulfinyl, 2-Methylpropyl-sulfinyl, 1,1-Dimethylethylsulfinyl, Pentylsulfinyl, 1-Methylbutylsulfinyl, 2-Methylbutylsulfinyl, 3-Methylbutylsulfinyl, 2,2-Dimethylpropylsulfinyl, 1-Ethylpropyl-sulfinyl, 1,1-Dimethylpropylsulfinyl, 1,2-Dimethylpropylsulfinyl, Hexylsulfinyl, 1-Methylpentylsulfinyl, 2-Methylpentylsulfinyl, 3-Methylpentylsulfinyl, 4-Methylpentylsulfinyl, 1,1-Dimethylbutylsulfinyl, 1,2-Dimethylbutylsulfinyl, 1,3-Dimethylbutylsulfinyl, 2,2-Dimethylbutylsulfinyl, 2,3-Dimethylbutylsulfinyl, 3,3-Dimethylbutylsulfinyl, 1-Ethylbutylsulfinyl, 2-Ethylbutylsulfinyl, 1,1,2-Trimethylpropylsulfinyl, 1,2,2-Trimethylpropylsulfinyl, 1-Ethyl-1-methylpropylsulfinyl und 1-Ethyl-2-methylpropylsulfinyl;
- C₁-C₆-Halogenalkylsulfinyl sowie die C₁-C₆-Halogenalkylsulfinyl-Teile von C₁-C₆-Halogenalkylsulfinyl-C₁-C₄-alkyl: C₁-C₆-Alkylsulfinylrest wie voranstehend genannt, der partiell oder vollständig durch Fluor, Chlor, Brom und/oder Iod substituiert ist, also z.B. Fluormethylsulfinyl, Difluormethylsulfinyl, Trifluormethylsulfinyl, Chlordifluormethylsulfinyl, Bromdifluormethylsulfinyl, 2-Fluorethylsulfinyl, 2-Chlorethylsulfinyl, 2-Bromethylsulfinyl, 2-lodethylsulfinyl, 2,2-Difluorethylsulfinyl, 2,2,2-Trifluor-ethylsulfinyl, 2,2,2-Trichlorethylsulfinyl, 2-Chlor-2-fluorethylsulfinyl, 2-Chlor-2,2-difluorethylsulfinyl, 2,2-Dichlor-2-fluorethylsulfinyl, Pentafluorethylsulfinyl, 2-Fluorpropylsulfinyl, 3-Fluorpropylsulfinyl, 2-Chlorpropylsulfinyl, 3-Chlorpropyl-sulfinyl, 2-Brompropylsulfinyl, 3-Brompropylsulfinyl, 2,2-Difluorpropylsulfinyl, 2,3-Difluorpropylsulfinyl, 2,3-Dichlorpropylsulfinyl, 3,3,3-Trifluorpropylsulfinyl, 3,3,3-Trichlorpropylsulfinyl, 2,2,3,3,3-Pentafluorpropylsulfinyl, Heptafluorpropylsulfinyl, 1-(Fluormethyl)-2-fluorethylsulfinyl, 1-(Chlormethyl)-2-chlorethylsulfinyl, 1-(Brommethyl)-2-bromethylsulfinyl, 4-Fluorbutylsulfinyl, 4-Chlorbutylsulfinyl, 4-Brombutylsulfinyl, Nonafluorbutylsulfinyl, 5-Fluorpentylsulfinyl, 5-Chlorpentyl-sulfinyl, 5-Brompentylsulfinyl, 5-lodpentylsulfinyl, Undecafluorpentylsulfinyl, 6-Fluorhexylsulfinyl, 6-Chlorhexylsulfinyl, 6-Bromhexylsulfinyl, 6-lodhexylsulfinyl und Tridecafluorhexylsulfinyl;
- C₁-C₆-Alkylsulfonyl (C₁-C₆-Alkyl-S(O)₂-) sowie die C₁-C₆-Alkylsulfonyl-Teile von C₁-C₆-Alkylsulfonyl-C₁-C₄-alkyl, C₁-C₆-Alkylsulfonylamino, C₁-C₆-Alkylsulfonylamino-C₁-C₄-alkyl, C₁-C₆-Alkylsulfonyl-(C₁-C₆-alkylamino)-C₁-C₄-alkyl: z.B. Methylsulfonyl, Ethylsulfonyl, Propylsulfonyl, 1-Methylethylsulfonyl, Butylsulfonyl, 1-Methylpropylsulfonyl, 2-Methyl-propylsulfonyl, 1,1-Dimethylethylsulfonyl, Pentylsulfonyl, 1-Methylbutylsulfonyl, 2-Methylbutylsulfonyl, 3-Methylbutylsulfonyl, 1,1-Dimethylpropylsulfonyl, 1,2-Di-methylpropylsulfonyl, 2,2-Dimethylpropylsulfonyl, 1-Ethylpropylsulfonyl, Hexylsulfonyl, 1-Methylpentylsulfonyl, 2-Methylpentylsulfonyl, 3-Methylpentylsulfonyl, 4-Methylpentylsulfonyl, 1,1-Dimethylbutylsulfonyl, 1,2-Dimethylbutylsulfonyl, 1,3-Dimethylbutylsulfonyl, 2,2-Dimethylbutylsulfonyl, 2,3-Dimethylbutylsulfonyl, 3,3-Dimethylbutylsulfonyl, 1-Ethylbutylsulfonyl, 2-Ethylbutylsulfonyl, 1,1,2-Trimethylpropylsulfonyl, 1,2,2-Trimethylpropylsulfonyl, 1-Ethyl-1-methylpropylsulfonyl und 1-Ethyl-2-methylpropylsutfonyl;
- C₁-C₆-Halogenalkylsulfonyl sowie die C₁-C₆-Halogenalkylsulfonyl-Teile von C₁-C₆-Halogenalkylsulfonyl-C₁-C₄-alkyl, C₁-C₆-Halogenalkylsulfonylamino: einen C₁-C₆-Alkylsulfonylrest wie voranstehend genannt, der partiell oder vollständig durch Fluor, Chlor, Brom und/oder Iod substituiert ist, also z.B. Fluormethylsulfonyl, Difluormethylsulfonyl, Trifluormethylsulfonyl, Chlordifluormethylsulfonyl, Bromdifluormethylsulfonyl, 2-Fluorethylsulfonyl, 2-Chlorethylsulfonyl, 2-Bromethylsulfonyl, 2-lodethylsulfonyl, 2,2-Difluorethyl-sulfonyl, 2,2,2-Trifluorethylsulfonyl, 2-Chlor-2-fluorethylsulfonyl, 2-Chlor-2,2-difluorethylsulfonyl, 2,2-Dichlor-2-fluorethylsulfonyl, 2,2,2-Trichlorethylsulfonyl, Pentafluorethylsulfonyl, 2-Fluorpropylsulfonyl, 3-Fluorpropylsulfonyl, 2-Chlor-propylsulfonyl, 3-Chlorpropylsulfonyl, 2-Brompropylsulfonyl, 3-Brompropylsulfonyl, 2,2-Difluorpropylsulfonyl, 2,3-Difluorpropylsulfonyl, 2,3-Dichlorpropylsulfonyl, 3,3,3-Trifluorpropylsulfonyl, 3,3,3-Trichlorpropylsulfonyl, 2,2,3,3,3-Pentafluor-propylsulfonyl, Heptafluorpropylsulfonyl, 1-(Fluormethyl)-2-fluorethylsulfonyl, 1-(Chlormethyl)-2-chlorethylsulfonyl, 1-(Brommethyl)-2-bromethylsulfonyl, 4-Fluorbutylsulfonyl, 4-Chlorbutylsulfonyl, 4-Brombutylsulfonyl, Nonafluorbutylsulfonyl, 5-Fluorpentylsulfonyl, 5-Chlorpentylsulfonyl, 5-Brompentylsulfonyl, 5-lod-pentylsulfonyl, 6-Fluorhexylsulfonyl, 6-Bromhexylsulforiyl, 6-lodhexylsulfonyl und Dodecafluorhexylsulfonyl;
   C₁-C₄-Alkoxy sowie die Alkoxyteile von Hydroxycarbonyl-C₁-C₄-alkoxy, C₁-C₄-Alkoxycarbonyl-C₁-C₄-alkoxy, C₁-C₄-Alkoxy-C₁-C₄-alkoxy-C₁-C₄-alkyl und C₁-C₄-alkyl-C₁-C₄-alkoxycarbonylamino: z.B. Methoxy, Ethoxy, Propoxy, 1-Methyl-ethoxy, Butoxy, 1-Methylpropoxy, 2-Methylpropoxy und 1,1-Dimethylethoxy;
- C₁-C₆-Alkoxy sowie die Alkoxyteile von Hydroxycarbonyl-C₁-C₆-alkoxy, C₁-C₆-Alkoxycarbonyl-C₁-C₆-alkoxy, N-(C₁-C₆-Alkoxy)-N-(C₁-C₆-alkyl)-aminocarbonyl, N-(C₃-C₆-Alkenyl)-N-(Ci-C₆-alkoxy)-aminocarbonyl, N-(C₃-C₆-Alkinyl)-N-(C₁-C₆-alkoxy)-aminocarbonyl und C₁-C₆Alkoxyimino-C₁-C₆-alkyl: C₁-C₄-Alkoxy wie voranstehend genannt, sowie z.B. Pentoxy, 1-Methyl-butoxy, 2-Methylbutoxy, 3-Methoxylbutoxy, 1,1-Dimethyl-propoxy, 1,2-Dimethyl-propoxy, 2,2-Dimethylpropoxy, 1-Ethylpropoxy, Hexoxy, 1-Methylpentoxy, 2-Methylpentoxy, 3-Methylpentoxy, 4-Methylpentoxy, 1,1-Di-methylbutoxy,1,2-Dimethyl-butoxy, 1,3-Dimethylbutoxy, 2,2-Dimethylbutoxy, 2,3-Dimethylbutoxy, 3,3-Dimethyl-butoxy, 1-Ethylbutoxy, 2-Ethylbutoxy, 1,1,2-Tri-methylpropoxy, 1,2,2-Trimethyl-propoxy, 1-Ethyl-1-methylpropoxy und 1-Ethyl-2-methylpropoxy;
- C₁-C₄-Halogenalkoxy: ein C₁-C₄-Alkoxyrest wie voranstehend genannt, der partiell oder vollständig durch Fluor, Chlor, Brom und/oder Iod substituiert ist, also z.B. Fluormethoxy, Difluormethoxy, Trifluormethoxy, Chlordifluormethoxy, Bromdifluormethoxy, 2-Fluorethoxy, 2-Chlorethoxy, 2-Brommethoxy, 2-lodethoxy, 2,2-Difluorethoxy, 2,2,2-Trifluorethoxy, 2-Chlor-2-fluorethoxy, 2-Chlor-2,2-difluorethoxy, 2,2-Dichlor-2-fluorethoxy, 2,2,2-Trichlorethoxy, Pentafluorethoxy, 2-Fluorpropoxy, 3-Flüorpropoxy, 2-Chlorpropoxy, 3-Chlorpropoxy, 2-Brompropoxy, 3-Brompropoxy, 2,2-Difluorpropoxy, 2,3-Difluorpropoxy, 2,3-Dichlorpropoxy, 3,3,3-Trifluorpropoxy, 3,3,3-Trichlorpropoxy, 2,2,3,3,3-Pentafluorpropoxy, Heptafluorpropoxy, 1-(Fluormethyl)-2-fluorethoxy, 1-(Chlormethyl)-2-chlorethoxy, 1-(Brommethyl)-2-bromethoxy, 4-Fluorbutoxy, 4-Chlorbutoxy, 4-Brombutoxy und Nonafluorbutoxy;
- C₁-C₆-Halogenalkoxy sowie die C₁-C₆-Halogenalkoxy-Teile von C₁-C₆-Halogenalkoxy-C₁-C₄-alkyl, C₁-C₆-Halogenalkoxycarbonyl-C₁-C₄-alkyl: C₁-C₄-Halogenalkoxy wie voranstehend genannt, sowie z.B. 5-Fluorpentoxy, 5-Chlorpentoxy, 5-Brompentoxy, 5-lodpentoxy, Undecafluorpentoxy, 6-Fluorhexoxy, 6-Chlorhexoxy, 6-Bromhexoxy, 6-lodhexoxy und Dodecafluorhexoxy;
- C₁-C₆-Alkoxy-C₁-C₄-alkyl sowie die C₁-C₆-Alkoxy-C₁-C₄-alkyl-Teile von C₁-C₆-Alkoxy-C₁-C₄-alkoxy-C₁-C₄-alkyl: durch C₁-C₆-Alkoxy wie vorstehend genannt substituiertes C₁-C₄-Alkyl, also z.B. für Methoxymethyl, Ethoxymethyl, Propoxymethyl, (1-Methylethoxy)methyl, Butoxymethyl, (1-Methylpropoxy)methyl, (2-Methyl-propoxy)methyl, (1,1-Dimethylethoxy)methyl, 2-(Methoxy)ethyl, 2-(Ethoxy)ethyl, 2-(Propoxy)ethyl, 2-(1-Methylethoxy)ethyl, 2-(Butoxy)ethyl, 2-(1-Methylpropoxy)-ethyl, 2-(2-Methylpropoxy)ethyl, 2-(1,1-Dimethylethoxy)ethyl, 2-(Methoxy)-propyl, 2-(Ethoxy)propyl, 2-(Propoxy)propyl, 2-(1-Methylethoxy)propyl, 2-(Butoxy)propyl, 2-(1-Methylpropoxy)propyl, 2-(2-Methylpropoxy)propyl, 2-(1,1-Dimethylethoxy)propyl, 3-(Methoxy)propyl, 3-(Ethoxy)-propyl, 3-(Propoxy)propyl, 3-(1-Methylethoxy)-propyl, 3-(Butoxy)propyl, 3-(1-Methylpropoxy)propyl, 3-(2-Methylpropoxy)propyl, 3-(1,1-Dimethylethoxy)propyl, 2-(Methoxy)-butyl, 2-(Ethoxy)butyl, 2-(Propoxy)-butyl, 2-(1-Methylethoxy)butyl, 2-(Butoxy)butyl, 2-(1-Methylpropoxy)butyl, 2-(2-Methylpropoxy)butyl, 2-(1,1-Dimethylethoxy)butyl, 3-(Methoxy)butyl, 3-(Ethoxy)-butyl, 3-(Propoxy)butyl, 3-(1-Methylethoxy)butyl, 3-(Butoxy)-butyl, 3-(1-Methyl-propoxy)-butyl, 3-(2-Methylpropoxy)butyl, 3-(1,1-Dimethylethoxy)butyl, 4-(Methoxy)-butyl, 4-(Ethoxy)butyl, 4-(Propoxy)butyl, 4-(1-Methylethoxy)butyl, 4-(Butoxy)butyl, 4-(1-Methylpropoxy)butyl, 4-(2-Methylpropoxy)butyl und 4-(1,1-Dimethylethoxy)-butyl;
- C₁-C₄-Alkoxycarbonyl sowie die Alkoxycarbonylteile von C₁-C₄-Alkoxycarbonyl-C₁-C₄-alkoxy, C₁-C₄-Alkoxy-C₁-C₄-alkoxycarbonyl und Di-(C₁-C₄-alkyl)-amino-C₁-C₄-alkoxycarbonyl: z.B. Methoxycarbonyl, Ethoxycarbonyl, Propoxycarbonyl, 1-Methylethoxycarbonyl, Butoxycarbonyl, 1-Methylpropoxycarbonyl, 2-Methylpropoxycarbonyl oder 1,1-Dimethylethoxycarbonyl;
- C₁-C₆-Alkoxycarbonyl sowie die Alkoxycarbonylteile von C₁-C₆-Alkoxycarbonyl-C₁-C₆-alkoxy und C₁-C₆-Alkoxycarbonylamino-C₁-C₄-alkyl: C₁-C₄-Alkoxycarbonyl, wie voranstehend genannt, sowie z.B. Pentoxycarbonyl, 1-Methylbutoxycarbonyl, 2-Methylbutoxycarbonyl, 3-Methyl-butoxycarbonyl, 2,2-Dimethylpropoxycarbonyl, 1-Ethylpropoxycarbonyl, Hexoxy-carbonyl, 1,1-Dimethylpropoxycarbonyl, 1,2-Dimethylpropoxycarbonyl, 1-Methyl-pentoxycarbonyl, 2-Methylpentoxycarbonyl, 3-Methylpentoxycarbonyl, 4-Methyl-pentoxycarbonyl, 1,1-Dimethylbutoxycarbonyl, 1,2-Dimethylbutoxycarbonyl, 1,3-Dimethylbutoxycarbonyl, 2,2-Dimethylbutoxycarbonyl, 2,3-Dimethylbutoxycarbonyl, 3,3-Dimethylbutoxycarbonyl, 1-Ethylbutoxycarbonyl, 2-Ethylbutoxycarbonyl; 1,1,2-Trimethylpropoxycarbonyl, 1,2,2-Trimethylpropoxycarbonyl, 1-Ethyl-1-methyl-propoxycarbonyl oder 1-Ethyl-2-methylpropoxycarbonyl;
- C₁-C₄-Alkylthio sowie die C₁-C₄-Alkylthio-Teile von C₁-C₆-Halogenalkyl-C₁-C₄-thioalkyl, C₂-C₆-Halogenalkenyl-C₁-C₄-thioalkyl, C₂-C₆-Halogenalkinyl-C₁-C₄-thioalkyl: z.B. Methylthio, Ethylthio, Propylthio, 1-Methylethylthio, Butylthio, 1-Methylpropylthio, 2-Methylpropylthio und 1,1-Dimethylethylthio;
- C₁-C₆-Alkylthio sowie die C₁-C₆-Alkylthio-Teile von C₁-C₆-Alkylthio-C₁-C₄-alkyl: C₁-C₄-Alkylthio wie voranstehend genannt, sowie z.B. Pentylthio, 1-Methylbutylthio, 2-Methylbutylthio; 3-Methylbutylthio, 2,2-Dimethylpropylthio, 1-Ethylpropylthio, Hexylthio, 1,1-Dimethylpropylthio, 1,2-Dimethylpropylthio, 1-Methylpentylthio, 2-Methylpentylthio, 3-Methylpentylthio, 4-Methylpentylthio, 1,1-Dimethylbutylthio, 1,2-Dimethylbutylthio, 1,3-Dimethylbutylthio, 2,2-Dimethylbutyl-thio, 2,3-Dimethylbutylthio, 3,3-Dimethylbutylthio, 1-Ethylbutylthio, 2-Ethylbutyl-thio, 1,1,2-Trimethylpropylthio, 1,2,2-Trimethylpropylthio, 1-Ethyl-1-methylpropyl-thio.und 1-Ethyl-2-methylpropylthio;
- C₁-C₆-Alkylamino sowie die C₁-C₆-Alkylaminoreste von N(C₁-C₆-Alkylamino)imino-C₁-C₆-alkyl, C₁-C₆-Alkylamino-C₁-C₄-alkyl, C₁-C₆-Alkylsulfonyl-(C₁-C₆-alkylamino)-C₁-C₄-alkyl, C₁-C₆-Alkylcarbonyl-(C₁-C₆-alkylamino)-C₁-C₄-alkyl und [(C₁-C₆-Alkyl)amino]cyanoimino: z.B. Methylamino, Ethylamino, Propylamino, 1-Methylethylamino, Butylamino, 1-Methylpropylamino, 2-Methylpropylamino, 1,1-Dimethylethylamino, Pentylamino, 1-Methylbutylamino, 2-Methylbutylamino, 3-Methylbutylamino, 2,2-Dimethylpropylamino, 1-Ethylpropylamino, Hexylamino, 1,1-Dimethylpropylamino, 1,2-Dimethylpropylamino, 1-Methylpentylamino, 2-Methylpentylamino, 3-Methyl-pentylamino, 4-Methylpentylamino, 1,1-Dimethylbutylamino, 1,2-Dimethylbutyl-amino, 1,3-Dimethylbutylamino, 2,2-Dimethylbutylamino, 2,3-Dimethylbutylamino, 3,3-Dimethylbutylamino, 1-Ethylbutylamino, 2-Ethylbutylamino, 1,1,2-Trimethyl-propylamino, 1,2,2-Trimethylpropylamino, 1-Ethyl-1-methylpropylamino oder 1-Ethyl-2-methylpropylamino;
- Di(C₁-C₄-alkyl)amino: z.B. N,N-Dimethylamino, N,N-Diethylamino, N,N-Dipropylamino, N,N-Di-(1-methylethyl)-amino, N,N-Dibutylamino, N,N-Di-(1-methylpropyl)amino, N,N-Di-(2-methylpropyl)-amino, N,N-Di-(1,1-dimethylethyl)-amino, N-Ethyl-N-methylamino, N-Methyl-N-propylamino, N-Methyl-N-(1-methylethyl)amino, N-Butyl-N-methylamino, N-Methyl-N-(1-methylpropyl)amino, N-Methyl-N-(2-methylpropyl)amino, N-(1,1-Dimethyl-ethyl)-N-methylamino, N-Ethyl-N-propylamino, N-Ethyl-N-(1-methylethyl)amino, N-Butyl-N-ethylamino, N-Ethyl-N-(1-methylpropyl)amino, N-Ethyl-N-(2-methylpropyl)-amino, N-Ethyl-N-(1,1-dimethylethyl)amino, N-(1-Methylethyl)-N-propylamino, N-Butyl-N-propylamino, N-(1-Methylpropyl)-N-propylamino, N-(2-Methylpropyl)-N-propylamino, N-(1,1-Dimethy)-ethyl)-N-propylamino, N-Butyl-N-(1-methylethyl-)amino, N-(1-Methylethyl)-N-(1-methylpropyl)amino, N-(1-Methylethyl)-N-(2-methyl-propyl)amino, N-(1,1-Dimethylethyl)-N-(1-methylethyl)amino, N-Butyl-N-(1-methylpropyl)amino, N-Butyl-N-(2-methylpropyl)amino, N-Butyl-N-(1,1-dimethyl-ethyl)amino, N-(1-Methylpropyl)-N-(2-methylpropyl)amino, N-(1,1-Dimethylethyl)-N-(1-methylpropyl)amino und N-(1,1-Dimethylethyl)-N-(2-methylpropyl)amino;
- Di(C₁-C₆-alkyl)amino sowie die Dialkylaminoreste von N-(Di-C₁-C₆-alkylamino)-imino-C₁-C₆-alkyl, Di(C₁-C₆-alkyl)amino-C₁-C₄-alkyl, [Di(C₁-C₆-alkyl)aminocarbonyloxy]-C₁-C₄-alkyl, {Di[di(C₁-C₆-alkyl)amino]carbonyloxy}-C₁-C₄-alkyl und [Di(C₁-C₆-alkyl)amino]cyanoimino: Di(C₁-C₄-alkyl)amino wie voranstehend genannt sowie: z.B. N,N-Dipentylamino, N,N-Dihexylamino, N-Methyl-N-pentylamino, N-Ethyl-N-pentylamino, N-Methyl-N-hexylamino und N-Ethyl-N-hexylamino;
- (C₁-C₄-Alkylamino)carbonyl sowie die (C₁-C₄-Alkylamino)carbonyl-Teile von (C₁-C₄-Alkylamino)carbonylamino: z.B. Methylaminocarbonyl, Ethylaminocarbonyl, Propylaminocarbonyl, 1-Methylethylaminocarbonyl, Butylaminocarbonyl, 1-Methylpropylaminocarbonyl, 2-Methylpropylaminocarbonyl oder 1,1-Dimethylethylaminocarbonyl;
- (C₁-C₄-Alkylamino)thiocarbonyl sowie die (C₁-C₄-Alkylamino)thiocarbonyl-Teile von (C₁-C₄-Alkylamino)thiocarbonylamino: z.B. Methylaminothiocarbonyl, Ethylaminothiocarbonyl, Propylaminothiocarbonyl, 1-Methylethylaminothiocarbonyl, Butylaminothiocarbonyl, 1-Methylpropylaminothiocarbonyl, 2-Methylpropylaminothiocarbonyl oder 1,1-Dimethylethylaminothiocarbonyl;
- Di(C₁-C₄-alkyl)aminocarbonyl sowie die Di(C₁-C₄-alkyl)aminocarbonyl-Teile von Di(C₁-C₄-alkyl)aminocarbonylamino: z.B. N,N-Dimethylaminocarbonyl, N,N-Diethylaminocarbonyl, N,N-Di-(1-methylethyl)aminocarbonyl, N,N-Dipropylaminocarbonyl, N,N-Dibutylaminocarbonyl, N,N-Di-(1-methylpropyl)aminocarbonyl, N,N-Di-(2-methylpropyl)aminocarbonyl, N,N-Di-(1,1-dimethylethyl)aminocarbonyl, N-Ethyl-N-methylaminocarbonyl, N-Methyl-N-propylaminocarbonyl, N-Methyl-N-(1-methylethyl)aminocarbonyl, N-Butyl-N-methylaminocarbonyl, N-Methyl-N-(1-methylpropyl)aminocarbonyl, N-Methyl-N-(2-methylpropyl)aminocarbonyl, N-(1,1-Dimethylethyl)-N-methylaminocarbonyl, N-Ethyl-N-propylaminocarbonyl, N-Ethyl-N-(1-methylethyl)aminocarbonyl, N-Butyl-N-ethylaminocarbonyl, N-Ethyl-N-(1-methylpropyl)aminocarbonyl, N-Ethyl-N-(2-methylpropyl)aminocarbonyl, N-Ethyl-N-(1,1-dimethylethyl)aminocarbonyl, N-(1-Methylethyl)-N-propylaminocarbonyl, N-Butyl-N-propylaminocarbonyl, N-(1-Methylpropyl)-N-propylaminocarbonyl, N-(2-Methylpropyl)-N-propylaminocarbonyl, N-(1,1-Dimethylethyl)-N-propylaminocarbonyl, N-Butyl-N-(1-methylethyl)aminocarbonyl, N-(1-Methylethyl)-N-(1-methylpropyl)-amino-carbonyl, N-(1-Methylethyl)-N-(2-methylpropyl)aminocarbonyl, N-(1,1-Dimethylethyl)-N-(1-methylethyl)aminocarbonyl, N-Butyl-N-(1-methylpropyl)-aminocarbonyl, N-Butyl-N-(2-methylpropyl)aminocarbonyl, N-Butyl-N-(1,1-dimethyl-ethyl)amino-carbonyl, N-(1-Methylpropyl)-N-(2-methylpropyl)aminocarbonyl, N-(1,1-Dimethylethyl)-N-(1-methylpropyl)aminocarbonyl oder N-(1,1-Dimethylethyl)-N-(2-methylpropyl)aminocarbonyl;
- (C₁-C₆-Alkylamino)carbonyl sowie die (C₁-C₆-Alkylamino)carbonyl-Teile von (C₁-C₆-Alkylamino)carbonylamino, (C₁-C₆-Alkylamino)carbonyloxy-C₁-C₄-alkyl, C₁-C₆-Alkylaminocarbonyl-C₁-C₄-alkyl und [(C₁-C₆-Alkyl)aminocarbonylamino]-C₁-C₄-alkyl: (C₁-C₄-Alkylamino)carbonyl, wie voranstehend genannt, sowie z.B. Pentylaminocarbonyl, 1-Methylbutylaminocarbonyl, 2-Methyl-butylaminocarbonyl, 3-Methyl-butylaminocarbonyl, 2,2-Dimethylpropylamino-carbonyl, 1-Ethylpropylaminocarbonyl, Hexylaminocarbonyl, 1,1-Dimethylpropyl-aminocarbonyl, 1,2-Dimethylpropylaminocarbonyl, 1-Methylpentylaminocarbonyl, 2-Methylpentylaminocarbonyl, 3-Methylpentylaminocarbonyl, 4-Methylpentylamino-carbonyl, 1,1-Dimethylbutylaminocarbonyl, 1,2-Dimethylbutylaminocarbonyl, 1,3-Dimethylbutylaminocarbonyl, 2,2-Dimethylbutylaminocarbonyl, 2,3-Dimethylbutyl-aminocarbonyl, 3,3-Dimethylbutylaminocarbonyl, 1-Ethylbutylaminocarbonyl, 2-Ethylbutylaminocarbonyl, 1,1,2-Trimethylpropylaminocarbonyl, 1,2,2-Trimethyl-propylaminocarbonyl, 1-Ethyl-1-methylpropylaminocarbonyl oder 1-Ethyl-2-methylpropylaminocarbonyl;
- Di(C₁-C₆-alkyl)aminocarbonyl sowie die Di(C₁-C₆-alkyl)aminocarbonyl-Teile von Di(C₁-C₆-alkyl)aminocarbonylamino, Di(C₁-C₆-alkyl)aminocarbonyl-C₁-C₄-alkyl und [Di(C₁-C₆-alkyl)aminocarbonylamino]-C₁-C₄-alkyl: Di(C₁-C₄-alkyl)aminocarbonyl, wie voranstehend genannt, sowie z.B. N-Methyl-N-pentylaminocarbonyl, N-Methyl-N-(1-methylbutyl)-aminocarbonyl, N-Methyl-N-(2-methylbutyl)-aminocarbonyl, N-Methyl-N-(3-methylbutyl)-aminocarbonyl, N-Methyl-N-(2,2-dimethylpropyl)-amino-carbonyl, N-Methyl-N-(1-ethylpropyl)-aminocarbonyl, N-Methyl-N-hexylaminocarbonyl, N-Methyl-N-(1,1-dimethylpropyl)-aminocarbonyl, N-Methyl-N-(1,2-dimethylpropyl)-aminocarbonyl, N-Methyl-N-(1-methylpentyl)-aminocarbonyl, N-Methyl-N-(2-methylpentyl)-aminocarbonyl, N-Methyl-N-(3-methylpentyl)-amino-carbonyl, N-Methyl-N-(4-methylpentyl)-aminocarbonyl, N-Methyl-N-(1,1-dimethylbutyl)-aminocarbonyl, N-Methyl-N-(1,2-dimethylbutyl)-aminocarbonyl, N-Methyl-N-(1,3-dimethylbutyl)-aminocarbonyl, N-Methyl-N-(2,2-dimethylbutyl)-aminocarbonyl, N-Methyl-N-(2,3-dimethylbutyl)- amino-carbonyl, N-Methyl-N-(3,3-dimethylbutyl)-aminocarbonyl, N-Methyl-N-(1-ethyl-butyl)-aminocarbonyl, N-Methyl-N-(2-ethylbutyl)-aminocarbonyl, N-Methyl-N-(1,1,2-trimethylpropyl)-amino-carbonyl, N-Methyl-N-(1,2,2-trimethylpropyl)-aminocarbonyl, N-Methyl-N-(1-ethyl-1-methylpropyl)-aminocarbonyl, N-Methyl-N-(1-ethyl-2-methylpropyl)-amino-carbonyl, N-Ethyl-N-pentylaminocarbonyl, N-Ethyl-N-(1-methylbutyl)-aminocarbonyl, N-Ethyl-N-(2-methylbutyl)-aminocarbonyl, N-Ethyl-N-(3-methylbutyl)-aminocarbonyl, N-Ethyl-N-(2,2-dimethylpropyl)-aminocarbonyl, N-Ethyl-N-(1-ethylpropyl)-aminocarbonyl, N-Ethyl-N-hexylaminocarbonyl, N-Ethyl-N-(1,1-dimethylpropyl)-aminocarbonyl, N-Ethyl-N-(1,2-dimethylpropyl)-aminocarbonyl, N-Ethyl-N-(1-methylpentyl)-aminocarbonyl, N-Ethyl-N-(2-methylpentyl)-aminocarbonyl, N-Ethyl-N-(3-methylpentyl)-aminocarbonyl, N-Ethyl-N-(4-methylpentyl)-aminocarbonyl, N-Ethyl-N-(1,1-dimethylbutyl)-aminocarbonyl, N-Ethyl-N-(1,2-dimethylbutyl)-aminocarbonyl, N-Ethyl-N-(1,3-dimethylbutyl)-aminocarbonyl, N-Ethyl-N-(2,2-dimethylbutyl)- aminocarbonyl, N-Ethyl-N-(2,3-dimethylbutyl)-aminocarbonyl, N-Ethyl-N-(3,3-dimethylbutyl)-aminocarbonyl, N-Ethyl-N-(1-ethylbutyl)-amino-carbonyl, N-Ethyl-N-(2-ethylbutyl)-aminocarbonyl, N-Ethyl-N-(1,1,2-trimethyl-propyl)-aminocarbonyl, N-Ethyl-N-(1,2,2-trimethylpropyl)-aminocarbonyl, N-Ethyl-N- (1-ethyl-1-methylpropyl)-aminocarbonyl, N-Ethyl-N-(1-ethyl-2-methylpropyl)-aminocarbonyl, N-Propyl-N-pentylaminocarbonyl, N-Butyl-N-pentylaminocarbonyl, N,N-Dipentylaminocarbonyl, N-Propyl-N-hexyl-aminocarbonyl, N-Butyl-N-hexylaminocarbonyl, N-Pentyl-N-hexylaminocarbonyl oder N,N-Dihexylamino-carbonyl;
- Di(C₁-C₆-alkyl)aminothiocarbonyl: z.B. N,N-Dimethylaminothiocarbonyl, N,N-Diethylaminothiocarbonyl, N,N-Di-(1-methylethyl)aminothiocarbonyl, N,N-Dipropyl-aminothiocarbonyl, N,N-Dibutylaminothiocarbonyl, N,N-Di-(1-methylpropyl)-aminothiocarbonyl, N,N-Di-(2-methylpropyl)-aminothiocarbonyl, N,N-Di-(1,1-dimethylethyl)-aminothiocarbonyl, N-Ethyl-N-methylaminothiocarbonyl, N-Methyl-N-propylaminothiocarbonyl, N-Methyl-N-(1-methylethyl)-aminothiocarbonyl, N-Butyl-N-methylaminothiocarbonyl, N-Methyl-N-(1-methylpropyl)-aminothiocarbonyl, N-Methyl-N-(2-methylpropyl)aminothiocarbonyl, N-(1,1-Dimethylethyl)-N-methylaminothiocarbonyl, N-Ethyl-N-propylaminothiocarbonyl, N-Ethyl-N-(1-methylethyl)-aminothiocarbonyl, N-Butyl-N-ethylaminothiocarbonyl, N-Ethyl-N-(1-methylpropyl)-aminothiocarbonyl, N-Ethyl-N-(2-methylpropyl)-aminothiocarbonyl, N-Ethyl-N-(1,1-dimethylethyl)-aminothiocarbonyl, N-(1-Methylethyl)-N-propylaminothiocarbonyl, N-Butyl-N-propylaminothiocarbonyl, N-(1-Methylpropyl)-N-propylaminothiocarbonyl, N-(2-Methylpropyl)-N-propylamino-thiocarbonyl, N-(1,1-Dimethylethyl)-N-propylaminothiocarbonyl, N-Butyl-N-(1-methylethyl)-aminothiocarbonyl, N-(1-Methylethyl)-N-(1-methylpropyl)-aminothiocarbonyl, N-(1-Methylethyl)-N-(2-methylpropyl)-aminothiocarbonyl, N-(1,1-Dimethylethyl)-N-(1-methylethyl)-aminothiocarbonyl, N-Butyl-N-(1-methylpropyl)-aminothiocarbonyl, N-Butyl-N-(2-methylpropyl)-aminothiocarbonyl, N-Butyl-N-(1,1-dimethylethyl)-aminothiocarbonyl, N-(1-Methylpropyl)-N-(2-methylpropyl)-aminothiocarbonyl, N-(1,1-Dimethylethyl)-N-(1-methylpropyl)-aminothiocarbonyl, N-(1,1-Dimethylethyl)-N-(2-methylpropyl)-aminothiocarbonyl, N-Methyl-N-pentylaminothiocarbonyl, N-Methyl-N-(1-methylbutyl)-aminothiocarbonyl, N-Methyl-N-(2-methylbutyl)-aminothiocarbonyl, N-Methyl-N-(3-methylbutyl)-aminothiocarbonyl, N-Methyl-N-(2,2-dimethylpropyl)-aminothiocarbonyl, N-Methyl-N-(1-ethylpropyl)-aminothiocarbonyl, N-Methyl-N-hexylaminothiocarbonyl, N-Methyl-N- (1,1-dimethylpropyl)-aminothiocarbonyl, N-Methyl-N-(1,2-dimethylpropyl)-aminothiocarbonyl, N-Methyl-N-(1-methylpentyl)-aminothiocarbonyl, N-Methyl-N-(2-methylpentyl)-aminothiocarbonyl, N-Methyl-N-(3-methylpentyl)-aminothiocarbonyl, N-Methyl-N-(4-methylpentyl)-aminothiocarbonyl, N-Methyl-N- (1,1-dimethylbutyl)-aminothiocarbonyl, N-Methyl-N-(1,2-dimethylbutyl)-aminothiocarbonyl, N-Methyl-N-(1,3-dimethylbutyl)-aminothiocarbonyl, N-Methyl-N-(2,2-dimethylbutyl)-aminothiocarbonyl, N-Methyl-N-(2,3-dimethylbutyl)-aminothiocarbonyl, N-Methyl-N-(3,3-dimethylbutyl)-aminothiocarbonyl, N-Methyl-N-(1-ethylbutyl)-aminothiocarbonyl, N-Methyl-N-(2-ethylbutyl)-aminothiocarbonyl, N-Methyl-N-ethyl-N-(1,1,2-trimethylpropyl)-aminothiocarbonyl, N-Methyl-N-(1,2,2-trimethylpropyl)-aminothiocarbonyl, N-Methyl-N-(1-ethyl-1-methylpropyl)-aminothiocarbonyl, N-Methyl-N-(1-ethyl-2-methylpropyl)-aminothiocarbonyl, N-Ethyl-N-pentyl-aminothiocarbonyl, N-Ethyl-N-(1-methylbutyl)-aminothiocarbonyl, N-Ethyl-N-(2-methylbutyl)-aminothiocarbonyl, N-Ethyl-N-(3-methylbutyl)- aminothiocarbonyl, N-Ethyl-N-(2,2-dimethylpropyl)-aminothiocarbonyl, N-Ethyl-N-(1-ethylpropyl)-aminothiocarbonyl, N-Ethyl-N-hexylaminothiocarbonyl, N-Ethyl-N-(1,1-dimethyl-propyl)-aminothiocarbonyl, N-Ethyl-N-(1,2-dimethylpropyl)-amino-thiocarbonyl, N-Ethyl-N-(1-methylpentyl)-aminothiocarbonyl, N-Ethyl-N-(2-methylpentyl)-aminothiocarbonyl, N-Ethyl-N-(3-methylpentyl)-aminothiocarbonyl, N-Ethyl-N-(4-methylpentyl)-aminothiocarbonyl, N-Ethyl-N-(1,1-dimethylbutyl)-amino-thiocarbonyl, N-Ethyl-N-(1,2-dimethylbutyl)-aminothiocarbonyl, N-Ethyl-N-(1,3-dimethylbutyl)-aminothiocarbonyl, N-Ethyl-N-(2,2-dimethylbutyl)-aminothio-carbonyl, N-Ethyl-N-(2,3-dimethylbutyl)-aminothiocarbonyl, N-Ethyl-N-(3,3-dimethylbutyl)-aminothiocarbonyl, N-Ethyl-N-(1-ethylbutyl)-aminothiocarbonyl, N-Ethyl-N-(2-ethylbutyl)-aminothiocarbonyl, N-Ethyl-N-(1,1,2-trimethylpropyl)-aminothiocarbonyl, N-Ethyl-N-(1,2,2-trimethylpropyl)-aminothiocarbonyl, N-Ethyl-N-(1-ethyl-1-methylpropyl)-aminothiocarbonyl, N-Ethyl-N-(1-ethyl-2-methylpropyl)-aminothiocarbonyl, N-Propyl-N-pentylaminothiocarbonyl, N-Butyl-N-pentylaminothiocarbonyl, N,N-Dipentylaminothiocarbonyl, N-Propyl-N-hexyl-aminothiocarbonyl, N-Butyl-N-hexylaminothiocarbonyl, N-Pentyl-N-hexyl-aminothiocarbonyl oder N,N-Dihexylaminothiocarbonyl;
- drei- bis sechsgliedriges Heterocyclyl sowie die drei- bis sechsgliedrigen Heterocyclyl-Teile von drei- bis sechsgliedriges Heterocyclyl-C₁-C₄-alkyl: monocyclische, gesättigte oder partiell ungesättigte Kohlenwasserstoffe mit drei bis sechs Ringgliedern wie voranstehend genannt, welche neben Kohlenstoffatomen ein bis vier Stickstoffatome, oder ein bis drei Stickstoffatome und ein Sauerstoff- oder Schwefelatom, oder ein bis drei Sauerstoffatome, oder ein bis drei Schwefelatome enthalten können, und welche über ein C-Atom oder ein N-Atom verknüpft sein können,
   z.B. 2-Oxiranyl, 2-Oxetanyl, 3-Oxetanyl, 2-Aziridinyl, 3-Thiethanyl, 1-Azetidinyl, 2-Azetidinyl,
   z.B. 2-Tetrahydrofuranyl, 3-Tetrahydrofuranyl, 2-Tetrahydrothienyl, 3-Tetrahydrothienyl, 2-Pyrrolidinyl, 3-Pyrrolidinyl, 3-Isoxazolidinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 1,2,3,4-Tetrazolidin-5-yl,
   z.B. 1-Pyrrolidinyl, 2-Isothiazolidinyl, 2-Isothiazolidinyl, 1-Pyrazolidinyl, 3-Oxazolidinyl, 3-Thiazolidinyl, 1-Imidazolidinyl, 1,2,4-Triazolidin-1-yl, 1,2,4-Oxadiazolidin-2-yl, 1,2,4-Oxadiazolidin-4-yl, 1,2,4-Thiadiazolidin-2-yl, 1,2,4-Thiadiazolidin-4-yl, 1,2,3,4-Tetrazolidin-1-yl,
   z.B. 2,3-Dihydrofur-2-yl, 2,3-Dihydrofur-3-yl, 2,4-Dihydrofur-2-yl, 2,4-Dihydrofur-3-yl, 2,3-Dihydrothien-2-yl, 2,3-Dihydrothien-3-yl, 2,4-Dihydrothien-2-yl, 2,4-Dihydrothien-3-yl, 4,5-Dihydropyrrol-2-yl, 4,5-Dihydropyrrol-3-yl, 2,5-Dihydropyrrol-2-yl, 2,5-Dihydropyrrol-3-yl, 4,5-Dihydroisoxazol-3-yl, 2,5-Dihydroisoxazol-3-yl, 2,3-Dihydroisoxazol-3-yl, 4,5-Dihydroisoxazol-4-yl, 2,5-Dihydroisoxazol-4-yl, 2,3-Dihydroisoxazol-4-yl, 4,5-Dihydroisoxazol-5-yl, 2,5-Dihydroisoxazol-5-yl, 2,3-Dihydroisoxazol-5-yl, 4,5-Dihydroisothiazol-3-yl, 2,5-Dihydroisothiazol-3-yl, 2,3-Dihydroisothiazol-3-yl, 4,5-Dihydroisothiazol-4-yl, 2,5-Dihydroisothiazol-4-yl, 2,3-Dihydroisothiazol-4-yl, 4,5-Dihydroisothiazol-5-yl, 2,5-Dihydroisothiazol-5-yl, 2,3-Dihydroisothiazol -5-yl, 2,3-Dihydropyrazol-2-yl, 2,3-Dihydropyrazol-3-yl, 2,3-Dihydropyrazol-4-yl, 2,3-Dihydropyrazol-5-yl, 3,4-Dihydropyrazol-3-yl, 3,4-Dihydropyrazol-4-yl, 3,4-Dihydropyrazol-5-yl, 4,5-Dihydropyrazol-3-yl, 4,5-Dihydropyrazol-4-yl, 4,5-Dihydropyrazol-5-yl, 2,3-Dihydroimidazol-2-yl, 2,3-Dihydroimidazol-3-yl ,2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2,3-Dihydrooxazol-3-yl, 2,3-Dihydrooxazol-4-yl, 2,3-Dihydrooxazol-5-yl, 3,4-Dihydrooxazol-3-yl, 3,4-Dihydrooxazol-4-yl, 3,4-Dihydrooxazol-5-yl, 2,3-Dihydrothiazol-3-yl, 2,3-Dihydrothiazol-4-yl, 2,3-Dihydrothiazol-5-yl, 3,4-Dihydrothiazol-3-yl, 3,4-Dihydrothiazol-4-yl, 3,4-Dihydrothiazol-5-yl, 3,4-Dihydrothiazol-2-yl, 3,4-Dihydrothiazol-3-yl, 3,4-Dihydrothiazol-4-yl,
   z.B. 4,5-Dihydropyrrol-1-yl, 2,5-Dihydropyrrol-1-yl, 4,5-Dihydroisoxazol-2-yl, 2,3-Dihydroisoxazol-1-yl, 4,5-Dihydroisothiazol-1-yl, 2,3-Dihydroisothiazol-1-yl, 2,3-Dihydropyrazol-1-yl, 4,5-Dihydropyrazol-1-yl, 3,4-Dihydropyrazol-1-yl, 2,3-Dihydroimidazol-1-yl, 4,5-Dihydroimidazol-1-yl, 2,5-Dihydroimidazol-1-yl, 2,3-Dihydrooxazol-2-yl, 3,4-Dihydrooxazol-2-yl, 2,3-Dihydrothiazol-2-yl, 3,4-Dihydrothiazol-2-yl,
   z.B. 2-Piperidinyl, 3-Piperidinyl, 4-Piperidinyl, 1,3-Dioxan-2-yl, 1,3-Dioxan-4-yl, 1,3-Dioxan-5-yl, 1,4-Dioxan-2-yl, 1,3-Dithian-2-yl, 1,3-Dithian-3-yl, 1,3-Dithian-4-yl, 1,4-Dithian-2-yl, 1,3-Dithian-5-yl, 2-Tetrahydropyranyl, 3-Tetrahydropyranyl, 4-Tetrahydropyranyl, 2-Tetrahydrothiopyranyl, 3-Tetrahydrothiopyranyl, 4-Tetrahydrothiopyranyl, 3-Hexahydropyridazinyl, 4-Hexahydropyridazinyl, 2-Hexahydropyrimidinyl, 4-Hexahydropyrimidinyl, 5-Hexahydropyrimidinyl, 2-Piperazinyl, 1,3,5-Hexahydrotriazin-2-yl, 1,2,4-Hexahydrotriazin-3-yl, Tetrahydro-1,3-oxazin-2-yl, Tetrahydro-1,3-oxazin-6-yl, 2-Morpholinyl, 3-Morpholinyl, 1,3,5-Trioxan-2-yl,
   z.B. 1-Piperidinyl, 1-Hexahydropyridazinyl, 1-Hexahydropyrimidinyl, 1-Piperazinyl, 1,3,5-Hexahydrotriazin-1-yl, 1,2,4-Hexahydrotriazin-1-yl, Tetrahydro-1,3-oxazin-1-yl, 1-Morpholinyl,
   z.B. 2H-Pyran-2-yl, 2H-Pyran-3-yl, 2H-Pyran-4-yl, 2H-Pyran-5-yl, 2H-Pyran-6-yl, 3,6-Dihydro-2H-pyran-2-yl, 3,6-Dihydro-2H-pyran-3-yl, 3,6-Dihydro-2H-pyran-4-yl, 3,6-Dihydro-2H-pyran-5-yl, 3,6-Dihydro-2H-pyran-6-yl, 3,4-Dihydro-2H-pyran-3-yl, 3,4-Dihydro-2H-pyran-4-yl, 3,4-Dihydro-2H-pyran-6-yl, 2H-Thiopyran-2-yl, 2H-Thiopyran-3-yl, 2H-Thiopyran-4-yl, 2H-Thiopyran-5-yl, 2H-Thiopyran-6-yl, 5,6-Dihydro-4H-1,3-oxazin-2-yl;
- Aryl sowie der Arylteil von Aryl(C₁-C₄-alkyl): ein- bis dreikerniger aromatischer Carbocyclus mit 6 bis 14 Ringgliedern, wie z.B. Phenyl, Naphthyl und Anthracenyl;
- Heteroaryl sowie die Heteroarylreste in Heteroaryl-C₁-C₄-alkyl, Heteroaryl-C₁-C₄-alkyl, Heteroaryl-C₂-C₄-alkenyl, Heteroaryl-C₂-C₄-alkinyl, Heteroaryl-C₁-C₄-halogenalkyl, Heteroaryl-C₂-C₄-halogenalkenyl, Heteroaryl-C₂-C₄-halogenalkinyl, Heteroaryl-C₁-C₄-hydroxyalkyl, Heteroaryl-C₂-C₄-hydroxyalkenyl, Heteroaryl-C₂-C₄-hydroxyalkinyl, Heteroarylcarbonyl-C₁-C₄-alkyl, Heteroarylcarbonyloxy-C₁-C₄-alkyl, Heteroaryloxycarbonyl-C₁-C₄-alkyl, Heteroaryloxy-C₁-C₄-alkyl, Heteroarylthio-C₁-C₄-alkyl, Heteroarylsulfinyl-C₁-C₄-alkyl, Heteroarylsulfonyl-C₁-C₄-alkyl:
   mono- oder bicyclisches aromatisches Heteroaryl mit 5 bis 10 Ringgliedern, welches neben Kohlenstoffatomen 1 bis 4 Stickstoffatome, oder 1 bis 3 Stickstoffatome und ein Sauerstoff- oder ein Schwefelatom, oder ein Sauerstoff- oder ein Schwefelatom enthält, z.B.
   Monocyclen wie Furyl (z.B. 2-Furyl, 3-Furyl), Thienyl (z.B. 2-Thienyl, 3-Thienyl), Pyrrolyl (z.B. Pyrrol-2-yl, Pyrrol-3-yl), Pyrazolyl (z.B. Pyrazol-3-yl, Pyrazol-4-yl), Isoxazolyl (z.B. Isoxazol-3-yl, Isoxazol-4-yl, Isoxazol-5-yl), Isothiazolyl (z.B. Isothiazol-3-yl, Isothiazol-4-yl, Isothiazol-5-yl), Imidazolyl (z.B. Imidazol-2-yl, Imidazol-4-yl), Oxazolyl (z.B. Oxazol-2-yl, Oxazol-4-yl, Oxazol-5-yl), Thiazolyl (z.B. Thiazol-2-yl, Thiazol-4-yl, Thiazol-5-yl), Oxadiazolyl (z.B. 1,2,3-Oxadiazol-4-yl, 1,2,3-Oxadiazol-5-yl, 1,2,4-Oxadiazol-3-yl, 1,2,4,-Oxadiazol-5-yl, 1,3,4-Oxadiazol-2-yl), Thiadiazolyl (z.B. 1,2,3-Thiadiazol-4-yl, 1,2,3-Thiadiazol-5-yl, 1,2,4-Thiadiazol-3-yl, 1,2,4-Thiadiazol-5-yl, 1,3,4-Thiadiazolyl-2-yl), Triazolyl (z.B. 1,2,3-Triazol-4-yl, 1,2,4-Triazol-3-yl), Tetrazol-5-yl, Pyridyl (z.B. Pyridin-2-yl, Pyridin-3-yl, Pyridin-4-yl), Pyrazinyl (z.B. Pyridazin-3-yl, Pyridazin-4-yl), Pyrimidinyl (z.B. Pyrimidin-2-yl, Pyrimidin-4-yl, Pyrimidin-5-yl), Pyrazin-2-yl, Triazinyl (z.B. 1,3,5-Triazin-2-yl, 1,2,4-Triazin-3-yl, 1,2,4-Triazin-5-yl, 1,2,4-Triazin-6-yl), Tetrazinyl (z.B. 1,2,4,5-Tetrazin-3-yl); sowie
   Bicyclen wie die benzanellierten Derivate der vorgenannten Monocyclen, z.B. Chinolinyl, Isochinolinyl, Indolyl, Benzthienyl, Benzofuranyl, Benzoxazolyl, Benzthiazolyl, Benzisothiazolyl, Benzimidazolyl, Benzopyrazolyl, Benzthiadiazolyl, Benzotriazolyl.
- 5- oder 6-gliedriges Heteroaryl mit ein bis vier Stickstoffatomen, oder ein bis drei Stickstoffatomen und einem Sauerstoff- oder Schwefelatom, oder mit einem Sauerstoff- oder Schwefelatom:
   z.B. über ein C-Atom verknüpfte aromatische 5-Ring-Heterocyclen, welche neben Kohlenstoffatomen ein bis vier Stickstoffatome, oder ein bis drei Stickstoffatome und ein Schwefel- oder Sauerstoffatom, oder ein Schwefel- oder Sauerstoffatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl und 1,3,4-Triazol-2-yl;
   z.B. über ein C-Atom verknüpfte aromatische 6-Ring Heterocyclen, welche neben Kohlenstoffatomen ein bis vier, vorzugsweise ein bis drei Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl und 1,2,4-Triazin-3-yl.

Alle Phenyl- und Arylringe bzw. Heterocyclyl- und Heteroarylreste sowie alle Phenylkomponenten in Phenyl-C₁-C₆-alkyl, Phenylcarbonyl, Phenylcarbonyl-C₁-C₆-alkyl, Phenylcarbonylamino-C₁-C₄-alkyl, Phenoxycarbonyl, Phenylaminocarbonyl, Phenylsulfonylaminocarbonyl, N-(C₁-C₆-Alkyl)-N-phenylaminocarbonyl und Phenyl-C₁-C₆-alkylcarbonyl, alle Arylkomponenten in Aryl(C₁-C₄-alkyl), alle Heteroarylkomponenten in mono- oder bicyclischem Heteroaryl und alle Heterocyclylkomponenten in Heterocylyl, Heterocydyl-C₁-C₆-alkyl, Heterocyclylcarbonyl, Heterocyclylcarbonyl-C₁-C₆-alkyl, Heterocyclyloxycarbonyl, Heterocyclylaminocarbonyl, Heterocyclylsulfonylaminocarbonyl, N-(C₁-C₆-Alkyl)-N-heterocyclylaminocarbonyl und Heterocyclyl-C₁-C₆-alkylcarbonyl sind, soweit nicht anders angegeben, vorzugsweise unsubstituiert oder tragen ein bis drei Halogenatome und/oder eine Nitrogruppe, einen Cyanorest und/oder einen oder zwei Methyl-, Trifluormethyl-, Methoxy- oder Trifluormethoxysubstituenten.

In einer besonderen Ausführungsform haben die Variablen der heteroaroylsubstituierten Serin-Amide der Formel (I) folgende Bedeutungen, wobei diese sowohl für sich allein betrachtet als auch in Kombination miteinander bevorzugte Ausgestaltungen der Verbindungen der Formel (I) darstellen:

Bevorzugt sind die heteroaroyl-substituierten Serin-Amide der Formel (I), in der
- A: 5-gliedriges Heteroaryl mit ein bis vier Stickstoffatomen, oder ein bis drei Stick- stoffatomen und einem Sauerstoff- oder Schwefelatom, oder mit einem Sauer- stoff oder Schwefelatom; besonders bevorzugt 5-gliedriges Heteroaryl ausgewählt aus der Gruppe Thie- nyl, Furyl, Pyrazolyl, Imidazolyl, Thiazolyl und Oxazolyl;
insbesonders bevorzugt 5-gliedriges Heteroaryl ausgewählt aus der Gruppe Thienyl, Furyl, Pyrazolyl und Imidazolyl; wobei die genannten Heteroarylreste durch einen C₁-C₆-Halogenalkyl- Rest, bevorzugt in 2-Position durch einen C₁-C₆-Halogenalkyl-Rest substituiert sind, und 1 bis 3 Reste aus der Gruppe Halogen, Cyano, C₁- C₆-Alkyl, C₃-C₆-Cycloalkyl, C₁-C₆-Alkoxy, C₁-C₆-Halogenalkoxy und C₁- C₆-Alkoxy-C₁-C₄-alkyl tragen können;
bedeutet.

Ebenso bevorzugt sind die heteroaroyl-substituierten Serin-Amide der Formel (I), in der
- A: 5-gliedriges Heteroaryl mit ein bis vier Stickstoffatomen, oder ein bis drei Stick- stoffatomen und einem Sauerstoff- oder Schwefelatom, oder mit einem Sauer- stoff oder Schwefelatom;
besonders bevorzugt 5-gliedriges Heteroaryl ausgewählt aus der Gruppe Thie- nyl, Furyl, Pyrazolyl, Imidazolyl, Thiazolyl und Oxazolyl; insbesonders bevorzugt 5-gliedriges Heteroaryl ausgewählt aus der Gruppe Thienyl, Furyl, Pyrazolyl und Imidazolyl;
wobei die genannten Heteroarylreste partiell oder vollständig halogeniert sein können und/oder 1 bis 3 Reste aus der Gruppe Cyano, C₁-C₆-Alkyl, C₃-C₆-Cycloalkyl, C₁-C₆-Halogenalkyl, C₁-C₆-Alkoxy, C₁-C₆- Halogenalkoxy und C₁-C₆-Alkoxy-C₁-C₄-alkyl tragen können;
bedeutet.

Ebenso bevorzugt sind die heteroaroyl-substituierten Serin-Amide der Formel (I), in der
- A: 5-gliedriges Heteroaryl mit ein bis vier Stickstoffatomen, oder ein bis drei Stick- stoffatomen und einem Sauerstoff- oder Schwefelatom, oder mit einem Sauer- stoffatom;
besonders bevorzugt 5-gliedriges Heteroaryl ausgewählt aus der Gruppe Furyl, Pyrazolyl, Imidazolyl, Thiazolyl und Oxazolyl; insbesonders bevorzugt 5-gliedriges Heteroaryl ausgewählt aus der Gruppe Fu- ryl, Pyrazolyl und Imidazolyl,
wobei die genannten Heteroarylreste partiell oder vollständig halogeniert sein können und/oder 1 bis 3 Reste aus der Gruppe Cyano, C₁-C₆-Alkyl, C₃-C₆-Cycloalkyl, C₁-C₆-Halogenalkyl, C₁-C₆-Alkoxy, C₁-C₆- Halogenalkoxy und C₁-C₆-Alkoxy-C₁-C₄-alkyl tragen können;
bedeutet.

Ebenso bevorzugt sind die heteroaroyl-substituierten Serin-Amide der Formel (I), in der
- A: 6-gliedriges Heteroaryl mit ein bis vier Stickstoffatomen; besonders bevorzugt Pyridyl oder Pyrimidyl. insbesondere bevorzugt Pyrimidyl,
wobei die genannten Heteroarylreste partiell oder vollständig halogeniert sein können und/oder 1 bis 3 Reste aus der Gruppe Cyano, C₁-C₆-Alkyl, C₃-C₆-Cycloalkyl, C₁-C₆-Halogenalkyl, C₁-C₆-Alkoxy, C₁-C₆- Halogenalkoxy und C₁-C₆-Alkoxy-C₁-C₄-alkyl tragen können;
bedeutet.

Ebenso bevorzugt sind die heteroaroylsubstituierten Serin-Amide der Formel (I), in der
- A: 5- oder 6-gliedriges Heteroaryl mit ein bis vier Stickstoffatomen, oder mit ein bis drei Stickstoffatomen und einem Sauerstoff- oder Schwefelatom, oder mit einem Sauerstoff- oder Schwefelatom,
welches durch einen C₁-C₆-Halogenalkyl-Rest, bevorzugt in 2-Position durch einen C₁-C₆-Halogenalkyl-Rest,substituiert sind, und 1 bis 3 Reste aus der Gruppe Cyano, C₁-C₆-Alkyl, C₃-C₆-Cycloalkyl, C₁-C₆- Halogenalkyl, C₁-C₆-Alkoxy, C₁-C₆-Halogenalkoxy und C₁-C₆-Alkoxy-C₁- C₄-alkyl tragen kann;
bedeutet.

Ebenso bevorzugt sind die heteroaroylsubstituierten Serin-Amide der Formel (I), in der
- A: 5- oder 6-gliedriges Heteroaryl ausgewählt aus der Gruppe Pyrrolyl, Thienyl, Furyl, Pyrazolyl, Imidazolyl, Thiazolyl, Oxazolyl, Tetrazolyl, Pyridyl und Pyrimidi- nyl,
wobei die genannten Heteroarylreste partiell oder vollständig halogeniert sein können und/oder 1 bis 3 Reste aus der Gruppe Cyano, C₁-C₆-Alkyl, C₃-C₆-Cycloalkyl, C₁-C₆-Halogenalkyl, C₁-C₆-Alkoxy, C₁-C₆- Halogenalkoxy und C₁-C₆-Alkoxy-C₁-C₄-alkyl tragen können;
besonders bevorzugt 5- oder 6-gliedriges Heteroaryl ausgewählt aus der Gruppe Thienyl, Furyl, Pyrazolyl, Imidazolyl, Thiazolyl, Oxazolyl und Pyridyl,
wobei die genannten Heteroarylreste partiell oder vollständig halogeniert sein können und/oder 1 bis 3 Reste aus der Gruppe C₁-C₆-Alkyl, C₃-C₆- Cycloalkyl und C₁-C₆-Halogenalkyl tragen können;
insbesondere bevorzugt 5-gliedriges Heteroaryl ausgewählt aus der Gruppe Thienyl, Furyl, Pyrazolyl, Imidazolyl, Thiazolyl und Oxazolyl,
wobei die genannten Heteroarylreste partiell halogeniert sein können und/oder 1 bis 2 Reste aus der Gruppe C₁-C₆-Alkyl und C₁-C₄- Halogenalkyl tragen können;
außerordentlich bevorzugt 5-gliedriges Heteroaryl ausgewählt aus der Gruppe Thienyl, Furyl, Pyrazolyl und Imidazolyl,
wobei die genannten Heteroarylreste partiell halogeniert sein können und/oder 1 bis 2 Reste aus der Gruppe C₁-C₆-Alkyl und C₁-C₄- Halogenalkyl tragen können.
bedeutet.

Ebenso bevorzugt sind die heteroaroylsubstituierten Serin-Amide der Formel (I), in der
- A: 5- oder 6-gliedriges Heteroaryl ausgewählt aus der Gruppe Pyrrolyl, Furyl, Pyra- zolyl, Imidazolyl, Thiazolyl, Oxazolyl, Tetrazolyl, Pyridyl und Pyrimidinyl,
wobei die genannten Heteroarylreste partiell oder vollständig halogeniert sein können und/oder 1 bis 3 Reste aus der Gruppe Cyano, C₁-C₆-Alkyl, C₃-C₆-Cycloalkyl, C₁-C₆-Halogenalkyl, C₁-C₆-Alkoxy, C₁-C₆- Halogenalkoxy und C₁-C₆-Alkoxy-C₁-C₄-alkyl tragen können;
besonders bevorzugt 5- oder 6-gliedriges Heteroaryl ausgewählt aus der Gruppe Furyl, Pyrazolyl, Imidazolyl, Thiazolyl, Oxazolyl und Pyridyl,
wobei die genannten Heteroarylreste partiell oder vollständig halogeniert sein können und/oder 1 bis 3 Reste aus der Gruppe C₁-C₆-Alkyl, C₃-C₆- Cycloalkyl und C₁-C₆-Halogenalkyl tragen können;
insbesondere bevorzugt 5-gliedriges Heteroaryl ausgewählt aus der Gruppe Fu- ryl, Pyrazolyl, Imidazolyl, Thiazolyl und Oxazolyl;
wobei die genannten Heteroarylreste partiell halogeniert sein können und/oder 1 bis 2 Reste aus der Gruppe C₁-C₆-Alkyl und C₁-C₄- Halogenalkyl tragen können;
außerordentlich bevorzugt 5-gliedriges Heteroaryl ausgewählt aus der Gruppe Furyl, Pyrazolyl und Imidazolyl,
wobei die genannten Heteroarylreste partiell halogeniert sein können und/oder 1 bis 2 Reste aus der Gruppe C₁-C₆-Alkyl und C₁-C₄- Halogenalkyl tragen können.
bedeutet.

Ebenso bevorzugt sind die heteroaroyl-substituierten Serin-Amide der Formel (I), in der
- A: C-verknüpftes 5-oder 6-gliedriges Heteroaryl ausgewählt aus der Gruppe A1 bis A14 mit 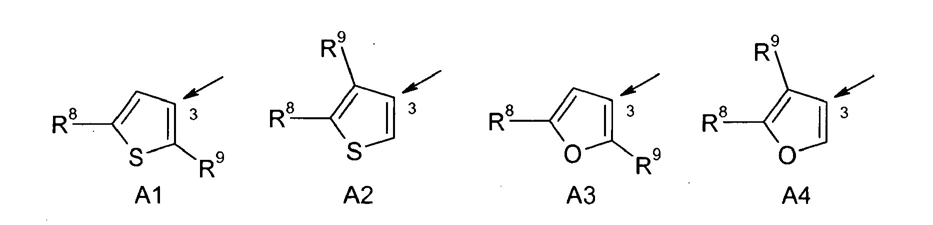 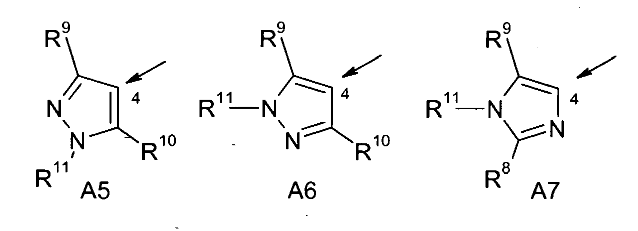 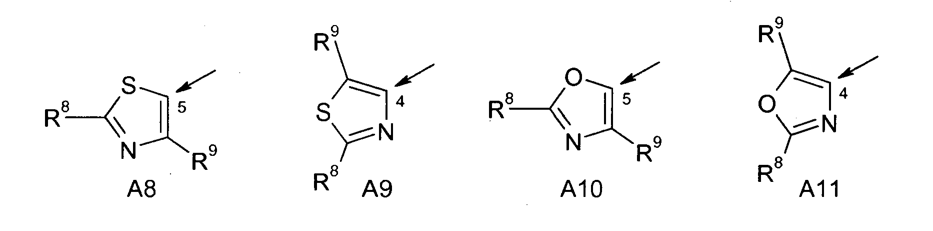 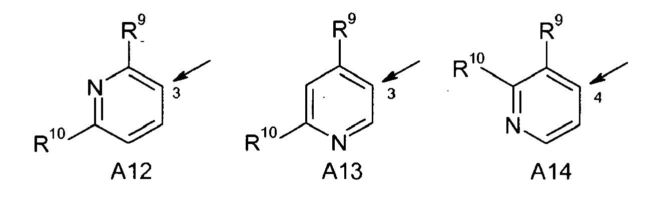 wobei der Pfeil die Verknüpfungsposition anzeigt und
- R⁸: Wasserstoff, Halogen, C₁-C₆-Alkyl oder C₁-C₆-Halogenalkyl; besonders bevorzugt Wasserstoff, C₁-C₄-Alkyl oder C₁-C₄-Halogenalkyl; insbesondere bevorzugt Wasserstoff oder C₁-C₄-Alkyl; außerordentlich bevorzugt Wasserstoff;
- R⁹: Halogen, C₁-C₆-Alkyl, C₁-C₆-Halogenalkyl oder C₁-C₆-Halogenalkoxy; besonders bevorzugt Halogen, C₁-C₄-Alkyl oder C₁-C₆-Halogenalkyl; insbesondere bevorzugt Halogen oder C₁-C₆-Halogenalkyl; sehr bevorzugt C₁-C₆-Halogenalkyl; außerordentlich bevorzugt C₁-C₄-Halogenalkyl sehr außerordentlich bevorzugt CF₃;
- R¹⁰: Wasserstoff, Halogen, C₁-C₆-Alkyl oder C₁-C₆-Halogenalkyl; besonders bevorzugt Wasserstoff, Halogen oder C₁-C₄-Halogenalkyl; insbesondere bevorzugt Wasserstoff oder Halogen; außerordentlich bevorzugt Wasserstoff; und
- R¹¹: Wasserstoff, C₁-C₆-Alkyl, C₃-C₆-Cycloalkyl, C₁-C₆-Halogenalkyl oder C₁- C₆-Alkoxy-C₁-C₄-alkyl; besonders bevorzugt C₁-C₄-Alkyl, C₃-C₆-Cycloalkyl, C₁-C₄-Halogenalkyl oder C₁-C₄-Alkoxy-C₁-C₄-alkyl; insbesondere bevorzugt C₁-C₄-Alkyl oder C₁-C₄-Halogenalkyl; außerordentlich bevorzugt C₁-C₄-Alkyl; sehr außerordentlich bevorzugt CH₃;
bedeuten;
besonders bevorzugt A1, A2, A3, A4, A5, A6, A8 oder A9;
wobei. R⁸ bis R¹¹ wie voranstehend genannt definiert werden;
außerordentlich bevorzugt A1, A2, A5 oder A6;
wobei R⁸ bis R¹¹ wie voranstehend genannt definiert werden;
bedeutet.

Ebenso bevorzugt sind die heteroaroyl-substituierten Serin-Amide der Formel (I), in der
- R¹: Wasserstoff;
bedeutet.

Ebenso bevorzugt sind die heteroaroyl-substituierten Serin-Amide der Formel (I), in der
- R²: Wasserstoff oder Hydroxy; besonders bevorzugt Wasserstoff;
bedeutet.

Ebenso bevorzugt sind die heteroaroyl-substituierten Serin-Amide der Formel (I), in der
- R¹: Wasserstoff; und
- R²: Wasserstoff oder Hydroxy; besonders bevorzugt Wasserstoff;
bedeuten.

Ebenso bevorzugt sind die heteroaroyl-substituierten Serin-Amide der Formel (I), in der
- R³: C₁-C₆-Alkyl oder C₁-C₆-Halogenalkyl; besonders bevorzugt C₁-C₆-Alkyl; insbesondere bevorzugt C₁-C₄-Alkyl; außerordentlich bevorzugt CH₃;
bedeutet.

Ebenso bevorzugt sind die heteroaroyl-substituierten Serin-Amide der Formel (I), in der
- R⁴: Wasserstoff, C₁-C₆-Alkyl, C₃-C₆-Alkenyl, C₃-C₆-Alkinyl, Formyl, C₁-C₆- Alkylcarbonyl, C₂-C₆-Alkenylcarbonyl, C₃-C₆-Cycloalkylcarbonyl, C₁-C₆- Alkoxycarbonyl, C₁-C₆-Alkylaminocarbonyl, C₁-C₆-Alkylsulfonylaminocarbonyl, (C₁-C₆)-Alkylaminothiocarbonyl, Di-(C₁-C₆-alkyl)-aminocarbonyl, N-(C₁-C₆- Alkoxy)-N-(C₁-C₆-alkyl)-aminocarbonyl, Di-(C₁-C₆-alkyl)-aminothiocarbonyl, C₁- C₆-Alkoxyimino-C₁-C₆-alkyl,
wobei die genannten Alkyl, Cycloalkyl- und Alkoxyreste partiell oder voll- ständig halogeniert sein können und/oder eine bis drei der folgenden Grup- pen tragen können: Cyano, Hydroxy, C₃-C₆-Cycloalkyl, C₁-C₄-Alkoxy, C₁-C₄- Alkylthio, Di-(C₁-C₄-alkyl)-amino, C₁-C₄-Alkylcarbonyl, Hydroxycarbonyl, C₁- C₄-Alkoxycarbonyl, Aminocarbonyl, C₁-C₄-Alkylaminocarbonyl" Di-(C₁-C₄- alkyl)-aminocarbonyl, oder C₁-C₄-Alkylcarbonyloxy;
Phenyl, Phenyl-C₁-C₆-alkyl, Phenylcarbonyl, Phenylcarbonyl-C₁-C₆-alkyl, Phenyl- sulfonylaminocarbonyl oder Phenyl-C₁-C₆-alkylcarbonyl,
wobei der Phenylring partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy; oder
SO₂R⁷ ;
besonders bevorzugt Wasserstoff, C₁-C₆-Alkyl, C₃-C₆-Alkenyl, C₃-C₆-Alkinyl, Formyl, C₁-C₆-Alkylcarbonyl, CrC6-Alkenylcarbonyl, C₁-C₆-Alkoxycarbonyl, C₁- C₆-Alkylsulfonylaminocarbonyl, (C₁-C₆)-Alkylaminocarbonyl, Di-(C₁-C₆-alkyl)- aminocarbonyl, N-(C₁-C₆-Alkoxy)-N-(C₁-C₆-alkyl)-aminocarbonyl, (C₁-C₆)- Alkylaminothiocarbonyl oder Di-(C₁-C₆-alkyl)-aminothiocarbonyl,
wobei die genannten Alkyl- oder Alkoxyreste partiell oder vollständig halo- geniert sein können und/oder eine bis drei der folgenden Gruppen tragen können: Cyano, C₁-C₄-Alkoxy, C₁-C₄-Alkoxycarbonyl, C₁-C₄-Alkylamino- carbonyl, Di-(C₁-C₄-alkyl)-aminocarbonyl oder C₁-C₄-Alkylcarbonyloxy;

Phenyl-C₁-C₆-alkyl, Phenylcarbonyl, Phenylcarbonyl-C₁-C₆-alkyl, Phenylsulfonyl- aminocarbonyl oder Phenyl-C₁-C₆-alkylcarbonyl,
wobei der Phenylring partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy; oder
SP₂R⁷;
insbesondere bevorzugt Wasserstoff, C₁-C₆-Alkyl, C₃-C₆-Alkenyl, C₃-C₆-Alkinyl, Formyl, C₁-C₆-Alkyl-carbonyl, C₂-C₆-Alkenylcarbonyl, C₁-C₆-Alkoxycarbonyl, (C₁- C₆-Alkyl)-amino-carbonyl, Di-(C₁-C₆-alkyl)-amino-carbonyl, N-(C₁-C₆-Alkoxy)-N- (C₁-C₆-alkyl)-aminocarbonyl, (C₁-C₆)-Alkylaminothiocarbonyl, Di-(C₁-C₆-alkyl)- aminothiocarbonyl, Phenyl-C₁-C₆-alkyl, Phenylcarbonyl, Phenylcarbonyl-C₁-C₆- alkyl oder Phenyl-C₁-C₆-alkylcarbonyl
wobei der Phenylring partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogen-alkoxy; oder
SO₂R⁷;
bedeutet.

Ebenso bevorzugt sind die heteroaroyl-substituierten Serin-Amide der Formel (I), in der
- R⁴: Wasserstoff, C₁-C₆-Alkyl, C₃-C₆-Alkenyl, C₃-C₆-Alkinyl, Formyl, C₁-C₆- Alkylcarbonyl, C₂-C₆-Alkenylcarbonyl, C₃-C₆-Cycloalkylcarbonyl, C₁-C₆-Alkoxy- carbonyl, C₁-C₆-Alkylaminocarbonyl, Di-(C₁-C₆-alkyl)-aminocarbonyl, N-(C₁-C₆- Alkoxy)-N-(C₁-C₆-alkyl)-aminocarbonyl, (C₁-C₆)-Alkylaminothiocarbonyl, Di-(C₁- C₆-alkyl)-aminothiocarbonyl, C₁-C₆-Alkoxyimino-C₁-C₆-alkyl,
wobei die genannten Alkyl-, Cycloalkyl- oder Alkoxyreste partiell oder voll- ständig halogeniert sein können und/oder eine bis drei der folgenden Grup- pen tragen können: Cyano, Hydroxy, C₃-C₆-Cycloalkyl, C₁-C₄-Alkoxy, C₁-C₄- Alkylthio, Di-(C₁-C₄-alkyl)-amino, C₁-C₄-Alkylcarbonyl, Hydroxycarbonyl, C₁- C₄-Alkoxycarbonyl, Aminocarbonyl, C₁-C₄-Alkylaminocarbonyl, Di-(C₁-C₄- alkyl)-aminocarbonyl oder C₁-C₄-Alkylcarbonyloxy; oder
SO₂R⁷;
bedeutet.

Ebenso bevorzugt sind die heteroaroyl-substituierten Serin-Amide der Formel (I), in der
- R⁴: Wasserstoff, C₁-C₆-Alkyl, C₃-C₆-Alkenyl, C₃-C₆-Alkinyl, Formyl, C₁-C₆-Alkyl- carbonyl, C₁-C₆-Alkoxycarbonyl, C₁-C₆-Alkylaminocarbonyl, Di-(C₁-C₆-alkyl)- aminocarbonyl, N-(C₁-C₆-Alkoxy)-N-(C₁-C₆-alkyl)-amino-carbonyl,
wobei die genannten Alkyl-, und Alkoxyreste partiell oder vollständig halo- geniert sein können und/oder eine bis drei der folgenden Gruppen tragen können: Cyano, C₁-C₄-Alkoxy, C₁-C₄-Alkylaminocarbonyl oder Di-(C₁-C₄- alkyl)-aminocarbonyl;
Phenyl-C₁-C₆-alkyl, Phenylcarbonyl, Phenylcarbonyl-C₁-C₆-alkyl, Phenylamino- carbonyl, oder N-(C₁-C₆-Alkyl)-N-(phenyl)-aminocarbonyl,
wobei der Phenylring partiell oder vollständig halogeniert sein kann und/oder eine bis drei der folgenden Gruppen tragen kann: Cyano, C₁-C₄- Alkyl oder C₁-C₄-Halogenalkyl; oder
SP₂R⁷;
besonders bevorzugt Wasserstoff, Formyl, C₁-C₄-Alkylcarbonyl, C₁-C₄- Alkylaminocarbonyl, Di-(C₁-C₄-alkyl)-aminocarbonyl, Phenylaminocarbonyl, N- (C₁-C₄-Alkyl)-N-(phenyl)-aminocarbonyl, SO₂CH₃, SO₂CF₃ oder SO₂(C₆H₅); bedeutet.

Ebenso bevorzugt sind die heteroaroyl-substituierten Serin-Amide der Formel (I), in der
- R⁵ und R⁶: zusammen mit dem Kohlenstoffatom, an das sie gebunden sind, einen 3 bis 7-gliedrigen, gesättigten oder partiell ungesättigten Ring, bedeuten, der car- bocyclisch ist oder 1 oder 2 N-Atome, 0 oder 1 N-Atom und 1 O-Atom oder S- Atom, 0 oder 1 N-Atom und 1 O- und 1 S-Atom oder 2 O- oder S-Atome enthält,
wobei der Ring unsubstituiert ist oder wie in der Formel (I) angegeben substituiert ist,
und wobei der Ring monocyclisch ist oder anelliert mit einem weiteren 3 bis 6- gliedrigen gesättigten, oder partiell ungesättigten Ring, der carbocyclisch ist oder 1 oder 2 N-Atome, 0 oder 1 N-Atom oder 1 O-Atom oder S-Atom, 2 O-Atome o- der S-Atome, 0 oder 1 N-Atom und 1 O-Atom und 1 S-Atome enthält,
wobei der anellierte Ring unsubstituiert ist oder substiutiert durch 1 bis 3, im Fall von Halogen auch bis zur maximal möglichen Anzahl, Substituen- ten aus der Gruppe Halogen, C₁-C₆-Alkyl, C₁-C₆-Halogenalkyl und C₁-C₆- Alkoxy,
und wobei der Ring nicht verbrückt ist oder überbrückt mit einer 1 bis 3-zähligen gesättigten oder ungesättigten Kette, die keine Heteroatome enthält oder 1 N- Atom, 0 oder 1 N-Atom und 1 O-Atom oder 1 S-Atom enthält,
wobei die Brücke unsubstituiert ist oder substituiert mit 1 bis 3, im Fall von Halogen auch bis zur maximal möglichen Anzahl, Substituenten aus der Gruppe Halogen, C₁-C₆-Alkyl, Hydroxy und C₁-C₆-Alkoxy;
besonders bevorzugt zusammen mit dem Kohlenstoffatom, an das sie gebunden sind, einen 3 bis 7-gliedrigen, gesättigten oder partiell ungesättigten Ringe, be- deuten, der carbocyclisch ist oder 1 oder 2 N-Atome, 0 oder 1 N-Atom und 1 O- Atom oder S-Atom, 0 oder 1 N-Atom und 1 O- und 1 S-Atom, 2 O- oder S-Atome, enthält,
wobei der Ring unsubstituiert ist oder substituiert durch 1 bis 3, im Fall von Halogen auch bis zur maximal möglichen Anzahl, Substituenten aus der Gruppe Halogen, C₁-C₆-Alkyl, Hydroxy, C₁-C₆-Alkoxy, Formyl, C₁-C₆-Alkyl- carbonyl, C₁-C₆-Alkoxycarbonyl, C₁-C₆-Alkylaminocarbonyl, Di-(C₁-C₆- alkyl)-aminocarbonyl, Alkylsulfonylamino, Carbonyl, und Alkoxyimino,
wobei die genannten Alkyl- und Alkoxyreste partiell oder vollständig halogeniert sein können und/oder eine bis drei der folgenden Grup- pen tragen können: Cyano, Hydroxy und C₁-C₄-Alkoxy,
Phenyl, partiell oder vollständig halogeniert,
und wobei der Ring monocyclisch ist oder anelliert mit einem weiteren 3 bis 6- gliedrigen gesättigtem oder partiell ungesättigtem Ring, der carbocyclisch ist o- der 1 oder 2 N-Atome, 0 oder 1 N-Atom und 1 O-Atom oder S-Atom, 2 O-Atome oder S-Atome, 0 oder 1 N-Atom und 1 O-Atom und 1 S-Atom enthält,
wobei der anellierte Ring unsubstituiert ist oder substituiert durch 1 bis 3, im Fall von Halogen auch bis zur maximal möglichen Anzahl, Substituen- ten aus der Gruppe Halogen, C₁-C₆-Alkyl, C₁-C₆-Halogenalkyl und C₁-C₆- Alkoxy,
und wobei der Ring unverbrückt ist oder überbrückt mit einer 1 bis 3-zähligen gesättigten oder ungesättigten Kette, die keine Heteroatome enthält oder 1 N- Atom oder 0 oder 1 N-Atom und 1 O-Atom oder 1 S-Atom enthält,
wobei die Brücke unsubstituiert ist oder substituiert mit 1 bis 3, im Fall von Halogen auch bis zur maximal möglichen Anzahl, Substituenten aus der Gruppe Halogen, C₁-C₆-Alkyl, Hydroxy und C₁-C₆-Alkoxy.

Ebenso bevorzugt sind die heteroaroyl-substituierten Serin-Amide der Formel (I), in der
- R⁵ und R⁶: zusammen mit dem Kohlenstoffatom, an das sie gebunden sind, einen 3 bis 12-gliedrigen, monocyclischen, gesättigten oder partiell ungesättigten Ringe bedeuten, der carbocyclisch ist oder 1 bis 3 N-Atome, 0 bis 3 N-Atome und 1 O- Atom oder S-Atom, 0 bis 2 N-Atome und 2 O- oder S-Atome, 0 oder 1 N-Atom und 1 O- und 1 S-Atom, 3 O- oder S-Atome, 2 O-Atome und 1 S-Atom, oder 1 O- und 2 S-Atome enthält, wobei der Ring unsubstituiert ist oder wie in der Formel (I) angegeben substituiert ist;
besonders bevorzugt zusammen mit dem Kohlenstoffatom, an das sie ge- bunden sind, einen 3 bis 7-gliedrigen, monocyclischen, gesättigten oder partiell ungesättigten Ring bedeuten, der carbocyclisch ist oder 1 oder 2 N- Atome, 0 oder 1 N-Atom und 1 O-Atom oder S-Atom, 0 oder 1 N-Atom und 1 O- und 1 S-Atom, 3 O- oder S-Atome, enthält,
wobei der Ring unsubstituiert ist oder wie in der Formel (I) angegeben substituiert;
ganz besonders bevorzugt zusammen mit dem Kohlenstoffatom, an das sie gebunden sind, einen 3 bis 7-gliedrigen, monocyclischen, gesättigten oder partiell ungesättigten Ring bedeuten, der carbocyclisch ist oder 1 bis 3 N-Atome, 0 bis 3 N-Atome und 1 O-Atom oder S-Atom, 0 bis 2 N-Atome und 2 O- oder S-Atome, 0 oder 1 N-Atom und 1 O- und 1 S-Atom, 3 O- oder S-Atome, 2 O-Atome und 1 S-Atom, oder 1 O- und 2 S-Atome ent- hält,
wobei der Ring unsubstituiert ist oder substiutiert ist durch 1 bis 3, im Fall von Halogen auch bis zur maximal möglichen Anzahl, Substi- tuenten aus der Gruppe Halogen, C₁-C₆-Alkyl, Hydroxy, C₁-C₆-Alkoxy, Formyl, C₁-C₆-Alkyl-carbonyl, C₁-C₆-Alkoxycarbonyl, C₁-C₆- Alkylaminocarbonyl, Di-(C₁-C₆-alkyl)-aminocarbonyl, Alkylsulfonyla- mino, Carbonyl, Alkoxyimino,
wobei die genannten Alkyl- und Alkoxyreste partiell oder voll- ständig halogeniert sein können und/oder eine bis drei der fol- genden Gruppen tragen können: Cyano, Hydroxy, C₁-C₄- Alkoxy,
Phenyl, partiell oder vollständig halogeniert.

Ebenso bevorzugt sind die heteroaroyl-substituierten Serin-Amide der Formel (I), in der
- R⁵ und R⁶: zusammen mit dem Kohlenstoffatom, an das sie gebunden sind, einen 3 bis 12-gliedrigen, gesättigten oder partiell ungesättigten Ring bedeuten, der car- bocyclisch ist oder 1 bis 3 N-Atome, 0 bis 3 N-Atome und 1 O- oder S-Atom, 0 bis 2 N-Atome und 2 O- oder S-Atome, 0 oder 1 N-Atom und 1 O- und 1 S-Atom, 3 O- oder S-Atome, 2 O-Atome und 1 S-Atom, oder 1 O- und 2 S-Atome enthält,
wobei der Ring unsubstituiert ist oder wie in der Formel (I) angegeben substituiert
und wobei der Ring anelliert ist mit einem weiteren 3 bis 7-gliedrigen gesättigten, partiell ungesättigen oder vollständig ungesättigten Ring, der carbocyclisch ist oder 1 bis 3 N-Atome, 0 bis 2 N-Atome und 1 O-Atom oder S-Atom, 0 oder 1 N- Atom und 2 O-Atome oder S-Atome, 0 oder 1 N-Atom und 1 O-Atom und 1 S- Atom, 2 O-Atome und 1 S-Atom oder 1 O-Atom und 2 S-Atome enthält,
wobei der anellierte Ring unsubstituiert ist oder substituiert durch 1 bis 3, im Fall von Halogen auch bis zur maximalen möglichen Anzahl, Substi- tuenten aus der Gruppe Halogen, Cyano, Nitro, C₁-C₆-Alkyl, C₃-C₆-Alkenyl, C₃-C₆-Alkinyl, C₁-C₆-Halogenalkyl, C₃-C₆-Halogenalkenyl, C₃-C₆- Halogenalkinyl, Hydroxy, C₁-C₆-Alkoxy, C₁-C₆-Halogenalkoxy und C₁-C₆- Alkylsulfonyl;
besonders bevorzugt zusammen mit dem Kohlenstoffatom, an das sie ge- bunden sind, einen 3 bis 7-gliedrigen, gesättigten oder partiell ungesättig- ten Ring bedeuten, der carbocyclisch ist oder 1 oder 2 N-Atome, 0 oder 1 N-Atom und 1 O- Atom oder S-Atom, 0 oder 1 N-Atom und 1 O- und 1 S- Atom, 2 O- oder S-Atome enthält,
wobei der Ring unsubstituiert ist oder wie in der Formel (I) angegeben substituiert
und wobei der Ring anelliert ist mit einem weiteren 3 bis 7-gliedrigen ge- sättigten, partiell ungesättigten oder vollständig ungesättigten Ring, der carbocyclisch ist oder 1 bis 3 N-Atome, 0 bis 2 N-Atome und 1 O-Atom o- der S-Atom, 0 oder 1 N-Atom und 2 O-Atome oder S-Atome, 0 oder 1 N- Atom und 1 O-Atom und 1 S-Atom, 2 O-Atome und 1 S-Atom oder 1 O- Atom und 2 S-Atome enthält,
wobei der anellierte Ring unsubstituiert ist oder substituiert durch 1 bis 3, im Fall von Halogen auch bis zur maximal möglichen Anzahl, Substituenten aus der Gruppe Halogen, Cyano, Nitro, C₁-C₆-Alkyl, C₃- C₆-Alkenyl, C₃-C₆-Alkinyl, C₁-C₆-Halogenalkyl, C₃-C₆-Halogenalkenyl, C₃-C₆-Halogenalkinyl, Hydroxy, C₁-C₆-Alkoxy, C₁-C₆-Halogenalkoxy und C₁-C₆-Alkylsulfonyl;
ganz besonders bevorzugt zusammen mit dem Kohlenstoffatom, an das sie gebunden sind, einen 3 bis 7-gliedrigen, gesättigten oder partiell ungesättig- ten Ring bedeuten, der carbocyclisch ist oder 1 oder 2 N-Atome, 0 oder 1 N-Atom und 1 O-Atom oder S-Atom, 0 oder 1 N-Atom und 1 O- und 1 S- Atom, 2 O- oder S-Atome enthält,
wobei der Ring unsubstituiert ist oder substituiert durch 1 bis 3, im Fall von Halogen auch bis zur maximal möglichen Anzahl, Substi- tuenten aus der Gruppe Halogen, C₁-C₆-Alkyl, Hydroxy, C₁-C₆-Alkoxy, Formyl, C₁-C₆-Alkyl-carbonyl, C₁-C₆-Alkoxycarbonyl, C₁-C₆- Alkylaminocarbonyl, Di-(C₁-C₆-alkyl)-aminocarbonyl, Alkylsulfonyla- mino, Carbonyl, Alkoxyimino,
wobei die genannten Alkyl- und Alkoxyreste partiell oder voll- ständig halogeniert sein können und/oder eine bis drei der fol- genden Gruppen tragen können: Cyano, Hydroxy, C₁-C₄- Alkoxy,
Phenyl, partiell oder vollständig halogeniert,
und wobei der Ring anelliert ist mit einem weiteren 3 bis 6-gliedrigen gesät- tigten oder partiell ungesättigten Ring, der carbocyclisch ist oder 1 oder 2 N-Atome, 0 oder 1 N-Atom und 1 O-Atom oder S-Atom, 2 O-Atome oder S- Atome, 0 oder 1 N-Atom und 1 O-Atom und 1 S-Atom enthält,
wobei der anellierte Ring unsubstituiert ist oder substituiert durch 1 bis 3, im Fall von Halogen auch bis zur maximal möglichen Anzahl, Substituenten aus der Gruppe Halogen, C₁-C₆-Alkyl, C₁-C₆- Halogenalkyl und C₁-C₆-Alkoxy.

Ebenso bevorzugt sind die heteroaroyl-substituierten Serin-Amide der Formel (I), in der
- R⁵ und R⁶: zusammen mit dem Kohlenstoffatom, an das sie gebunden sind, einen 3 bis 12-gliedrigen, gesättigten oder partiell ungesättigten Ring bedeuten, der car- bocyclisch ist oder 1 bis 3 N-Atome, 0 bis 3 N-Atome und 1 O- oder S-Atom, 0 bis 2 N-Atome und 2 O- oder S-Atome, 0 oder 1 N-Atom und 1 O- und 1 S-Atom, 3 O- oder S-Atome, 2 O-Atome und 1 S-Atom, oder 1 O- und 2 S-Atome enthält, wobei der Ring unsubstituiert oder wie in der Formel (I) angegeben substi- tuiert ist, und wobei der Ring mit einer 1 bis 4-zähligen gesättigten oder ungesättigten Kette überbrückt ist, die keine Heteroatome enthält oder 1 oder 2 N-Atome, 0 oder 1 N-Atom und 1 O-Atom oder 1 S-Atom, 0 oder 1 N-Atom und 2 O-Atome oder 2 S-Atome, oder 0 oder 1 N-Atom und 1 O-Atom und 1 S-Atom enthält,
wobei die Brücke unsubstituiert ist oder substituiert mit 1 bis 3, im Fall von Halogen auch bis zur maximal möglichen Anzahl, Substituenten aus der Gruppe Halogen, Cyano, Nitro, C₁-C₆-Alkyl, C₃-C₆-Alkenyl, C₃-C₆-Alkinyl, C₁-C₆-Halogenalkyl, C₃-C₆-Halogenalkenyl, C₃-C₆-Halogenalkinyl, Hydroxy, C₁-C₆-Alkoxy, C₁-C₆-Halogenalkoxy und C₁-C₆-Alkylsulfonyl;
besonders bevorzugt zusammen mit dem Kohlenstoffatom, an das sie ge- bunden sind, einen 3 bis 7-gliedrigen, gesättigten oder partiell ungesättigten Ring bedeuten, der carbocyclisch ist oder 1 oder 2 N-Atome, 0 oder 1 N- Atom und 1 O-Atom oder S-Atom, 0 oder 1 N-Atom und 1 O- und 1 S-Atom, 2 O- oder S-Atome enthält,
wobei der Ring unsubstituiert oder wie in der Formel (I) angegeben substituiert ist,
und wobei der Ring mit einer 1 bis 4-zähligen gesättigten oder ungesättig- ten Kette überbrückt ist, die keine Heteroatome enthält oder 1 bis 2 N- Atome, 0 oder 1 N-Atom und 1 O-Atom oder 1 S-Atom, 0 oder 1 N-Atom und 2 O-Atome oder 2 S-Atome oder 0 oder 1 N-Atom und 1 O-Atom und 1 S-Atom enthält,
wobei die Brücke unsubstituiert ist oder substituiert mit 1 bis 3, im Fall von Halogen auch bis zur maximal möglichen Anzahl, Substituenten aus der Gruppe Halogen, Cyano, Nitro, C₁-C₆-Alkyl, C₃-C₆-Alkenyl, C₃-C₆-Alkinyl, C₁-C₆-Halogenalkyl, C₃-C₆-Halogenalkenyl, C₃-C₆-Halogenalkinyl, Hydroxy, C₁-C₆-Alkoxy, C₁-C₆-Halogenalkoxy und C₁-C₆-Alkylsulfonyl;
ganz besonders bevorzugt zusammen mit dem Kohlenstoffatom, an das sie gebunden sind, einen 3 bis 7-gliedrigen, gesättigten oder partiell ungesättig- ten Ring bedeuten, der carbocyclisch ist oder 1 oder 2 N-Atome, 0 oder 1 N-Atom und 1 O-Atom oder S-Atom, 0 oder 1 N-Atom und 1 O- und 1 S- Atom, 2 O- oder S-Atome enthält,
wobei der Ring unsubstituiert ist oder substituiert durch 1 bis 3, im Fall von Halogen auch bis zur maximal möglichen Anzahl, Substi- tuenten aus der Gruppe Halogen, C₁-C₆-Alkyl, Hydroxy, C₁-C₆-Alkoxy, Formyl, C₁-C₆-Alkyl-carbonyl, C₁-C₆-Alkoxycarbonyl, C₁-C₆- Alkylaminocarbonyl, Di-(C₁-C₆-alkyl)-aminocarbonyl, Alkylsulfonyla- mino, Carbonyl, Alkoxyimino,
wobei die genannten Alkyl- und Alkoxyreste partiell oder voll- ständig halogeniert sein können und/oder eine bis drei der fol- genden Gruppen tragen können: Cyano, Hydroxy, C₁-C₄- Alkoxy, Phenyl, partiell oder vollständig halogeniert,
und wobei der Ring mit einer 1 bis 4-zähligen gesättigten oder ungesättig- ten Kette überbrückt ist, die keine Heteroatome enthält oder 1 bis 2 N- Atome, 0 oder 1 N-Atom und 1 O-Atom oder 1 S-Atom, 0 oder 1 N-Atom und 2 O-Atome oder 2 S-Atome oder 0 oder 1 N-Atom und 1 O-Atom und 1 S-Atom enthält,
- wobei die Brücke unsubstituiert ist oder substituiert mit 1 bis 3, im Fall von Halogen auch bis zur maximal möglichen Anzahl, Substituenten aus der Gruppe Halogen, Cyano, Nitro, C₁-C₆-Alkyl, C₃-C₆-Alkenyl, C₃-C₆-Alkinyl, C₁-C₆-Halogenalkyl, C₃-C₆-Halogenalkenyl, C₃-C₆- Halogenalkinyl, Hydroxy, C₁-C₆-Alkoxy, C₁-C₆-Halogenalkoxy und C₁- C₆-Alkylsulfonyl.

Ebenso bevorzugt sind die heteroaroyl-substituierten Serin-Amide der Formel (I), in der
- R⁷: C₁-C₆-Alkyl, C₁-C₆-Halogenalkyl oder Phenyl,
wobei der Phenylrest partiell oder teilweise halogeniert sein kann und/oder durch C₁-C₄-Alkyl substituiert sein kann; ,
besonders bevorzugt C₁-C₄-Alkyl, C₁-C₄-Halogenalkyl oder Phenyl;
insbesondere bevorzugt Methyl, Trifluormethyl oder Phenyl,
bedeutet.

Besonders bevorzugt sind die heteroaroyl-substituierten Serin-Amide der Formel (I), in der
- A: 5- oder 6-gliedriges Heteroaryl ausgewählt aus der Gruppe Thienyl, Furyl, Pyra- zolyl, Imidazolyl, Thiazolyl, Oxazolyl und Pyridyl; wobei die
genannten Heteroarylreste partiell oder vollständig halogeniert sein kön- nen und/oder 1 bis 3 Reste aus der Gruppe C₁-C₆-Alkyl, C₃-C₆-Cycloalkyl und C₁-C₆-Halogenalkyl tragen können;
- R¹ und R²: Wasserstoff;
- R³: C₁-C₄-Alkyl,
besonders bevorzugt CH₃;
- R⁴: Wasserstoff, Formyl, C₁-C₄-Alkylcarbonyl, C₁-C₄-Alkylaminocarbonyl, Di-(C₁-C₄- Alkyl)-aminocarbonyl, Phenylaminocarbonyl, N-(C₁-C₄-alkyl)-N-(phenyl)-amino- carbonyl, SO₂CH₃, SO₂CF₃ oder SO₂(C₆H₅);
- R⁵ und R⁶: zusammen mit dem Kohlenstoffatom, an das sie gebunden sind, einen 3 bis 7-gliedrigen, gesättigten oder partiell ungesättigten Ring, der carbocyclisch ist oder 1 oder 2 N-Atome, 0 oder 1 N-Atom und 1 O-Atom oder S-Atom, 0 oder 1 N-Atom und 1 O- und 1 S-Atom, 2 O- oder S-Atome enthält,
wobei der Ring unsubstituiert ist oder substituiert ist durch 1 bis 3, im Fall von Halogen auch bis zur maximal möglichen Anzahl, Substituenten aus der Gruppe Halogen, C₁-C₆-Alkyl, Hydroxy, C₁-C₆-Alkoxy, Formyl, C₁-C₆- Alkyl-carbonyl, C₁-C₆-Alkoxycarbonyl, C₁-C₆-Alkylaminocarbonyl, Di-(C₁- C₆-alkyl)-aminocarbonyl, Alkylsulfonylamino, Carbonyl, Alkoxyimino, wobei die genannten Alkyl- und Alkoxyreste partiell oder vollständig halogeniert sein können und/oder eine bis drei der folgenden Grup- pen tragen können: Cyano, Hydroxy, C₁-C₄-Alkoxy,
Phenyl, partiell oder vollständig halogeniert,
und der Ring monocyclisch ist oder anelliert mit einem weiteren 3 bis 6-gliedrigen gesättigten oder partiell ungesättigten Ring, der carbocyclisch ist oder 1 oder 2 N-Atome, 0 oder 1 N-Atom und 1 O-Atom oder S-Atom, 2 O-Atome oder S- Atome, 0 oder 1 N-Atom und 1 O-Atom und 1 S-Atom enthält,
wobei der anellierte Ring unsubstituiert ist oder substituiert durch 1 bis 3, im Fall von Halogen auch bis zur maximal möglichen Anzahl, Substituenten aus der Gruppe Halogen, C₁-C₆-Alkyl, C₁-C₆- Halogenalkyl und C₁-C₆-Alkoxy,
oder der Ring ist mit einer 1 bis 3-zähligen gesättigten oder ungesättigten Kette überbrückt, die keine Heteroatome enthält oder 1 N-Atom, 0 oder 1 N-Atom und 1 O-Atom oder 1 S-Atom enthält,
wobei die Brücke unsubstituiert ist oder substituiert mit 1 bis 3, im Fall von Halogen auch bis zur maximal möglichen Anzahl. Substituenten aus der Gruppe Halogen, C₁-C₆-Alkyl, Hydroxy und C₁-C₆-Alkoxy,
bedeuten.

Außerordentlich bevorzugt sind die Verbindungen der Formel (I.a) (entspricht Formel (I) mit A = A-1 mit R⁸ = H, R⁹ = CF₃, R¹, R² und R⁵ = H; R³ = CH₃), insbesondere die Verbindungen der Formel I.a.1 bis I.a.138 der Tabelle 1, wobei die Definitionen der Variablen A und R¹ bis R⁶ nicht nur in Kombination miteinander, sondern auch jeweils für sich allein betrachtet für die erfindungsgemäßen Verbindungen eine besondere Rolle spielen.

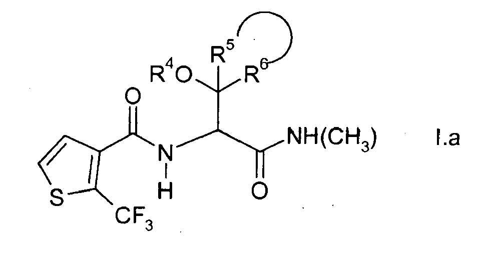

**Tabelle 1**

| Nr. | R⁴ | R⁵+R⁶ |
|---|---|---|
| I.a.1 | H | -CH₂-CH₂- |
| I.a.2 | H | -CH₂-CH₂-CH₂- |
| I.a.3 | H | -CH₂-O-CH₂- |
| I.a.4 | H | -CH₂-CH₂-CH₂-CH₂- |
| I.a.5 | H | -CHCH₃-CH₂-CH₂-CHCH₃- |
| I.a.6 | H | -CH₂-CH₂-CH₂-CHCH₃- |
| I.a.7 | H | -CH₂-CH₂-CH₂-CH(OCH₃)- |
| I.a.8 | H | -CH₂-CH₂-O-CH₂- |
| I.a.9 | H | -CH₂-CH₂-O-CHCH₃- |
| I.a.10 | H | -CH₂-CH₂-O-CH(OCH₃)- |
| I.a.11 | H | -CH₂-O-CH₂-CHCH₃- |
| I.a.12 | H | -CH₂-O-CH₂-CH(OCH₃)- |
| I.a.13 | H | -CH₂-CH₂-N(COH)-CH₂- |
| I.a.14 | H | -CH₂-CH₂-N(COCH₃)-CH₂- |
| I.a.15 | H | -CH₂-CH₂-N(COOCH₃)-CH₂- |
| I.a.16 | H | -CH₂-CH₂-N(CONHCH₃)-CH₂- |
| I.a.17 | H | -CH₂-CH₂-N(CON(CH₃)₂)-CH₂- |
| I.a.18 | H | -CH₂-CH₂-CH₂-CH₂-CH₂- |
| I.a.19 | H | -CH₂-CH₂-CH₂-CH₂-CHCH₃- |
| I.a.20 | H | -CH₂-CH₂-CH₂-CH₂-CH(OCH₃)- |
| I.a.21 | H | -CH₂-CH₂-CH₂-O-CH₂- |
| I.a.22 | H | -CH₂-CH₂-CH₂-O-CHCH₃- |
| I.a.23 | H | -CH₂-CH₂-CH₂-O-CH(OCH₃)- |
| I.a.24 | H | -CH₂-CH₂-O-CH₂-CH₂- |
| I.a.25 | H | -CH₂-CH₂-O-CH₂-CHCH₃- |
| I.a.26 | H | -CH₂-CH₂-CH₂-O-CH(OCH₃)- |
| I.a.27 | H | -CH₂-O-CH₂-CH₂-CH₂- |
| I.a.28 | H | -CH₂-O-CH₂-CH₂-CHCH₃- |
| I.a.29 | H | -CH₂-O-CH₂-CH₂-CH(OCH₃)- |
| I.a.30 | H | -CH₂-O-CH₂-O-CH₂- |
| I.a.31 | H | -CH₂-CH₂-CH₂-N(COH)-CH₂- |
| I.a.32 | H | -CH₂-CH₂-CH₂-N(COCH₃)-CH₂- |
| I.a.33 | H | -CH₂-CH2-CH₂-N(COOCH₃)-CH₂- |
| I.a.34 | H | -CH₂-CH₂-CH₂-N(CONHCH₃)-CH₂- |
| I.a.35 | H | -CH₂-CH₂-CH₂-N(CON(CH₃)₂)-CH₂- |
| I.a.36 | H | -CH₂-CH₂-N(COH)-CH₂-CH₂- |
| I.a.37 | H | -CH₂-CH₂-N(COCH₃)-CH₂-CH₂- |
| I.a.38 | H | -CH₂-CH₂-N(COOCH₃)-CH₂-CH₂- |
| I.a.39 | H | -CH₂-CH₂-N(CONHCH₃)-CH₂-CH₂- |
| I.a.40 | H | -CH₂-CH₂-N(CON(CH₃)₂)-CH₂-CH₂- |
| I.a.41 | H | -CH₂-CH₂-CH₂-CH₂-CH₂-CH₂- |
| I.a.42 | H | -CH₂-O-CH₂-CH₂-O-CH₂- |
| I.a.43 | C(O)CH₃ | -CH₂-CH₂- |
| I.a.44 | C(O)CH₃ | -CH₂-CH₂-CH₂- |
| I.a.45 | C(O)CH₃ | -CH₂-O-CH₂- |
| I.a.46 | C(O)CH₃ | -CH₂-CH₂-CH₂-CH₂- |
| I.a.47 | C(O)CH₃ | -CHCH₃-CH₂-CH₂-CHCH₃- |
| I.a.48 | C(O)CH₃ | -CH₂-CH₂-CH₂-CHCH₃- |
| I.a.49 | C(O)CH₃ | -CH₂-CH₂-CH₂-CH(OCH₃)- |
| I.a.50 | C(O)CH₃ | -CH₂-CH₂-O-CH₂- |
| I.a.51 | C(O)CH₃ | -CH₂-CH₂-O-CHCH₃- |
| I.a.52 | C(O)CH₃ | -CH₂-CH₂-O-CH(OCH₃)- |
| I.a.53 | C(O)CH₃ | -CH₂-O-CH₂-CHCH₃- |
| I.a.54 | C(O)CH₃ | -CH₂-O-CH₂-CH(OCH₃)- |
| I.a.55 | C(O)CH₃ | -CH₂-CH₂-N(COH)-CH₂- |
| I.a.56 | C(O)CH₃ | -CH₂-CH₂-N(COCH₃)-CH₂- |
| I.a.57 | C(O)CH₃ | -CH₂-CH₂-N(COOCH₃)-CH₂- |
| I.a.58 | C(O)CH₃ | -CH₂-CH₂-N(CONHCH₃)-CH₂- |
| I.a.59 | C(O)CH₃ | -CH₂-CH₂-N(CON(CH₃)₂)-CH₂- |
| I.a.60 | C(O)CH₃ | -CH₂-CH₂-CH₂-CH₂-CH₂- |
| I.a.61 | C(O)CH₃ | -CH₂-CH₂-CH₂-CH₂-CHCH₃- |
| I.a.62 | C(O)CH₃ | -CH₂-CH₂-CH₂-CH₂-CH(OCH₃)- |
| I.a.63 | C(O)CH₃ | -CH₂-CH₂-CH₂-O-CH₂- |
| I.a.64 | C(O)CH₃ | -CH₂-CH₂-CH₂-O-CHCH₃- |
| I.a.65 | C(O)CH₃ | -CH₂-CH₂-CH₂-O-CH(OCH₃)- |
| I.a.66 | C(O)CH₃ | -CH₂-CH₂-O-CH₂-CH₂- |
| I.a.67 | C(O)CH₃ | -CH₂-CH₂-O-CH₂-CHCH₃- |
| I.a.68 | C(O)CH₃ | -CH₂-CH₂-CH₂-O-CH(OCH₃)- |
| I.a.69 | C(O)CH₃ | -CH₂-O-CH₂-CH₂-CH₂- |
| I.a.70 | C(O)CH₃ | -CH₂-O-CH₂-CH₂-CHCH₃- |
| I.a.71 | C(O)CH₃ | -CH₂-O-CH₂-CH₂-CH(OCH₃)- |
| I.a.72 | C(O)CH₃ | -CH₂-O-CH₂-O-CH₂- |
| I.a.73 | C(O)CH₃ | -CH₂-CH₂-CH₂-N(COH)-CH₂- |
| I.a.74 | C(O)CH₃ | -CH₂-CH₂-CH₂-N(COCH₃)-CH₂- |
| I.a.75 | C(O)CH₃ | -CH₂-CH2-CH₂-N(COOCH₃)-CH₂- |
| I.a.76 | C(O)CH₃ | -CH₂-CH₂-CH₂-N(CONHCH₃)-CH₂- |
| I.a.77 | C(O)CH₃ | -CH₂-CH₂-CH₂-N(CON(CH₃)₂)-CH₂- |
| I.a.78 | . C(O)CH₃ | -CH₂-CH₂-N(COH)-CH₂-CH₂- |
| I.a.79 | C(O)CH₃ | -CH₂-CH₂-N(COCH₃)-CH₂-CH₂- |
| I.a.80 | C(O)CH₃ | -CH₂-CH₂-N(COOCH₃)-CH₂-CH₂- |
| I.a.81 | C(O)CH₃ | -CH₂-CH₂-N(CONHCH₃)-CH₂-CH₂- |
| I.a.82 | C(O)CH₃ | -CH₂-CH₂-N(CON(CH₃)₂)-CH₂-CH₂- |
| I.a.83 | C(O)CH₃ | -CH₂-CH₂-CH₂-CH₂-CH₂-CH₂- |
| I.a.84 | C(O)CH₃ | -CH₂-O-CH₂-CH₂-O-CH₂- |
| I.a.85 | C(O)NHCH₃ | -CH₂-CH₂- |
| I.a.86 | C(O)NHCH₃ | -CH₂-CH₂-CH₂- |
| I.a.87 | C(O)NHCH₃ | -CH₂-O-CH₂- |
| I.a.88 | C(O)NHCH₃ | -CH₂-CH₂-CH₂-CH₂- |
| I.a.89 | C(O)NHCH₃ | -CHCH₃-CH₂-CH₂-CHCH₃- |
| I.a.90 | C(O)NHCH₃ | -CH₂-CH₂-CH₂-CHCH₃- |
| I.a.91 | C(O)NHCH₃ | -CH₂-CH₂-CH₂-CH(OCH₃)- |
| I.a.92 | C(O)NHCH₃ | -CH₂-CH₂-O-CH₂- |
| I.a.93 | C(O)NHCH₃ | -CH₂-CH₂-O-CHCH₃- |
| I.a.94 | C(O)NHCH₃ | -CH₂-CH₂-O-CH(OCH₃)- |
| I.a.95 | C(O)NHCH₃ | -CH₂-O-CH₂-CHCH₃- |
| I.a.96 | C(O)NHCH₃ | -CH₂-O-CH₂-CH(OCH₃)- |
| I.a.97 | C(O)NHCH₃ | -CH₂-CH₂-N(COH)-CH₂- |
| I.a.98 | C(O)NHCH₃ | -CH₂-CH₂-N(COCH₃)-CH₂- |
| I.a.99 | C(O)NHCH₃ | -CH₂-CH₂-N(COOCH₃)-CH₂- |
| I.a.100 | C(O)NHCH₃ | -CH₂-CH₂-N(CONHCH₃)-CH₂- |
| I.a.101 | C(O)NHCH₃ | CH₂-CH₂-N(CON(CH₃)₂)-CH₂- |
| I.a.102 | C(O)NHCH₃ | -CH₂-CH₂-CH₂-CH₂-CH₂- |
| I.a.103 | C(O)NHCH₃ | -CH₂-CH₂-CH₂-CH₂-CHCH₃- |
| I.a.104 | C(O)NHCH₃ | -CH₂-CH₂-CH₂-CH₂-CH(OCH₃)- |
| I.a.105 | C(O)NHCH₃ | -CH₂-CH₂-CH₂-O-CH₂- |
| I.a.106 | C(O)NHCH₃ | -CH₂-CH₂-CH₂-O-CHCH₃- |
| I.a.107 | C(O)NHCH₃ | -CH₂-CH₂-CH₂-O-CH(OCH₃)- |
| I.a.108 | C(O)NHCH₃ | -CH₂-CH₂-O-CH₂-CH₂- |
| I.a.109 | C(O)NHCH₃ | -CH₂-CH₂-O-CH₂-CHCH₃- |
| I.a.110 | C(O)NHCH₃ | -CH₂-CH₂-CH₂-O-CH(OCH₃)- |
| I.a.111 | C(O)NHCH₃ | -CH₂-O-CH₂-CH₂-CH₂- |
| I.a.112 | C(O)NHCH₃ | -CH₂-O-CH₂-CH₂-CHCH₃- |
| I.a.113 | C(O)NHCH₃ | -CH₂-O-CH₂-CH₂-CH(OCH₃)- |
| I.a.114 | C(O)NHCH₃ | -CH₂-O-CH₂-O-CH₂- |
| I.a.115 | C(O)NHCH₃ | -CH₂-CH₂-CH₂-N(COH)-CH₂- |
| I.a.116 | C(O)NHCH₃ | -CH₂-CH₂-CH₂-N(COCH₃)-CH₂- |
| I.a.117 | C(O)NHCH₃ | -CH₂-CH2-CH₂-N(COOCH₃)-CH₂- |
| I.a.118 | C(O)NHCH₃ | -CH₂-CH₂-CH₂-N(CONHCH₃)-CH₂- |
| I.a.119 | C(O)NHCH₃ | -CH₂-CH₂-CH₂-N(CON(CH₃)₂)-CH₂- |
| I.a.120 | C(O)NHCH₃ | -CH₂-CH₂-N(COH)-CH₂-CH₂- |
| I.a.121 | C(O)NHCH₃ | -CH₂-CH₂-N(COCH₃)-CH₂-CH₂- |
| I.a.122 | C(O)NHCH₃ | -CH₂-CH₂-N(COOCH₃)-CH₂-CH₂- |
| I.a.123 | C(O)NHCH₃ | -CH₂-CH₂-N(CONHCH₃)-CH₂-CH₂- |
| I.a.124 | C(O)NHCH₃ | -CH₂-CH₂-N(CON(CH₃)₂)-CH₂-CH₂- |
| I.a.125 | C(O)NHCH₃ | -CH₂-CH₂-CH₂-CH₂-CH₂-CH₂- |
| I.a.126 | C(O)NHCH₃ | -CH₂-O-CH₂-CH₂-O-CH₂- |
| I.a.127 | CON(CH₃)₂ | -CH₂-CH₂- |
| I.a.128 | CON(CH₃)₂ | -CH₂-CH₂-CH₂- |
| I.a.129 | CON(CH₃)₂ | -CH₂-O-CH₂- |
| I.a.130 | CON(CH₃)₂ | -CH₂-CH₂-CH₂-CH₂- |
| I.a.131 | CON(CH₃)₂ | -CHCH₃-CH₂-CH₂-CHCH₃- |
| I.a.132 | CON(CH₃)₂ | -CH₂-CH₂-CH₂-CHCH₃- |
| I.a.133 | CON(CH₃)₂ | -CH₂-CH₂-CH₂-CH(OCH₃)- |
| I.a.134 | CON(CH₃)₂ | -CH₂-CH₂-O-CH₂- |
| I.a.135 | CON(CH₃)₂ | -CH₂-CH₂-O-CHCH₃- |
| I.a.136 | CON(CH₃)₂ | -CH₂-CH₂-O-CH(OCH₃)- |
| I.a.137 | CON(CH₃)₂ | -CH₂-O-CH₂-CHCH₃- |
| I.a.138 | CON(CH₃)₂ | -CH₂-O-CH₂-CH(OCH₃)- |
| I.a.139 | CON(CH₃)₂ | -CH₂-CH₂-N(COH)-CH₂- |
| I.a.140 | CON(CH₃)₂ | -CH₂-CH₂-N(COCH₃)-CH₂- |
| I.a.141 | CON(CH₃)₂ | -CH₂-CH₂-N(COOCH₃)-CH₂- |
| I.a.142 | CON(CH₃)₂ | -CH₂-CH₂-N(CONHCH₃)-CH₂- |
| I.a.143 | CON(CH₃)₂ | -CH₂-CH₂-N(CON(CH₃)₂)-CH₂- |
| I.a.144 | CON(CH₃)₂ | -CH₂-CH₂-CH₂-CH₂-CH₂- |
| I.a.145 | CON(CH₃)₂ | -CH₂-CH₂-CH₂-CH₂-CHCH₃- |
| I.a.146 | CON(CH₃)₂ | -CH₂-CH₂-CH₂-CH₂-CH(OCH₃)- |
| I.a.147 | CON(CH₃)₂ | -CH₂-CH₂-CH₂-O-CH₂- |
| I.a.148 | CON(CH₃)₂ | -CH₂-CH₂-CH₂-O-CHCH₃- |
| I.a.149 | CON(CH₃)₂ | -CH₂-CH₂-CH₂-O-CH(OCH₃)- |
| I.a.150 | CON(CH₃)₂ | -CH₂-CH₂-O-CH₂-CH₂- |
| I.a.151 | CON(CH₃)₂ | -CH₂-CH₂-O-CH₂-CHCH₃- |
| I.a.152 | CON(CH₃)₂ | -CH₂-CH₂-CH₂-O-CH(OCH₃)- |
| I.a.153 | CON(CH₃)₂ | -CH₂-O-CH₂-CH₂-CH₂- |
| I.a.154 | CON(CH₃)₂ | -CH₂-O-CH₂-CH₂-CHCH₃- |
| I.a.155 | CON(CH₃)₂ | -CH₂-O-CH₂-CH₂-CH(OCH₃)- |
| I.a.156 | CON(CH₃)₂ | -CH₂-O-CH₂-O-CH₂- |
| I.a.157 | CON(CH₃)₂ | -CH₂-CH₂-CH₂-N(COH)-CH₂- |
| I.a.158 | CON(CH₃)₂ | -CH₂-CH₂-CH₂-N(COCH₃)-CH₂- |
| I.a.159 | CON(CH₃)₂ | -CH₂-CH2-CH₂-N(COOCH₃)-CH₂- |
| I.a.160 | CON(CH₃)₂ | -CH₂-CH₂-CH₂-N(CONHCH₃)-CH₂- |
| I.a.161 | CON(CH₃)₂ | -CH₂-CH₂-CH₂-N(CON(CH₃)₂)-CH₂- |
| I.a.162 | CON(CH₃)₂ | -CH₂-CH₂-N(COH)-CH₂-CH₂- |
| I.a.163 | CON(CH₃)₂ | -CH₂-CH₂-N(COCH₃)-CH₂-CH₂- |
| I.a.164 | CON(CH₃)₂ | -CH₂-CH₂-N(COOCH₃)-CH₂-CH₂- |
| I.a.165 | CON(CH₃)₂ | -CH₂-CH₂-N(CONHCH₃)-CH₂-CH₂- |
| I.a.166 | CON(CH₃)₂ | -CH₂-CH₂-N(CON(CH₃)₂)-CH₂-CH₂- |
| I.a.167 | CON(CH₃)₂ | -CH₂-CH₂-CH₂-CH₂-CH₂-CH₂- |
| I.a.168 | CON(CH₃)₂ | -CH₂-O-CH₂-CH₂-O-CH₂- |

Ebenso außerordentlich bevorzugt sind die Verbindungen der Formel I.b, insbesondere die Verbindungen der Formel I.b.1 bis I.b. 168, die sich von den entsprechenden Verbindungen der Formel I.a.1 bis I.a.168 dadurch unterscheiden, dass A für A1 mit R⁸ = CH₃ und R⁹ = CF₃ steht:

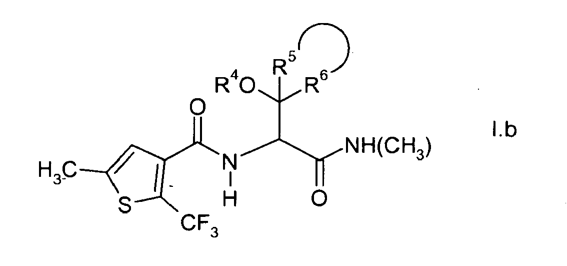

Ebenso außerordentlich bevorzugt sind die Verbindungen der Formel I.c, insbesondere die Verbindungen der Formel I.c.1 bis I.c.168, die sich von den entsprechenden Verbindungen der Formel I.a.1 bis I.a.168 dadurch unterscheiden, dass A für A2 mit R⁸ = H und R⁹ = CF₃ steht:

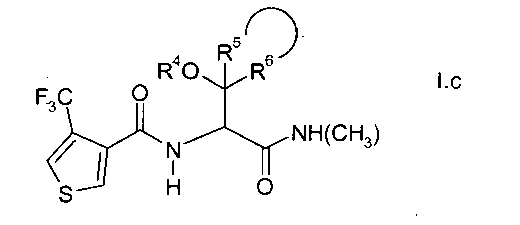

Ebenso außerordentlich bevorzugt sind die Verbindungen der Formel I.d, insbesondere die Verbindungen der Formel I.d.1 bis I.d.168, die sich von den entsprechenden Verbindungen der Formel I.a.1 bis I.a.168 dadurch unterscheiden, dass A für A3 mit R⁸ = H und R⁹ = CF₃ steht:

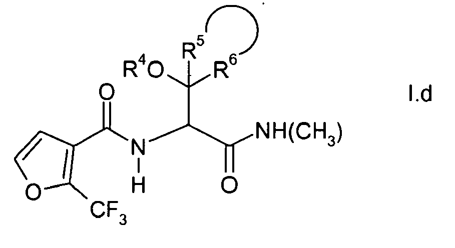

Ebenso außerordentlich bevorzugt sind die Verbindungen der Formel I.e, insbesondere die Verbindungen der Formel I.e.1 bis I.e.168, die sich von den entsprechenden Verbindungen der Formel I.a.1 bis I.a.168 dadurch unterscheiden, dass A für A3 mit R⁸ = CH₃ und R⁹ = CF₃ steht:

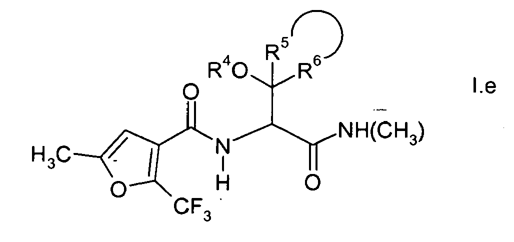

Ebenso außerordentlich bevorzugt sind die Verbindungen der Formel I.f, insbesondere die Verbindungen der Formel I.f.1 bis I.f.168, die sich von den entsprechenden Verbindungen der Formel I.a.1 bis I.a.168 dadurch unterscheiden, dass A für A4 mit R⁸ = H und R⁹ = CF₃ steht:

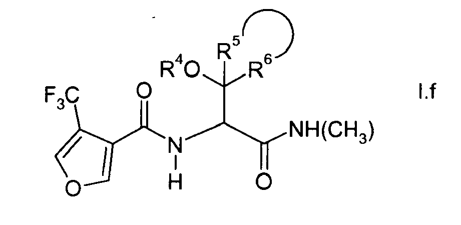

Ebenso außerordentlich bevorzugt sind die Verbindungen der Formel I.g, insbesondere die Verbindungen der Formel I.g.1 bis I.g.168, die sich von den entsprechenden Verbindungen der Formel I.a.1 bis I.a.168 dadurch unterscheiden, dass A für A5 mit R¹¹ = H, R⁹ = CF₃ und R¹⁰ = H steht:

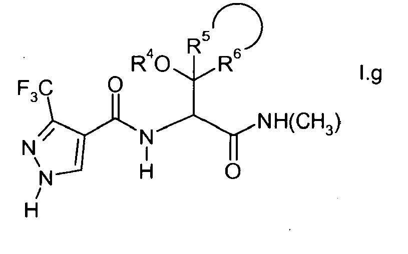

Ebenso außerordentlich bevorzugt sind die Verbindungen der Formel I.h, insbesondere die Verbindungen der Formel I.h.1 bis I.h.168, die sich von den entsprechenden Verbindungen der Formel I.a.1 bis I.a.168 dadurch unterscheiden, dass A für A5 mit R¹¹ = CH₃, R⁹ = CF₃ und R¹⁰ = H steht:

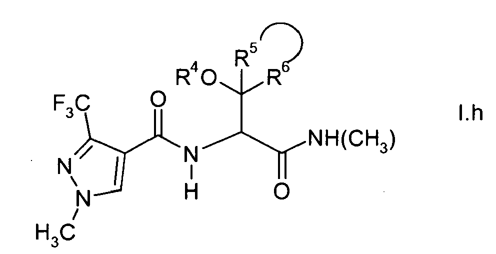

Ebenso außerordentlich bevorzugt sind die Verbindungen der Formel I.j, insbesondere die Verbindungen der Formel I.j.1 bis I.j.168, die sich von den entsprechenden Verbindungen der Formel I.a.1 bis I.a.168 dadurch unterscheiden, dass A für A8 mit R⁸ = H und R⁹ = CF₃ steht:

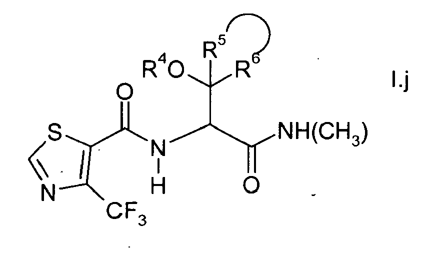

Ebenso außerordentlich bevorzugt sind die Verbindungen der Formel I.k, insbesondere die Verbindungen der Formel I.k.1 bis I.k.168, die sich von den entsprechenden Verbindungen der Formel I.a.1 bis I.a.168 dadurch unterscheiden, dass A für A8 mit R⁸ = CH₃ und R⁹ = CF₃ steht:

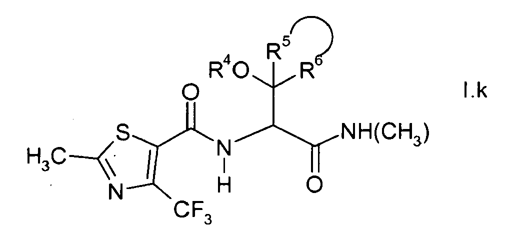

Die heteroaroyl-substituierten Serin-Amide der Formel (I) sind auf verschiedene Art und Weise erhältlich, beispielsweise nach folgenden Verfahren:

### Verfahren A

Serinderivate der Formel (V) werden zunächst mit Heteroarylsäure(derivate)n der Formel (IV) zu entsprechenden Heteroaroylderivaten der Formel (III) umgesetzt, welche anschließend mit Aminen der Formel (II) zu den gewünschten heteroaroyl-substituierten Serin-Amiden der Formel (I) reagieren:

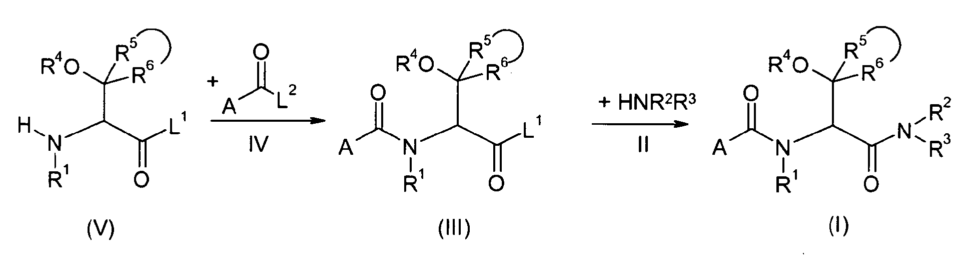

L¹ steht für eine nucleophil verdrängbare Abgangsgruppe, z.B. für Hydroxy oder C₁-C₆-Alkoxy.

L² steht für eine nucleophil verdrängbare Abgangsgruppe, z.B. für Hydroxy, Halogen, C₁-C₆-Alkylcarbonyl, C₁-C₆-Alkoxycarbonyl, C₁-C₄-Alkylsulfonyl, Phosphoryl oder Isoureyl.

Die Umsetzung der Serinderivate der Formel (V) mit Heteroarylsäure(derivate)n der Formel (IV), wobei L² für Hydroxy steht, zu Heteroaroylderivaten der Formel (III) erfolgt in Gegenwart eines Aktivierungsreagenz und einer Base üblicherweise bei Temperaturen von 0 °C bis zum Siedepunkt des Reaktionsgemisches, vorzugsweise 0°C bis 110°C, besonders bevorzugt bei Raumtemperatur, in einem inerten organischen Lösungsmittel [vgl. Bergmann, E. D.; et al., J Chem Soc 1951, 2673; Zhdankin, V. V.; et al., Tetrahedron Lett. 2000, 41 (28), 5299-5302; Martin, S. F. et al., Tetrahedron Lett.1998, 39 (12), 1517-1520; Jursic, B. S. et al., Synth Commun 2001, 31 (4), 555-564; Albrecht, M. et al., Synthesis 2001, (3), 468-472; Yadav, L. D. S. et al., Indian J. Chem B. 41(3),593-595(2002); Clark, J. E. et al., Sythesis (10),891-894 (1991)].

Geeignete Aktivierungsreagenzien sind Kondensationsmittel wie z.B. polystyrolgebundenes Dicyclohexylcarbodiimid, Diisopropylcarbodiimid, Carbonyldiimidazol, Chlorkohlensäureester wie Methylchloroformiat, Ethylchloroformiat, Isoropylchloroformiat, Isobutylchloroformiat, sec-Butylchloroformiat oder Allylchloroformiat, Pivaloylchlorid, Polyphosphorsäure, Propanphosphonsäureanhydrid, Bis(2-oxo-3-oxazolidinyl)-phosphorylchlorid (BOPCI) oder Sulfonylchloride wie Methansulfonylchlorid, Toluolsulfonylchlorid oder Benzolsulfonylchlorid.

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Benzol, Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran (THF), Nitrile wie Acetonitril und Propionitril, Ketone wie Aceton, Methylethylketon, Diethylketon und tert.-Butylmethylketon, sowie Dimethylsulfoxid, Dimethylformamid (DMF), Dimethylacetamid (DMA) und N-Methylpyrrolidon (NMP) oder auch in Wasser, besonders bevorzugt sind Methylenchlorid, THF und Wasser.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, außerdem organische Basen, z.B. tertiäre Amine wie Trimethylamin, Triethylamin, Diisopropylethylamin, N-Methylmorpholin, und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Triethylamin und Pyridin.

Die Basen werden im Allgemeinen in äquimolaren Mengen eingesetzt. Sie können aber auch im Überschuss oder gegebenenfalls als Lösungsmittel verwendet werden.

Die Edukte werden im Allgemeinen in äquimolaren Mengen miteinander umgesetzt. Es kann vorteilhaft sein, (IV) in einem Überschuss bezogen auf (V) einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z. T. in Form zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Umsetzung der Serinderivate der Formel (V) mit Heteroarylsäure(derivate)n der Formel (IV), wobei L² für Halogen, C₁-C₆-Alkylcarbonyl, C₁-C₆-Alkoxycarbonyl, C₁-C₄-Alkylsulfonyl, Phosphoryl oder Isoureyl steht, zu Heteroaroylderivaten der Formel (III) erfolgt in Gegenwart einer Base üblicherweise bei Temperaturen von 0°C bis zum Siedepunkt des Reaktionsgemisches, vorzugsweise 0°C bis 100°C, besonders bevorzugt bei Raumtemperatur in einem inerten organischen Lösungsmittel [vgl. Bergmann, E. D.; et al., J Chem Soc 1951, 2673; Zhdankin, V. V.; et al., Tetrahedron Lett. 2000, 41 (28), 5299-5302; Martin, S. F. et al., Tetrahedron Lett.1998, 39 (12), 1517-1520; Jursic, B. S. et al., Synth Commun 2001, 31 (4), 555-564; Albrecht, M. et al., Synthesis 2001, (3), 468-472; Yadav, L. D. S. et al., Indian J. Chem B. 41(3),593-595(2002); Clark, J. E. et al., Synthesis (10),891-894 (1991)].

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Benzol, Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran (THF), Nitrile wie Acetonitril und Propionitril, Ketone wie Aceton, Methylethylketon, Diethylketon und tert.-Butylmethylketon, sowie Dimethylsulfoxid, Dimethylformamid (DMF), Dimethylacetamid (DMA) und N-Methylpyrrolidon (NMP) oder auch in Wasser, besonders bevorzugt sind Methylenchlorid, THF und Wasser.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, außerdem organische Basen, z.B. tertiäre Amine wie Trimethylamin, Triethylamin, Diisopropylethylamin, N-Methylmorpholin, und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Triethylamin und Pyridin.

Die Basen werden im Allgemeinen in äquimolaren Mengen eingesetzt. Sie können aber auch im Überschuss oder gegebenenfalls als Lösungsmittel verwendet werden.

Die Edukte werden im Allgemeinen in äquimolaren Mengen miteinander umgesetzt. Es kann vorteilhaft sein, (IV) in einem Überschuss bezogen auf (V) einzusetzen.

Die Aufarbeitung und Isolierung der Produkte kann in an sich bekannter Weise erfolgen.

Natürlich können auch in analoger Weise zunächst die Serinderivate der Formel (V) mit Aminen der Formel (II) zu den entsprechenden Amiden umgesetzt werden, welche dann mit Heteroarylsäure(derivate)n der Formel (IV) zu den gewünschten heteroaroyl-substituierten Serin-Amiden der Formel (I) reagieren.

Die für die Herstellung der Heteroaroylderivate der Formel (III) benötigten Serinderivate der Formel (V) (z.B. mit L¹ = Hydroxy oder C₁-C₆-Alkoxy) sind, auch in enantiomeren- und diastereomerenreiner Form, in der Literatur bekannt oder können gemäß der zitierten Literatur hergestellt werden:
- durch Kondensation von Glycinenolat-Equivalenten mit cyclischen Ketonen [Blaser, D. et al., Liebigs Ann. Chem. 10, 1067-1078 (1991); Seethaler, T. et al., Liebigs Ann. Chem. 1, 11-17 (1991); Weltenauer, G. et al., Gazz. Chim. Ital. 81,162 (1951); Dalla Croce, P. et al., Heterocycles 52(3), 1337-1344 (2000); Van der Werf, A. W. et al., J. Chem. Soc. Chem. Commun. 100, 682-683 (1991); Caddick, S. et al., Tetrahedron 57 (30), 6615-6626 (2001); Owa, T. et al., Chem. Lett. 1, 83-86 (1988); Alker, D. et al., Tetrahedron 54 (22), 6089-6098 (1998); Rousseau, J. F. et al., J. Org. Chem. 63 (8), 2731-2737 (1998); Saeed, A. et al., Tetrahedron 48 (12), 2507-2514 (1992); Dong, L. et al., J. Org. Chem. 67 (14), 4759-4770 (2002)].
- durch Aminohydroxylierung von Acrylsäure-Derivaten [Zhang, H. X. et al., Tetrahedron Asymmetr. 11(16), 3439-3447 (2000); Fokin, V. V. et al., Angew. Chem. Int. Edit. 40(18), 3455 (2001); Sugiyama, H. et al., Tetrahedron Lett. 43(19), 3489-3492 (2002); Bushey, M. L. et al., J. Org. Chem. 64(9), 2984-2985 (1999); Raatz, D. et al., Synlett (12), 1907-1910 (1999)].
- durch nukleophile Substitution von Abgangsgruppen in 2-Position von 3-Hydroxy-Propionsäurederivaten [Owa, T. et al., Chem. Lett. (11), 1873-1874 (1988); Boger, D. L. et al., J. Org. Chem. 57(16), 4331-4333 (1992); Alcaide, B. et al., Tetrahedron Lett. 36(30), 5417-5420 (1995)].
- durch Kondensation von Ketonen mit Nukleophilen unter Ausbildung von Oxazolinen sowie anschließender Hydrolyse [Evans, D. A. et al., Angew. Chem. Int. Edit. 40(10), 1884-1888 (2001); Ito, Y. et al., Tetrahedron Lett. 26(47), 5781-5784 (1985); Togni, A. et al., J. Organomet. Chem. 381(1), C21-5 (1990); Longmire, J. M. et al., Organometallics 17(20), 4374-4379 (1998); Suga, H. et al., J. Org. Chem. 58(26), 7397-7405 (1993)].
- durch oxidative Cyclisierung von 2-Acylamino-Propionsäurederivaten zu Oxazolinen sowie anschließende Hydrolyse (JP10101655).

Die für die Herstellung der Heteroaroylderivate der Formel (III) benötigten Heteroarylsäure(derivate) der Formel (IV) können käuflich erworben werden oder können analog zu literaturbekannten Vorschrift über eine Grignard-Reaktion aus dem entsprechenden Halogenid hergestellt werden [z.B. A. Mannschuk et al., Angew. Chem. 100, 299 (1988)].

Die Umsetzung der Heteroaroylderivate der Formel (III) mit L¹ = Hydroxy bzw. deren Salze mit Aminen der Formel (II) zu den gewünschten heteroaroyl-substituierten Serin-Amiden der Formel (I) erfolgt in Gegenwart eines Aktivierungsreagenz und gegebenenfalls in Gegenwart einer Base üblicherweise bei Temperaturen von 0°C bis zum Siedepunkt des Reaktionsgemisches, vorzugsweise 0°C bis 100°C, besonders bevorzugt bei Raumtemperatur in einem inerten organischen Lösungsmittel. [vgl. Perich, J. W., Johns, R. B., J. Org. Chem. 53 (17), 4103-4105 (1988); Somlai, C. et al., Synthesis (3), 285-287 (1992) ; Gupta, A. et al., J. Chem. Soc. Perkin Trans. 2, 1911 (1990); Guan et al., J. Comb. Chem. 2, 297 (2000)].

Geeignete Aktivierungsreagenzien sind Kondensationsmittel wie z.B. polystyrolgebundenes Dicyclohexylcarbodiimid, Diisopropylcarbodiimid, Carbonyldiimidazol, Chlorkohlensäureester wie Methylchloroformiat, Ethylchloroformiat, Isoropylchloroformiat, Isobutylchloroformiat, sec-Butylchloroformiat oder Allylchloroformiat, Pivaloylchlorid, Polyphosphorsäure, Propanphosphonsäureanhydrid, Bis(2-oxo-3-oxazolidinyl)-phosphorylchlorid (BOPCI) oder Sulfonylchloride wie Methansulfonylchlorid, Toluolsulfonylchlorid oder Benzolsulfonylchlorid.

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Benzol, Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran (THF), Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid (DMF), Dimethylacetamid (DMA) und N-Methylpyrrolidon (NMP) oder auch in Wasser, besonders bevorzugt sind Methylenchlorid, THF, Methanol, Ethanol und Wasser.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, außerdem organische Basen, z.B. tertiäre Amine wie Trimethylamin, Triethylamin, Diisopropylethylamin, N-Methylmorpholin, und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Triethylamin, Ethyldiisopropylamin, N-methylmorpholin und Pyridin.

Die Basen werden im Allgemeinen in katalytischen Mengen eingesetzt, sie können aber auch äquimolar, im Überschuss oder gegebenenfalls als Lösungsmittel verwendet werden.

Die Edukte werden im Allgemeinen in äquimolaren Mengen miteinander umgesetzt. Es kann vorteilhaft sein (II) in einem Überschuss bezogen auf (III) einzusetzen.

Die Aufarbeitung und Isolierung der Produkte kann in an sich bekannter Weise erfolgen.

Die Umsetzung der Heteroaroylderivate der Formel (III) mit L¹ = C₁-C₆-Alkoxy mit Aminen der Formel (II) zu den gewünschten heteroaroyl-substituierten Serin-Amiden der Formel (I) erfolgt üblicherweise bei Temperaturen von 0°C bis zum Siedepunkt des Reaktionsgemisches, vorzugsweise 0°C bis 100°C, besonders bevorzugt bei Raumtemperatur in einem inerten organischen Lösungsmittel gegebenenfalls in Gegenwart einer Base [vgl. Kawahata, N. H. et al., Tetrahedron Lett. 43 (40), 7221-7223 (2002); Takahashi, K. et al., J. Org. Chem. 50 (18), 3414-3415 (1985); Lee, Y. et al., J. Am. Chem. Soc. 121 (36), 8407-8408 (1999)].

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Benzol, Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran (THF), Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid (DMF), Dimethylacetamid (DMA) und N-Methylpyrrolidon (NMP) oder auch in Wasser, besonders bevorzugt sind Methylenchlorid, THF, Methanol, Ethanol und Wasser.

Es können auch Gemische der genannten Lösungsmittel verwendet werden.

Die Umsetzung kann gegebenenfalls in Gegenwart einer Base erfolgen. Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, außerdem organische Basen, z.B. tertiäre Amine wie Trimethylamin, Triethylamin, Diisopropylethylamin, N-Methylmorpholin, und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Triethylamin, Ethyldiisopropylamin, N-methylmorpholin und Pyridin.

Die Basen werden im Allgemeinen in katalytischen Mengen eingesetzt, sie können aber auch äquimolar, im Überschuss oder gegebenenfalls als Lösungsmittel verwendet werden.

Die Edukte werden im Allgemeinen in äquimolaren Mengen miteinander umgesetzt. Es kann vorteilhaft sein, (II) in einem Überschuss bezogen auf (III) einzusetzen.

Die Aufarbeitung und Isolierung der Produkte kann in an sich bekannter Weise erfolgen.

Die für die Herstellung der heteroaroyl-substituierten Serin-Amide der Formel (I) benötigten Amine der Formel (II) können käuflich erworben werden.

### Verfahren B

Heteroaroylderivate der Formel (III) mit R⁴ = Wasserstoff können auch erhalten werden, indem acylierte Glycin-Derivate der Formel (VIII), wobei die Acylgruppe eine abspaltbare Schutzgruppe wie Benzyloxycarbonyl (vgl. VIIIa mit Σ = Benzyl) oder tert.-Butyloxycarbonyl (vgl. (VIIIa) mit Σ = tert-Butyl) sein kann, mit Carbonylverbindungen (VII) zu entsprechenden Aldolprodukten (VI) kondensiert werden. Anschließend wird die Schutzgruppe abgespalten und das so entstandene Serinderivat der Formel (V) mit R⁴ = Wasserstoff mit Heteroarylsäurederivaten der Formel (IV) acyliert.

Analog kann auch ein acyliertes Glycin-Derivat der Formel (VIII), wobei die Acylgruppe ein substituierter Heteroaroylrest (vgl. VIIIb) ist, unter Baseneinfluss mit einer Carbonylverbindung VII zum Heteroaroylderivat III mit R⁴ = Wasserstoff umgesetzt werden:

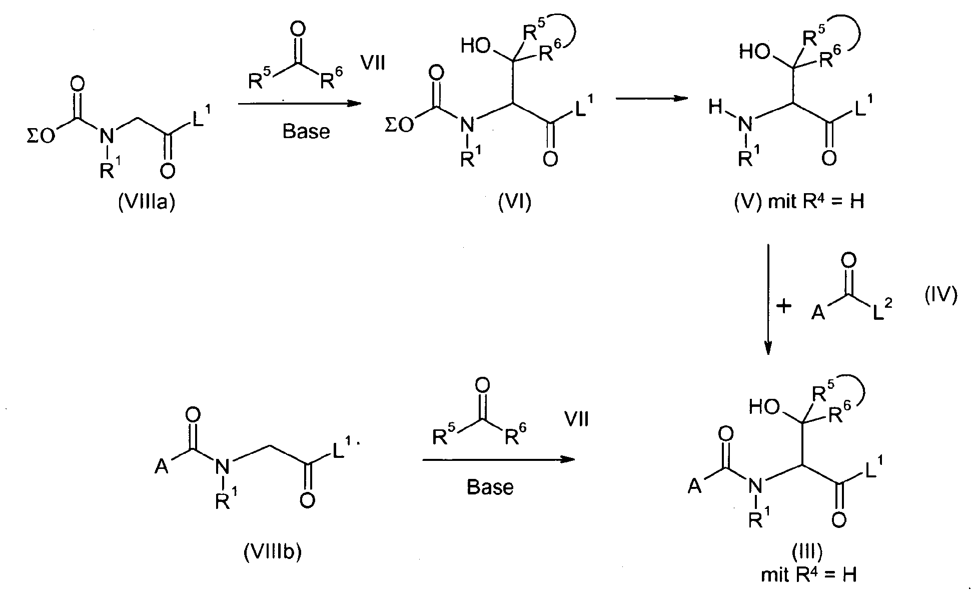

L¹ steht für eine nucleophil verdrängbare Abgangsgruppe, z.B. für Hydroxy oder C₁-C₆-Alkoxy.

L² steht für eine nucleophil verdrängbare Abgangsgruppe, z.B. für Hydroxy, Halogen, C₁-C₆-Alkylcarbonyl, C₁-C₆-Alkoxycarbonyl, C₁-C₄-Alkylsulfonyl, Phosphoryl oder Isoureyl.

Die Umsetzung der Glycinderivate (VIII) mit Carbonylverbindungen (VII) zum entsprechenden Aldolprodukt (VI) bzw. Heteroaroylderivat (III) mit R⁴ = Wasserstoff erfolgt üblicherweise bei Temperaturen von -100°C bis zum Siedepunkt der Reaktionsmischung, bevorzugt -80°C bis 20°C, insbesondere bevorzugt -80°C bis -20°C, in einem inerten organischen Lösungsmittel in Gegenwart einer Base [vgl. J.-F. Rousseau et al., J. Org. Chem. 63, 2731-2737 (1998)].

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, Ether wie Diethylether, Diisopropylether, tert.-Butylmethylether, Dioxan, Anisol und Tetrahydrofuran, sowie Dimethylsulfoxid, Dimethylformamid und Dimethylacetamid, besonders bevorzugt Diethylether, Dioxan und Tetrahydrofuran.

Es können auch Gemische der genannten Lösungsmittel verwendet werden.

Als Basen kommen allgemein anorganische Verbindungen wie Alkalimetall- und Erdalkalimetallhydride wie Lithiumhydrid, Natriumhydrid, Kaliumhydrid und Calciumhydrid, Alkalimetallamide wie Lithiumdiisopropylamid und Lithiumhexamethyldisilazid, metallorganische Verbindungen, insbesondere Alkalimetallalkyle wie Methyllithium, Butyllithium und Phenyllithium, sowie Alkalimetall- und Erdalkalimetallalkoholate wie Natriummethanolat, Natriumethanolat, Kaliumethanolat, Kalium- tert.-Butanolat, Kalium-tert.-Pentanolat und Dimethoxymagnesium, außerdem organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriumhydrid, Lithiumhexamethyldisilazid und Lithiumdiisopropylamid.

Die Basen werden im Allgemeinen in äquimolaren Mengen eingesetzt, sie können aber auch katalytisch, im Überschuss oder gegebenenfalls als Lösungsmittel verwendet werden.

Die Edukte werden im Allgemeinen in äquimolaren Mengen miteinander umgesetzt. Es kann vorteilhaft sein, die Base und/oder die Carbonylverbindungen (VII) in einem Überschuss bezogen auf die Glycinderivate (VIII) einzusetzen.

Die Aufarbeitung und Isolierung der Produkte kann in an sich bekannter Weise erfolgen.

Die für die Herstellung der Verbindungen (I) benötigten Glycinderivate der Formel (VIII) können käuflich erworben werden, sind in der Literatur bekannt [z.B. H. Pessoa-Mahana et al., Synth. Comm. 32, 1437 (2002] oder können gemäß der zitierten Literatur hergestellt werden.

Die Abspaltung der Schutzgruppe zu Serinderivaten der Formel (V) mit R⁴ = Wasserstoff erfolgt nach literaturbekannten Methoden [vgl. J.-F. Rousseau et al., J. Org. Chem. 63, 2731-2737 (1998)); J. M. Andres, Tetrahedron 56, 1523 (2000)]; im Fall von Σ = Benzyl durch Hydrogenolyse, bevorzugt durch Wasserstoff und Pd/C in Methanol; im Fall von Σ = tert.-Butyl durch Säure, bevorzugt Salzsäure in Dioxan.

Die Umsetzung der Serinderivate (V) mit R⁴= Wasserstoff mit Heteroarylsäure(derivate)n (IV) zu Heteroaroylderivaten (III) mit R⁴ = Wasserstoff erfolgt üblicherweise analog der unter Verfahren A genannten Umsetzung der Serinderivate der Formel (V) mit Heteroarylsäure(derivate)n der Formel (IV) zu Heteroaroylderivaten (III).

Die Heteroaroylderivate der Formel (III) mit R⁴ = Wasserstoff lassen sich anschließend mit Aminen der Formel (II) analog zu Verfahren A zu den gewünschten heteroaroyl-substituierten Serin-Amiden der Formel (I) mit R⁴ = Wasserstoff umsetzen, welche dann mit Verbindungen der Formel (IX) zu heteroaroyl-substituierten Serin-Amiden der

Formel (I) derivatisiert werden können [vgl. z.B. Yokokawa, F. et al., Tetrahedron Lett. 42 (34), 5903-5908 (2001); Arrault, A. etal., Tetrahedron Lett. 43(22), 4041-4044 (2002)].

Ebenso können die Heteroaroylderivate der Formel (III) mit R⁴ = Wasserstoff zunächst mit Verbindungen der Formel (IX) zu weiteren Heteroaroylderivaten der Formel (III) derivatisiert werden [vgl. z.B. Troast, D. et al., Org. Lett. 4 (6), 991-994 (2002); Ewing W. et al., Tetrahedron Lett., 30 (29), 3757-3760 (1989); Paulsen, H. et al., Liebigs Ann. Chem. 565 (1987)] und anschließend analog zu Verfahren A mit Aminen der Formel (II) zu den gewünschten heteroaroyl-substituierten Serin-Amiden der Formel (I) umgesetzt werden:

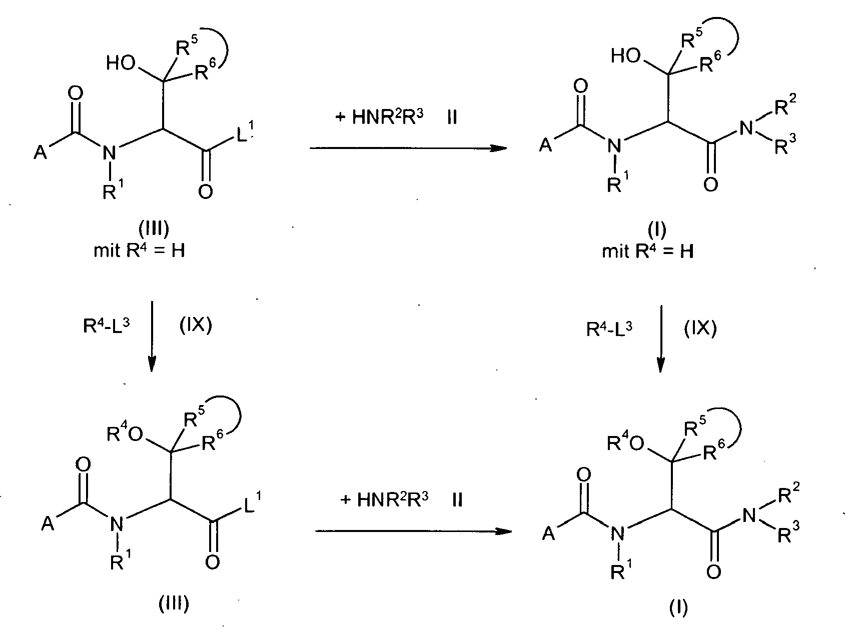

L¹ steht für eine nucleophil verdrängbare Abgangsgruppe, z.B. für Hydroxy oder C₁-C₆-Alkoxy.

L³ steht für eine nucleophil verdrängbare Abgangsgruppe, z.B. für Halogen, Hydroxy, oder C₁-C₆-Alkoxy.

Die Umsetzung der Heteroaroylderivate der Formel (III) (gegebenenfalls mit R⁴ = Wasserstoff) mit Aminen der Formel (II) zu heteroaroyl-substituierten Serin-Amiden der Formel (I) (gegebenenfalls mit R⁴ = Wasserstoff) erfolgt üblicherweise analog der unter Verfahren A geschilderten Umsetzung der Heteroaroylderivate der Formel (III) mit Aminen der Formel (II).

Die Umsetzung der Heteroaroylderivate der Formel (III) mit R⁴ = Wasserstoff bzw. der heteroaroyl-substituierten Serin-Amide der Formel (I) mit R⁴ = Wasserstoff mit Verbindungen der Formel (IX) zu Heteroaroylderivaten der Formel (III) bzw. heteroaroyl-substituierten Serin-Amiden der Formel (I) erfolgt üblicherweise bei Temperaturen von 0°C bis 100°C, vorzugsweise 10°C bis 50°C, in einem inerten organischen Lösungsmittel in Gegenwart einer Base [vgl. z.B. Troast, D. et al., Org. Lett. 4 (6), 991-994 (2002); Ewing W. et al., Tetrahedron Lett., 30 (29), 3757-3760 (1989); Paulsen, H. et al., Liebigs Ann. Chem. 565 (1987)].

Geeignete Lösungsmittel sind aliphatische Kohlenwasserstoffe wie Pentan, Hexan, Cyclohexan und Gemische von C₅-C₈-Alkanen, aromatische Kohlenwasserstoffe wie Toluol, o-, m- und p-Xylol, halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid und Dimethylacetamid, besonders bevorzugt Dichlormethan, tert.-Butylmethylether, Dioxan und Tetrahydrofuran.

Es können auch Gemische der genannten Lösungsmittel verwendet werden.

Als Basen kommen allgemein anorganische Verbindungen wie Alkalimetall- und Erdalkalimetallhydroxide wie Lithiumhydroxid, Natriumhydroxid, Kaliumhydroxid und Calciumhydroxid, Alkalimetall- und Erdalkalimetalloxide wie Lithiumoxid, Natriumoxid, Calciumoxid und Magnesiumoxid, Alkalimetall- und Erdalkalimetallhydride wie Lithiumhydrid, Natriumhydrid, Kaliumhydrid und Calciumhydrid, Alkalimetallamide wie Lithiumamid, Natriumamid und Kaliumamid, Alkalimetall- und Erdalkalimetallcarbonate wie Lithiumcarbonat, Kaliumcarbonat und Calciumcarbonat sowie Alkalimetallhydrogencarbonate wie Natriumhydrogencarbonat, metallorganische Verbindungen, insbesondere Alkalimetallalkyle wie Methyllithium, Butyllithium und Phenyllithium, Alkylmagnesiumhalogenide wie Methylmagnesiumchlorid sowie Alkalimetall- und Erdalkalimetallalkoholate wie Natriummethanolat, Natriumethanolat, Kaliumethanolat, Kalium- tert.-Butanolat, Kalium-tert.-Pentanolat und Dimethoxymagnesium, außerdem organische Basen, z.B. tertiäre Amine wie Trimethylamin, Triethylamin, Diisopropylethylamin und N-Methylpiperidin, Pyridin, substituierte Pyridine wie Collidin, Lutidin und 4-Dimethylaminopyridin sowie bicyclische Amine in Betracht. Besonders bevorzugt werden Natriumhydroxid, Natriumhydrid und Triethylamin.

Die Basen werden im Allgemeinen in äquimolaren Mengen eingesetzt, sie können aber auch katalytisch, im Überschuss oder gegebenenfalls als Lösungsmittel verwendet werden.

Die Edukte werden im Allgemeinen in äquimolaren Mengen miteinander umgesetzt. Es kann vorteilhaft sein, die Base und/oder (IX) in einem Überschuss bezogen auf (III) bzw. (I) einzusetzen.

Die Aufarbeitung und Isolierung der Produkte kann in an sich bekannter Weise erfolgen.

Die benötigten Verbindungen der Formel (VIII) können käuflich erworben werden.

### Verfahren C

Heteroaroylderivate der Formel (III) mit R⁴ = Wasserstoff und R⁶ = -C(OH)R'R" können auch erhalten werden, indem Vinylglycine der Formel (XIV) mit einem Oxidationsmittel wie Osmiumtetroxid oder Permanganat dihydroxyliert werden:

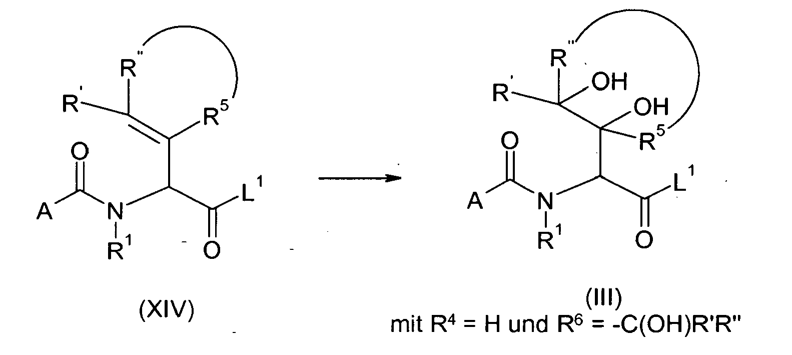

L¹ steht für eine nucleophil verdrängbare Abgangsgruppe, z.B. für Hydroxy oder C₁-C₆-Alkoxy.
R' steht für Wasserstoff, C₁-C₆-Alkyl, C₁-C₆-Halogenalkyl, C₁-C₆-Hydroxyalkyl, Phenyl oder C₁-C₆-Alkoxycarbonyl.
R" steht für Wasserstoff, C₁-C₆-Alkyl, C₁-C₆-Halogenalkyl, C₁-C₆-Hydroxyalkyl, Phenyl oder C₁-C₆-Alkoxycarbonyl.

Diese Umsetzung erfolgt üblicherweise bei Temperaturen von -78°C bis zum Siedepunkt der Reaktionsmischung, bevorzugt -10°C bis 120°C, insbesondere bevorzugt 0°C bis 50°C, in einem inerten organischen Lösungsmittel in gegebenenfalls in Gegenwart eines Reoxidationsmittels wie z.B. N-Methylmorpholin-N-oxid (D. Johnson et al., Tetrahedron 2000, 56, 5, 781).

Geeignete Lösungsmittel sind halogenierte Kohlenwasserstoffe wie Methylenchlorid, Chloroform und Chlorbenzol, Ether wie Diethylether, Diisopropylether, tert.-Butylmethylether, Dioxan, Anisol und Tetrahydrofuran, Nitrile wie Acetonitril und Propionitril, Ketone wie Aceton, Methylethylketon, Diethylketon und tert.-Butylmethylketon, Alkohole wie Methanol, Ethanol, n-Propanol, Isopropanol, n-Butanol und tert.-Butanol, sowie Dimethylsulfoxid, Dimethylformamid, Dimethylacetamid und Wasser; besonders bevorzugt Aceton oder Wasser.

Es können auch Gemische der genannten Lösungsmittel verwendet werden.

Die Edukte werden im Allgemeinen in äquimolaren Mengen miteinander umgesetzt. Es kann vorteilhaft sein, das Oxidationsmittel in einem Überschuss bezogen auf XIV einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die für die Herstellung der Heteroaroylderivate der Formel (III) mit R⁴ = Wasserstoff und R⁶ = -C(OH)R'R" benötigten Vinylglycine der Formel (XIV) sind in der Literatur bekannt [D. B. Berkowitz et al., J. Org. Chem. 2000, 65, 10, 2907; M. Koen et al., J. Chem. Soc. Perkin I 1997, 4, 487] oder können gemäß der zitierten Literatur hergestellt werden.

Die Heteroaroylderivate der Formel (III) mit R⁴ = Wasserstoff und R⁶ = -C(OH)R'R" lassen sich anschließend mit Aminen der Formel (II) analog zu Verfahren A zu den gewünschten heteroaroyl-substituierten Serin-Amiden der Formel (I) mit R⁴ = Wasserstoff und R⁶ = -C(OH)R'R" umsetzen, welche dann mit Verbindungen der Formel (IX) zu heteroaroylsubstituierten Serin-Amiden der Formel (I) mit R⁶ = -C(OR⁴)R'R" derivatisiert werden können [vgl. z.B. Yokokawa, F. et al., Tetrahedron Lett. 42 (34), 5903-5908 (2001); Arrault, A. et al., Tetrahedron Lett. 43(22), 4041-4044 (2002)];
ebenso können die Heteroaroylderivate der Formel (III) mit R⁴ = Wasserstoff zunächst mit Verbindungen der Formel (IX) zu weiteren Heteroaroylderivaten der Formel (III) mit R⁶ = -C(OR⁴)R'R" analog Verfahren B derivatisiert werden [vgl. z.B. Troast, D. et al., Org. Lett. 4 (6), 991-994 (2002); Ewing W. et al., Tetrahedron Lett., 30 (29), 3757-3760 (1989); Paulsen, H. et al., Liebigs Ann. Chem. 565 (1987)] und anschließend analog zu Verfahren A mit Aminen der Formel (II) zu den gewünschten heteroaroylsubstituierten Serin-Amiden der Formel (I) mit R⁶ = -C(OR⁴)R'R" umgesetzt werden: 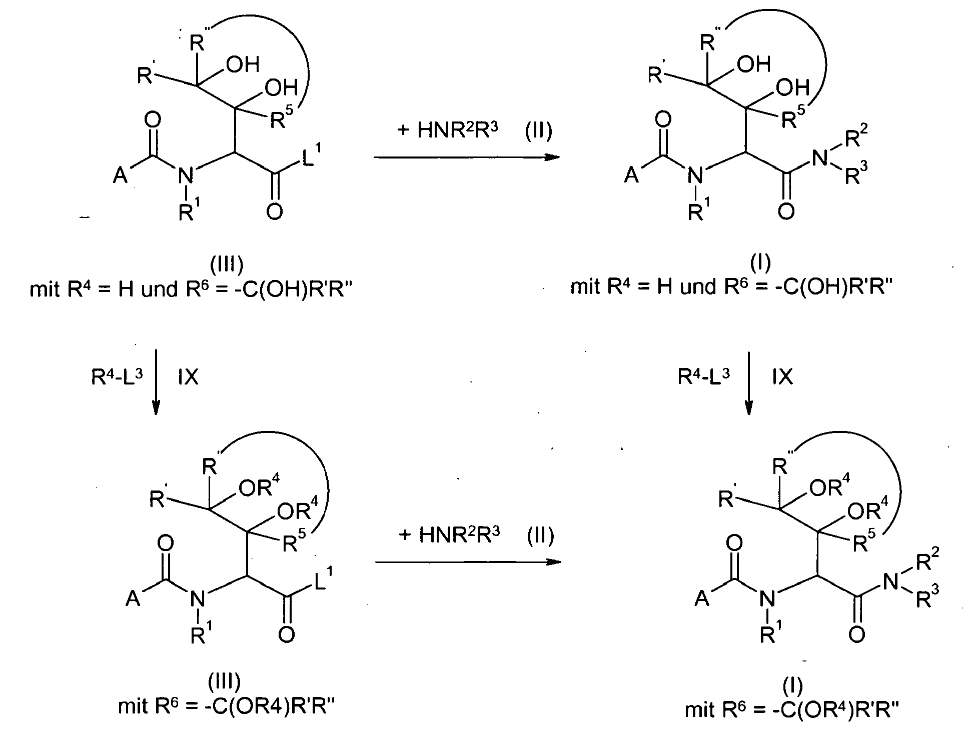 L¹ steht für eine nucleophil verdrängbare Abgangsgruppe, z.B. für Hydroxy oder C₁-C₆-Alkoxy.
L³ steht für eine nucleophil verdrängbare Abgangsgruppe, z.B. für Halogen, Hydroxy, oder C₁-C₆-Alkoxy.
R' steht für Wasserstoff, C₁-C₆-Alkyl, C₁-C₆-Halogenalkyl, C₁-C₆-Hydroxyalkyl, Phenyl oder C₁-C₆-Alkoxycarbonyl.
R" steht für Wasserstoff, C₁-C₆-Alkyl, C₁-C₆-Halogenalkyl, C₁-C₆-Hydroxyalkyl, Phenyl oder C₁-C₆-Alkoxycarbonyl.

### Verfahren D

Heteroaroylderivate der Formel (III) mit R⁴ = Wasserstoff und R⁶ = -C(Nuc)R'R" können auch erhalten werden, indem Vinylglycine der Formel (XIV) mit einem Epoxidierungsreagenz zu Epoxyglycinen der Formel (XV) epoxydiert werden und anschließend eine nucleophile Epoxidöffnung erfolgt: 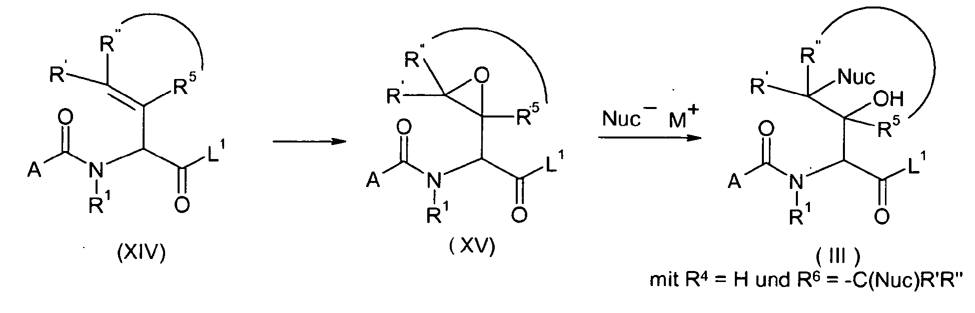 L¹ steht für eine nucleophil verdrängbare Abgangsgruppe, z.B. für Hydroxy oder C₁-C₆-Alkoxy.
R' steht für Wasserstoff, C₁-C₆-Alkyl, C₁-C₆-Halogenalkyl, C₁-C₆-Hydroxyalkyl, Phenyl oder C₁-C₆-Alkoxycarbonyl.
R" steht für Wasserstoff, C₁-C₆-Alkyl, C₁-C₆-Halogenalkyl, C₁-C₆-Hydroxyalkyl, Phenyl oder C₁-C₆-Alkoxycarbonyl.
Nuc⁻M⁺ steht beispielsweise für Thiolate wie z.B. Natriumthiophenolat, Alkoholate wie Kaliumphenolat, oder Amide wie Natriumimidazolat.

Die Epoxidierung erfolgt üblicherweise bei Temperaturen von -78°C bis zum Siedepunkt der Reaktionsmischung, bevorzugt -20°C bis 50°C, insbesondere bevorzugt 0°C bis 30°C, in einem inerten organischen Lösungsmittel [vgl. P. Meffre et al., Tetrahedron Lett. 1990, 31, 16, 2291.

Als Epoxidierungsreagenzien finden Verwendung Persäuren und Peroxide (z.B. Metachlorperbenzoesäure, Peressigsäure, Dimethyldioxiran, Wasserstoffperoxid).

Geeignete Lösungsmittel sind halogenierte Kohlenwasserstoffe wie Methylenchlorid, Chloroform und Chlorbenzol, Alkohole wie Methanol, Ethanol, n-Propanol, Isopropanol, n-Butanol und tert.-Butanol, sowie Wasser,
besonders bevorzugt halogenierte Kohlenwasserstoffe und Wasser.

Es können auch Gemische der genannten Lösungsmittel verwendet werden.

Die Edukte werden im Allgemeinen in äquimolaren Mengen miteinander umgesetzt. Es kann vorteilhaft sein, das Epoxidierungsmittel in einem Überschuss bezogen auf (XIV) einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die für die Herstellung der Heteroaroylderivate der Formel (III) mit R⁴ = Wasserstoff und R⁶ = -C(OH)R'R" benötigten Vinylglycine der Formel (XIV) sind in der Literatur bekannt [D. B. Berkowitz et al., J. Org. Chem. 2000, 65, 10, 2907; M. Koen et al., J. Chem. Soc. Perkin I 1997, 4, 487] oder können gemäß der zitierten Literatur hergestellt werden.

Die Epoxidöffnung erfolgt üblicherweise bei Temperaturen von -78°C bis zum Siedepunkt der Reaktionsmischung, bevorzugt -20°C bis 100°C, insbesondere bevorzugt 0°C bis 50°C, in einem inerten organischen Lösungsmittel gegebenenfalls in Gegenwart eines Katalysators [vgl. P. Meffre et al., Tetrahedron Lett. 1990, 31, 16, 2291; M. R. Paleo et al., J. Org. Chem. 2003, 68, 1, 130].

Geeignete Lösungsmittel sind Alkohole wie Methanol, Ethanol, n-Propanol, Isopropanol, n-Butanol und tert.-Butanol, sowie Dimethylsulfoxid, Dimethylformamid und Dimethylacetamid und Wasser, besonders bevorzugt Methanol und Wasser.

Es können auch Gemische der genannten Lösungsmittel verwendet werden.

Als saure Katalysatoren finden Lewis-Säuren wie Bortrifluorid, Aluminiumtrichlorid, Eisen-III-chlorid, Zinn-IV-chlorid, Titan-IV-chlorid, Zink-II-chlorid und Magnesiumperchlorat Verwendung.

Der Katalysator wird üblicher Weise in einem Mengenverhältnis von 1 bis 100 mol%, bevorzugt 1 bis 10 mol% bezogen auf die Verbindung (XV) eingesetzt.

Die Edukte werden im Allgemeinen in äquimolaren Mengen miteinander umgesetzt. Es kann vorteilhaft sein, Nuc⁻⁻M⁺ in einem Überschuss bezogen auf (XV) einzusetzen.

Die Reaktionsgemische werden in üblicher Weise aufgearbeitet, z.B. durch Mischen mit Wasser, Trennung der Phasen und gegebenenfalls chromatographische Reinigung der Rohprodukte. Die Zwischen- und Endprodukte fallen z.T. in Form zäher Öle an, die unter vermindertem Druck und bei mäßig erhöhter Temperatur von flüchtigen Anteilen befreit oder gereinigt werden. Sofern die Zwischen- und Endprodukte als Feststoffe erhalten werden, kann die Reinigung auch durch Umkristallisieren oder Digerieren erfolgen.

Die Heteroaroylderivate der Formel (III) mit R⁴ = Wasserstoff und R⁶ = -C(Nuc)R'R" lassen sich anschließend mit Aminen der Formel (II) analog zu Verfahren A zu den gewünschten benzoylsubstituierten Serin-Amiden der Formel (I) mit R⁴ = Wasserstoff und R⁶ = -C(Nuc)R'R" umsetzen, welche dann mit Verbindungen der Formel (IX) zu heteroaroyl-substituierten Serin-Amiden der Formel (I) mit R⁶ = -C(Nuc)R'R" derivatisiert werden können [vgl. z.B. Yokokawa, F. et al., Tetrahedron Lett. 42 (34), 5903-5908 (2001); Arrault, A. et al., Tetrahedron Lett. 43( 22), 4041-4044 (2002)];

Ebenso können die Heteroaroylderivate der Formel (III) mit R⁴ = Wasserstoff zunächst mit Verbindungen der Formel (IX) zu weiteren Benzoylderivaten der Formel (III) mit R⁶ = -C(Nuc)R'R" analog Verfahren B derivatisiert werden [vgl. z.B. Troast, D. et al., Org. Lett. 4 (6), 991-994 (2002); Ewing W. et al., Tetrahedron Lett., 30 (29), 3757-3760 (1989); Paulsen, H. et al., Liebigs Ann. Chem. 565 (1987)] und anschließend analog zu Verfahren A mit Aminen der Formel (II) zu den gewünschten heteroaroylsubstituierten Serin-Amiden der Formel (I) mit R⁶ = -C(Nuc)R'R" umgesetzt werden: 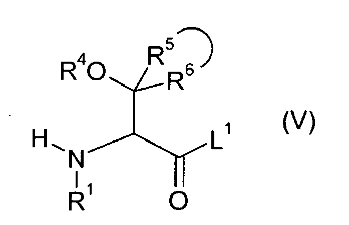 L¹ steht für eine nucleophil verdrängbare Abgangsgruppe, z.B. für Hydroxy oder C₁-C₆-Alkoxy.
L³ steht für eine nucleophil verdrängbare Abgangsgruppe, z.B. für Halogen, Hydroxy, oder C₁-C₆-Alk6xy.
R' steht für Wasserstoff, C₁-C₆-Alkyl, C₁-C₆-Halogenalkyl, C₁-C₆-Hydroxyalkyl, Phenyl oder C₁-C₆-Alkoxycarbonyl.
R" steht für Wasserstoff, C₁-C₆-Alkyl, C₁-C₆-Halogenalkyl, C₁-C₆-Hydroxyalkyl, Phenyl oder C₁-C₆-Alkoxycarbonyl.
Nuc⁻M⁺ steht beispielsweise für Thiolate wie z.B. Natriumthiophenolat, Alkoholate wie Kaliumphenolat, oder Amide wie Natriumimidazolat.

### Heteroaroylderivate der Formel (III),

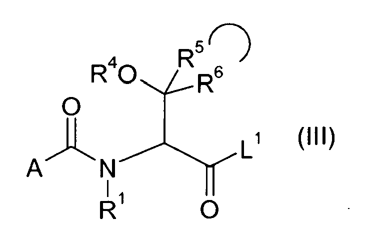

wobei A, R¹ sowie R⁴, R⁵ und R⁶ die voranstehend genannten Bedeutungen haben und L¹ für Hydroxy oder C₁-C₆-Alkoxy steht, sind ebenfalls ein Gegenstand der Erfindung.

Die besonders bevorzugten Ausführungsformen der Zwischenprodukte in Bezug auf die Variablen entsprechen denen der Reste A, R¹ sowie R⁴ bis R⁶ der Formel (I).

Besonders bevorzugt werden Heteroaroylderivate der Formel (III), in der
- A: 5- oder 6-gliedriges Heteroaryl ausgewählt aus der Gruppe Thienyl, Furyl, Pyra- zolyl, Imidazolyl, Thiazolyl, Oxazolyl und Pyridyl; wobei die
genannten Heteroarylreste partiell oder vollständig halogeniert sein kön- nen und/oder 1 bis 3 Reste aus der Gruppe C₁-C₆-Alkyl, C₃-C₆-Cycloalkyl, und C₁-C₆-Halogenalkyl tragen können;
- R¹: Wasserstoff;
- R⁴: Wasserstoff, Formyl, C₁-C₄-Alkylcarbonyl, C₁-C₄-Alkylaminocarbonyl, Di-(C₁-C₄- Alkyl)-aminocarbonyl, Phenylaminocarbonyl, N-(C₁-C₄-alkyl)-N-(phenyl)- aminocarbonyl, SO₂CH₃, SO₂CF₃ oder SO₂(C₆H₅);
- R⁵ und R⁶: zusammen mit dem Kohlenstoffatom, an das sie gebunden sind, einen 3 bis 7-gliedrigen, gesättigten oder partiell ungesättigten Ring, der carbocyclisch ist oder 1 oder 2 N-Atome, 0 oder 1 N-Atom und 1 O-Atom oder S-Atom, 0 oder 1 N-Atom und 1 O- und 1 S-Atom, 2 O- oder S-Atome enthält,
wobei der Ring unsubstituiert ist oder substituiert durch 1 bis 3, im Fall von Halogen auch bis zur maximal möglichen Anzahl, Substituenten aus der Gruppe Halogen, C₁-C₆-Alkyl, Hydroxy, C₁-C₆-Alkoxy, Formyl, C₁-C₆-Alkyl- carbonyl, C₁-C₆-Alkoxycarbonyl, C₁-C₆-Alkylaminocarbonyl, Di-(C₁-C₆- alkyl)-aminocarbonyl, Alkylsulfonylamino, Carbonyl, Alkoxyimino,
wobei die genannten Alkyl- und Alkoxyreste partiell oder vollständig halogeniert sein können und/oder eine bis drei der folgenden Grup- pen tragen können: Cyano, Hydroxy, C₁-C₄-Alkoxy,
Phenyl, partiell oder vollständig halogeniert,
und der Ring monocyclisch ist oder anelliert mit einem weiteren 3 bis 6-gliedrigen gesättigten oder partiell ungesättigten Ring, der carbocyclisch ist oder 1 oder 2 N-Atome, 0 oder 1 N-Atom und 1 O-Atom oder S-Atom, 2 O-Atome oder S- Atome, 0 oder 1 N-Atom und 1 O-Atom und 1 S-Atom enthält,
wobei der anellierte Ring unsubstituiert ist oder substituiert durch 1 bis 3, im Fall von Halogen auch bis zur maximal möglichen Anzahl, Substituenten aus der Gruppe Halogen, C₁-C₆-Alkyl, C₁-C₆-Halogenalkyl und C₁-C₆- Alkoxy,
oder der Ring ist mit einer 1 bis 3-zähligen gesättigten oder ungesättigten Kette überbrückt, die keine Heteroatome enthält oder 1 N-Atom, 0 oder 1 N-Atom und 1 O-Atom oder 1 S-Atom enthält,
wobei die Brücke unsubstituiert ist oder substituiert mit 1 bis 3, im Fall von Halogen auch bis zur maximal möglichen Anzahl. Substituenten aus der Gruppe Halogen, C₁-C₆-Alkyl, Hydroxy und C₁-C₆-Alkoxy.
bedeuten.

Die folgenden Beispiele dienen der Erläuterung der Erfindung.

### Herstellungsbeispiele

### Beispiel 1

### Dimethyl-carbaminsäure-1-{methylcarbamoyl-[(1-methyl-3-trifluormethyl-1H-pyrazol-4-carbonyl)-amino]-methyl}-cyclobutylester

### 1.1 [(1-Methyl-3-trifluormethyl-1H-pyrazol-4-carbonyl)-amino]-essigsäureethylester

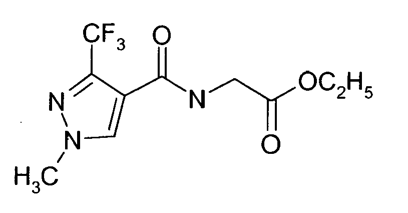

3.63 g (25.8 mmol) Glycinethylester Hydrochlorid wurde in CH₂Cl₂ gelöst und bei RT THF, 5.00 g (25.8 mmol) 1-Methyl-3-trifluormethy-4-carbonsäure und 7.82 g Triethylamin (77.3 mmol), sowie bei 0°C 6.56 g (25.8 mmol) Bis-(2-oxo)-3-oxazolidinyl)-phosphonsäurechlorid zugegeben. Es wurde 3 h bei 0°C und anschließend 16 h bei RT gerührt. Danach wuden die Lösungsmittel entfernt, der Rückstand mit Essigester aufgenommen, gewaschen, getrocknet und das Lösungsmittel entfernt. Man erhielt 3.88 g (54 % der Theorie) der Titelverbindung als rotes Öl.
¹H-NMR (DMSO): δ = 1.20 (t,3H); 3.95 (s, 6H); 4.15 (q, 2H); 8.35 (s, 1H); 8.65 (t, 1H).

### 1.2 (1-Hydroxy-cyclobutyl)-[(3-trifluormethyl-1H-pyrazol-4-carbonyl)-amino]-essigsäureethylester (Vbdg. 3.1)

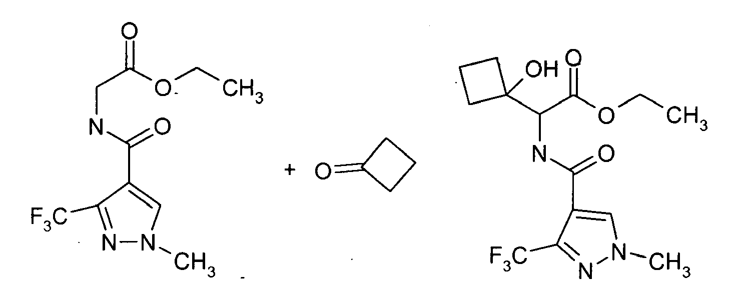

23.0ml Hexamethyldisilazan (mmol) wurden in 200ml THF gelöst und 46ml 2.5M (115mmol) Butyllithium-Lösung in Hexan bei -78°C zugetropft. Nach 30min wurden 7.90g (22.3mmol) [(1-Methyl-3-trifluoromethyl-1H-pyrazole-4-carbonyl)-amino]-essigsäureethylester gelöst in 50ml THF zugetropft und 1.5h bei -78°C gerührt. Es wurden 3.0g (42.8mmol) Cyclobutanon und 4.14g (29.2mmol) BF₃-Etherat gelöst in 50ml THF zugetropft und 2h bei -78°C, 1 h bei -50°C und 1 h bei 0°C gerührt. Es wurden 100ml gesättigter NH₄Cl-Lösung zugetropft und 30 min nachgerührt. Die organische Phase wurde abgetrennt und die Lösungsmittel abgezogen. Man erhielt 9.88g (100%) farblose Kristalle, die ohne weitere Reinigung umgesetzt wurden.
1H-NMR (DMSO): 1.2 (t, 3H); 1.5-2.2 (m, 6H); 4.1 (m, 2H); 4.6 (d, 1H); 5.4 (s, 1H); 8.0 (d, 1H); 8.5 (s, 1H)
MS (M+H): 350

In analoger Weise wurden die in der untenstehenden Tabelle 3 aufgeführten Zwischenprodukte hergestellt.

### 1.3 1-Methyl-3-trifluormethyl-1H-pyrazol-4-carbonsäure[1-hydroxy-cyclobutyl)-methylcarbamoyl-methyl]-amid

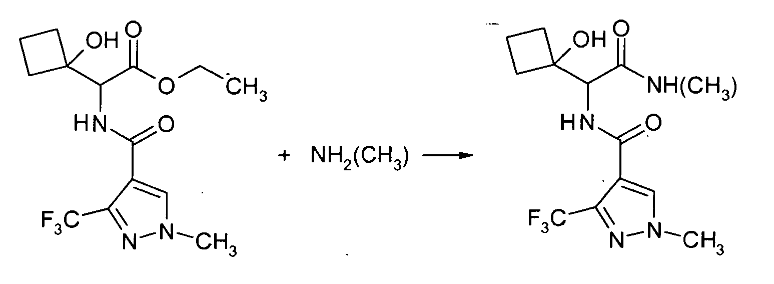

9.00g (25.8mmol) [(1-Methyl-3-trifluormethyl-1H-pyrazol-4-carbonyl)-amino]essigsäureethylester wurden in 400ml Methanol gelöst und bei 0°C 1 h Methylamin eingeleitet. Der entstehende Niederschlag wurde abgesaugt und mit Pentan gewaschen. Man erhielt 4.7g farblose Kristalle.
1H-NMR (DMSO): 1.5-2.1 (m, 5H); 2.4 (m, 1H); 2.6(d, 3H); 3.9 (s, 3H); 4.5 (d, 1H); 5.3 (s, 1H); 7.8 (s, 1H); 7.9 (d, 1H); 8.5 (s, 1H)
MS (M+H):335
MP: 210°C

### 1.4 Dimethyl-carbaminsäure-1-{methylcarbamoyl-1[(1-methyl-3-trifluormethyl-1H-pyrazol-4-carbonyl)-aminol-methyl}-cyclobutylester

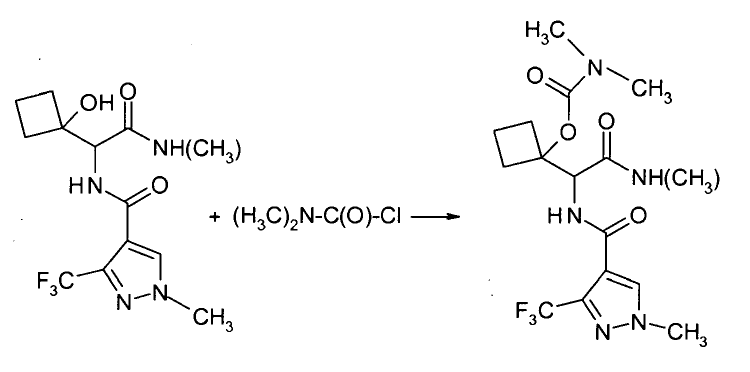

600mg (1.79mmol)1-Methyl-3-trifluromethyl-1H-pyrazol-4-carbonsäure[(1-hydroxycyclobutyl)-methylcarbamoyl-methyl]-amid wurden in 30ml THF gelöst und 0.130g (2.75mmol) Natriumhydrid (60%ig in Mineralöl) zugegeben. Es wurden 300mg (2.79mmol) Dimethylcarbamoylchlorid zugegeben und 15h bei 23°C gerührt. Es wurde eingeengt, in Essigester aufgenommen, mit Wasser gewaschen, mit Na₂SO₄ getrocknet und wieder eingeengt. Man erhielt 650mg (1.62mmol, 90% d.Th.) farblose Kristalle. 1H-NMR (DMSO): 1.2 (m, 1H); 1.6 (m,1H); 1.8 (m, 1H); 2.4 (m, 3H); 2.6 (d, 3H); 2.8 (2s, 6H); 3.9 (s, 3H); 8.0 (m, 1H); 8.3 (d, 1H); 8.4 (s, 1H)
MS (M+H): 406
MP: 190°C

In analoger Weise wurden die in der nachfolgenden Tabelle 2 aufgeführten Verbindungen der Formel (I) hergestellt.

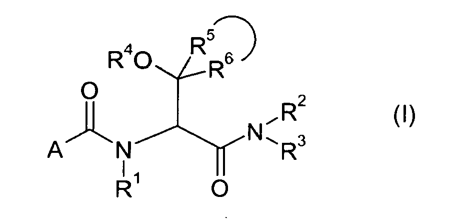

**Tabelle 2**

| Nr. | A | R¹ | R² | R³ | R⁴ | R⁵+R⁶ | Salz | MS (m⁺+H) | Fp(°C) |
|---|---|---|---|---|---|---|---|---|---|
| 2.1 | 1-CH₃-3-CF₃-4-Pyrazolyl | H | H | CH₃ | H | -CH₂-O-C(CH₃)₂-O-CH₂- | | 395 | 162 |
| 2.2 | 1-CH₃-3-CF₃-4-Pyrazolyl | H | H | CH₃ | H | -CH₂-CH₂-O-CH₂-CH₂- | | 365 | 195 |
| 2.3 | 1-CH₃-3-CF₃-4-Pyrazolyl | H | H | CH₃ | (CO)N(CH₃)₂ | -CH₂-CH₂-O-CH₂-CH₂- | | 436 | 222 |
| 2.4 | 1-CH₃-3-CF₃-4-Pyrazolyl | H | H | CH₃ | (CO)NH(CH₃) | -CH₂-CH₂-O-CH₂-CH₂- | | 422 | 198 |
| 2.5 | 1-CH₃-3-CF₃-4-Pyrazolyl | H | H | CH₃ | (CO)NH₂ | -CH₂-CH₂-O-CH₂-CH₂- | | 408 | 198 |
| 2.6 | 1-CH₃-3-CF₃-4-Pyrazolyl | H | H | CH₃ | H | -CH₂-CH₂-S-CH₂-CH₂- | | 381 | 223 |
| 2.7 | 1-CH₃-3-CF₃-4-Pyrazolyl | H | H | CH₃ | (CO)N(CH₃)₂ | -CH₂-CH₂-S-CH₂-CH₂- | | 452 | 215 |
| 2.8 | 1-CH₃-3-CF₃-4-Pyrazolyl | H | H | CH₃ | (CO)N(CH₃)₂ | -CH₂-CH₂-S(O)-CH₂-CH₂- | | 468 | 225 |
| 2.9 | 1-CH₃-3-CF₃-4-Pyrazolyl | H | H | CH₃ | (CO)N(CH₃)₂ | -CH₂-CH₂-S(O)₂-CH₂-CH₂- | | 484 | |
| 2.10 | 1-CH₃-3-CF₃-4-Pyrazolyl | H | H | CH₃ | H | -CH₂-CH₂-CH₂-CH₂-CH₂- | | 363 | 211 |
| 2.11 | 1-CH₃-3-CF₃-4-Pyrazolyl | H | H | CH₃ | H | -CH₂-CH₂-CH₂-CH₂- | | 349 | 204 |
| 2.12 | 1-CH₃-3-CF₃-4-Pyrazolyl | H | H | CH₃ | (CO)N(CH₃)₂ | -CH₂-CH₂-CH₂-CH₂-CH₂- | | 434 | 195 |
| 2.13 | 1-CH₃-3-CF₃-4-Pyrazolyl | H | H | CH₃ | (CO)NH₂ | -CH₂-CH₂-CH₂-CH₂-CH₂- | | 406 | 205 |
| 2.14 | 1-CH₃-3-CF₃-4-Pyrazolyl | H | H | CH₃ | H | -CH₂-CH₂-O-CH(CH₃)- | | 365 | 210 |
| 2.15 | 1-CH₃-3-CF₃-4-Pyrazolyl | H | H | CH₃ | (CO)N(CH₃)₂ | -CH₂-CH₂-S-CH₂- | | 438 | 205 |
| 2.16 | 1-CH₃-3-CF₃-4-Pyrazolyl | H | H | CH₃ | H | -CH₂-CH₂-S-CH₂- | | 367 | 215 |
| 2.17 | 1-CH₃-3-CF₃-4-Pyrazolyl | H | H | CH₃ | H | -CH₂-CH₂-CH₂-CH(CH₂CH₂CONHCH₃)- | | 434 | 156 |
| 2.18 | 1-CH₃-3-CF₃-4-Pyrazolyl | H | H | CH₃ | H | -CH₂-CH₂-N(COOC(CH₃)₃)-CH₂- | | 450 | 215 |
| 2.19 | 1-CH₃-3-CF₃-4-Pyrazolyl | H | H | CH₃ | (CO)CH₃ | -CH₂-CH₂-CH₂-CH₂-CH₂- | | 405 | 182 |
| 2.20 | 1-CH₃-3-CF₃-4-Pyrazolyl | H | H | CH₃ | (CO)NH(CH₃) | -CH₂-CH₂-CH₂-CH₂-CH₂- | | 420 | 145 |
| 2.21 | 1-CH₃-3-CF₃-4-Pyrazolyl | H | H | CH₃ | H | -CH₂-CH₂-C(-O-CH₂-CH₂-O-)-CH₂-CH₂- | | 421 | 214 |
| 2.22 | 1-CH₃-3-CF₃-4-Pyrazolyl | H | H | CH₃ | (CO)N(CH₃)₂ | -CH₂-CH₂-C(-O-CH₂-CH₂-O-)-CH₂-CH₂- | | 492 | 220 |
| 2.23 | 1-CH₃-3-CF₃-4-Pyrazolyl | H | H | CH₃ | H | -CH₂-CH₂-CH(OSi(CH₃)₂C(CH₃)₃)-CH₂-CH₂- | | 493 | 174 |
| 2.24 | 1-CH₃-3-CF₃-4-Pyrazolyl | H | H | CH₃ | (CO)NH(CH₃) | -CH₂-CH₂-O-CH(CH₃)- | | 422 | 200 |
| 2.25 | 1-CH₃-3-CF₃-4-Pyrazolyl | H | H | CH₃ | H | -CH₂-CH₂-CH₂-CH(CH₃)- | | 363 | 203 |
| 2.26 | 1-CH₃-3-CF₃-4-Pyrazolyl | H | H | CH₃ | H | -CH(CH₃)-CH₂-CH₂-CH(CH₃)- | | 377 | 194 |
| 2.27 | 1-CH₃-3-CF₃-4-Pyrazolyl | H | H | CH₃ | (CO)NH(CH₃) | -CH(CH₃)-CH₂-CH₂-CH(CH₃)- | | 434 | 192 |
| 2.28 | 1-CH₃-3-CF₃-4-Pyrazolyl | H | H | CH₃ | (CO)NH(CH₃) | -CH₂-CH₂-N(COOC(CH₃)₃)-CH₂- | | 507 | |
| 2.29 | 1-CH₃-3-CF₃-4-Pyrazolyl | H | H | CH₃ | H | -CH₂-CH₂-CH₂-CH₂-CH₂-CH₂-CH₂- | | 391 | 175 |
| 2.30 | 1-CH₃-3-CF₃-4-Pyrazolyl | H | H | CH₃ | H | -CH₂-CH₂-CH₂-CH₂-CH₂-CH₂- | | 377 | 185 |
| 2.31 | 1-CH₃-3-CF₃-4-Pyrazolyl | H | H | CH₃ | (CO)NH(CH₃) | -CH₂-CH₂-CH₂-CH₂-CH₂-CH₂- | | 434 | 177 |
| 2.32 | 1-CH₃-3-CF₃-4-Pyrazolyl | H | H | CH₃ | (CO)N(CH₃)₂ | -CH₂-CH₂-N(COO(CH₃)₃)-CH₂-CH₂- | | 535 | 224 |
| 2.33 | 1-CH₃-3-CF₃-4-Pyrazolyl | H | H | CH₃ | H | -CH₂-CH₂-CH₂- | | 335 | 210 |
| 2.34 | 1-CH₃-3-CF₃-4-Pyrazolyl | H | H | CH₃ | (CO)N(CH₃)₂ | -CH₂-CH₂-CH₂- | | 406 | 190 |
| 2.35 | 1-CH₃-3-CF₃-4-Pyrazolyl | H | H | CH₃ | (CO)CH₃ | -CH₂-CH₂-CH₂-CH₂- | | 391 | 170 |
| 2.36 | 1-CH₃-3-CF₃-4-Pyrazolyl | H | H | CH₃ | (CO)N(CH₃)₂ | -CH₂-CH₂-CH₂-CH₂- | | 420 | 228 |
| 2.37 | 1-CH₃-3-CF₃-4-Pyrazolyl | H | H | CH₃ | (CO)NH(CH₃) | -CH₂-CH₂-CH₂-CH₂- | | 406 | |
| 2.38 | 1-CH₃-3-CF₃-4-Pyrazolyl | H | H | CH₃ | (CO)NH₂ | -CH₂-CH₂-CH₂-CH₂- | | 392 | 218 |
| 2.39 | 1-CH₃-3-CF₃-4-Pyrazolyl | H | H | CH₃ | (CO)NH(CH₃) | -CH₂-CH₂-CH₂- | | 392 | 200 |
| 2.40 | 1-CH3-3-CF3-4-Pyrazolyl | H | H | CH₃ | H | 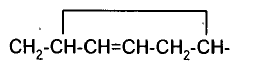 | | 373 | 132 |
| 2.41 | 1-CH₃-3-CF₃-4-Pyrazolyl | H | H | CH₃ | H | 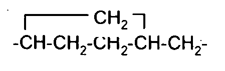 | | 375 | 210 |
| 2.42 | 1-CH₃-3-CF₃-4-Pyrazolyl | H | H | CH₃ | H | 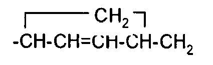 | | 373 | 192 |
| 2.43 | 1-CH₃-3-CF₃-4-Pyrazolyl | H | H | CH₃ | (CO)NH(CH₃) | 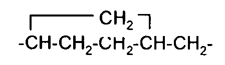 | | 431 | 194 |
| 2.44 | 1-CH₃-3-CF₃-4-Pyrazolyl | H | H | CH₃ | (CO)N(CH₃)₂ | 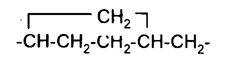 | | 446 | 207 |
| 2.45 | 1-CH₃-3-CF₃-4-Pyrazolyl | H | H | CH₃ | (CO)NH₂ | 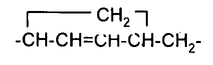 | | 416 | 190 |
| 2.46 | 1-CH₃-3-CF₃-4-Pyrazolyl | H | H | CH₃ | (CO)N(CH₃)₂ | -CH₂-CH₂-CH(OSi(CH₃)₂C(CH₃)₃)-CH₂-CH₂- | | 537 | 175 |
| 2.47 | 1-CH₃-3-CF₃-4-Pyrazolyl | H | H | CH₃ | (CO)NH₂ | 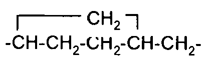 | | 418 | 182 |
| 2.48 | 1-CH₃-3-CF₃-4-Pyrazolyl | H | H | CH₃ | (CO)N(CH₃)₂ | 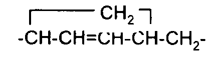 | | 444 | 210 |
| 2.49 | 1-CH₃-3-CF₃-4-Pyrazolyl | H | H | CH₃ | (CO)NH(CH₃) | 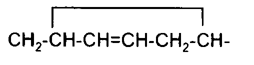 | | 429 | 95 |
| 2.50 | 1-CH₃-3-CF₃-4-Pyrazolyl | H | H | CH₃ | (CO)N(CH₃)₂ | 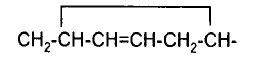 | | 444 | 156 |
| 2.51 | 1-CH₃-3-CF₃-4-Pyrazolyl | H | H | CH₃ | (CO)NH(CH₃) | 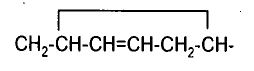 | | 429 | 182 |
| 2.52 | 1-CH₃-3-CF₃-4-Pyrazolyl | H | H | CH₃ | (CO)CH₃ | -CH₂-CH₂-CH₂- | | 377 | 190 |
| 2.53 | 1-CH₃-3-CF₃-4-Pyrazolyl | H | H | CH₃ | (CO)N(CH₃)₂ | -CH₂-CH₂-CH(OH)-CH₂-CH₂- | | 450 | |
| 2.54 | 1-CH₃-3-CF₃-4-Pyrazolyl | H | H | CH₃ | H | -CH₂-CH₂-CH(OH)-CH₂-CH₂- | | 379 | 80 |
| 2.55 | 1-CH₃-3-CF₃-4-Pyrazolyl | H | H | CH₃ | (CO)N(CH₃)₂ | -CH₂-CH₂-C(O)-CH₂-CH₂- | | 448 | 188 |
| 2.56 | 1-CH₃-3-CF₃-4-Pyrazolyl | H | H | CH₃ | H | -CH₂-CH₂-CH₂-O-CH₂- | | 365 | |
| 2.57 | 1-CH₃-3-CF₃-4-Pyrazolyl | H | H | CH₃ | H | -CH₂-CH₂-N(CON(CH₃)₂)-CH₂-CH₂- | | 435 | 222 |
| 2.58 | 1-CH₃-3-CF₃-4-Pyrazolyl | H | H | CH₃ | H | -CH₂-CH₂-N(COCH₃)-CH₂-CH₂- | | 406 | |
| 2.59 | 1-CH₃-3-CF₃-4-Pyrazolyl | H | H | CH₃ | H | -CH₂-CH₂-NH-CH₂-CH₂- | HCl | 364 | |
| 2.60 | 1-CH₃-3-CF₃-4-Pyrazolyl | H | H | CH₃ | (CO)N(CH₃)₂ | -CH₂-CH₂-N(CON(CH₃)₂)-CH₂-CH₂- | | 596 | 184 |
| 2.61 | 1-CH₃-3-CF₃-4-Pyrazolyl | H | H | CH₃ | (CO)N(CH₃)₂ | -CH₂-CH₂-N(COCH₃)-CH₂-CH₂- | | 477 | 88 |
| 2.62 | 1-CH₃-3-CF₃-4-Pyrazolyl | H | H | CH₃ | (CO)N(CH₃)₂ | -CH₂-CH₂-N(CH₃)-CH₂-CH₂- | | 449 | 212 |
| 2.63 | 1-CH₃-3-CF₃-4-Pyrazolyl | H | H | CH₃ | (CO)N(CH₃)₂ | -CH₂-CH₂-NH-CH₂-CH₂- | HCl | 471 | 175 |
| 2.64 | 1-CH₃-3-CF₃-4-Pyrazolyl | H | H | CH₃ | (CO)N(CH₃)₂ | -CH₂-CH₂-N(CH₂CN)-CH₂-CH₂- | | 474 | 197 |
| 2.65 | 1-CH₃-3-CF₃-4-Pyrazolyl | H | H | CH₃ | H | -CH₂-CH₂-O-CH₂- | | 351 | |
| 2.66 | 1-CH₃-3-CF₃-4-Pyrazolyl | H | H | CH₃ | H | -CH₂-O-CH₂- | | 337 | |
| 2.67 | 1-CH₃-3-CF₃-4-Pyrazolyl | H | H | CH₃ | (CO)CH₃ | 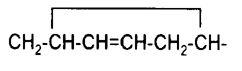 | | 415 | |
| 2.68 | 1-CH₃-3-CF₃-4-Pyrazolyl | H | H | CH₃ | (CO)N(CH₃)₂ | -CH₂-O-CH₂- | | 408 | |
| 2.69 | 1-CH₃-3-CF₃-4-Pyrazolyl | H | H | CH₃ | (CO)NH(CH₃) | -CH₂-CH₂-O-CH₂- | | 408 | |
| 2.70 | 1-CH₃-3-CF₃-4-Pyrazolyl | H | H | CH₃ | (CO)N(CH₃)₂ | -CH₂-CH₂-O-CH₂- | | 422 | |
| 2.71 | 1-CH₃-3-CF₃-4-Pyrazolyl | H | H | CH₃ | (CO)N(CH₃)₂ | -CH₂-CH₂-O-CH(CH₃)- | | 436 | 152 |
| 2.72 | 1-CH₃-3-CF₃-4-Pyrazolyl | H | H | CH₃ | (CO)N(CH₃)₂ | -CH₂-CH₂-CH₂-CH(CH₃)- | | 434 | 198 |
| 2.73 | 1-CH₃-3-CF₃-4-Pyrazolyl | H | H | CH₃ | (CO)NH(CH₃) | -CH₂-CH₂-CH₂-CH(CH₃)- | | 420 | 160 |
| 2.74 | 1-CH₃-3-CF₃-4-Pyrazolyl | H | H | CH₃ | (CO)N(CH₃)₂ | -CH₂-CH₂-N(COOC(CH₃)₃)-CH₂- | | 521 | |
| 2.75 | 1-CH₃-3-CF₃-4-Pyrazolyl | H | H | CH₃ | (CO)N(CH₃)₂ | -CH₂-CH₂-CH₂-CH₂-CH₂-CH₂- | | 448 | 188 |
| 2.76 | 1-CH3-3-CF3-4-Pyrazolyl | H | H | CH₃ | H | -C(-NH-CH₃)=C(-O-CH₃)-C(=O)- | | 406 | 120 |
| 2.77 | 1-CH₃-3-CF₃-4-Pyrazolyl | H | H | CH₃ | H | -C(-O-CH₃)=C(-O-CH₃)-C(=O)- | | 407 | |
| 2.78 | 1-CH₃-3-CF₃-4-Pyrazolyl | H | H | CH₃ | H | -C(-O-CH₃)=C(-OH)-C(=O)- | | 393 | 165 |
| 2.79 | 1-CH₃-3-CF₃-4-Pyrazolyl | H | H | CH₃ | (CO)N(CH₃)₂ | -CH₂-CH₂-NH-CH₂- | CF₃CO₂H | 420 | |
| 2.80 | 1-CH₃-3-CF₃-4-Pyrazolyl | H | H | CH₃ | (CO)N(CH₃)₂ | -CH₂-CH₂-N(COO(CH₃))-CH₂- | | 479 | |
| 2.81 | 1-CH₃-3-CF₃-4-Pyrazolyl | H | H | CH₃ | (CO)N(CH₃)₂ | -CH₂-CH₂-CH₂-O-CH₂- | | 436 | 198 |
| 2.82 | 1-CH₃-3-CF₃-4-Pyrazolyl | H | H | CH₃ | (CO)N(CH₃)₂ | -CH₂-CH₂-C(=N-N-CO-CH₃)-CH₂-CH₂- | | 520 | |
| 2.83 | 1-CH₃-3-CF₃-4-Pyrazolyl | H | H | CH₃ | (CO)N(CH₃)₂ | -CH₂-CH₂-C(=N-N-SO₂-CH₃)-CH₂-CH₂- | | 540 | |
| 2.84 | 1-CH₃-3-CF₃-4-Pyrazolyl | H | H | CH₃ | (CO)N(CH₃)₂ | -CH₂-CH₂-C(=N-OH)-CH₂-CH₂- | | 463 | |
| 2.85 | 1-CH₃-3-CF₃-4-Pyrazolyl | H | H | CH₃ | (CO)N(CH₃)₂ | -CH₂-CH₂-C(=N-O-CH₃)-CH₂-CH₂- | | 477 | |
| 2.86 | 1-CH₃-3-CF₃-4-Pyrazolyl | H | H | CH₃ | H | -CH₂-CH₂-CF₂-CH₂-CH₂- | | 399 | |
| 2.87 | 1-CH₃-3-CF₃-4-Pyrazolyl | H | H | CH₃ | (CO)N(CH₃)₂ | -CH₂-CH₂-CF₂-CH₂-CH₂- | | 470 | |
| 2.88 | 1-CH₃-3-CF₃-4-Pyrazolyl | H | H | CH₃ | (CO)NH(CH₃) | -CH₂-CH₂-CF₂-CH₂-CH₂- | | 456 | |
| 2.89 | 1-CH₃-3-CF₃-4-Pyrazolyl | H | H | CH₃ | (CO)CH₃ | 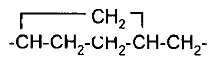 | | 417 | 95 |
| 2.90 | 1-CH₃-3-CF₃-4-Pyrazolyl | H | H | CH₃ | (CO)CH₃ | 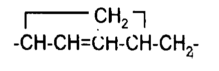 | | 415 | |
| 2.91 | 1-CH₃-3-CF₃-4-Pyrazolyl | H | H | CH₃ | H | 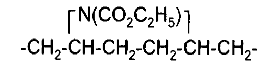 | | 462 | |
| 2.92 | 1-CH₃-3-CF₃-4-Pyrazolyl | H | H | CH₃ | (CO)NH(CH₃) | 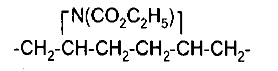 | | 519 | 134 |
| 2.93 | 1-CH₃-3-CF₃-4-Pyrazolyl | H | H | CH₃ | H | -CH₂-CH₂-CH₂-CH(CH₂-CH₂-CH₂-CH₂-CH₃)- | | 419 | 148 |
| 2.94 | 1-CH₃-3-CF₃-4-Pyrazolyl | H | H | CH₂CH₃ | H | -CH₂-CH₂-CH=CH-CH₂-CH₂-CH₂- | | 404 | |
| 2.95 | 1-CH₃-3-CF₃-4-Pyrazolyl | H | H | CH₂CH₃ | H | -CH₂-CH₂-CH₂- | | 350 | |
| 2.96 | 1-CH₃-3-CF₃-4-Pyrazolyl | H | H | CH₂CH₃ | H | 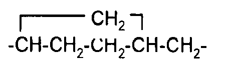 | | 390 | 97 |
| 2.97 | 1-CH₃-3-CF₃-4-Fyrazolyl | H | H | CH₂CH₃ | H | -C(-O-CH(CH₃)₂)=C(-O-CH(CH₃)₂)-C(=O)- | | 478 | |
| 2.98 | 1-CH₃-3-CF₃-4-Pyrazolyl | H | H | CH₃ | H | -CH₂-O-C(CH₃)₂-O-CH₂- | | 395 | 162 |
| 2.99 | 1-CH₃-3-CF₃-4-Pyrazolyl | H | H | CH₃ | H | -CH₂-CH₂-O-CH₂-CH₂- | | 365 | 195 |
| 2.100 | 1-CH₃-3-CF₃-4-Pyrazolyl | H | H | CH₃ | (CO)N(CH₃)₂ | -CH₂-CH₂-O-CH₂-CH₂- | | 436 | 222 |
| 2.101 | 1-CH₃-3-CF₃-4-Pyrazolyl | H | H | CH₃ | (CO)NH(CH₃) | -CH₂-CH₂-O-CH₂-CH₂- | | 422 | 198 |
| 2.102 | 1-CH₃-3-CF₃-4-Pyrazolyl | H | H | CH₃ | (CO)NH₂ | -CH₂-CH₂-O-CH₂-CH₂- | | 408 | 198 |
| 2.103 | 1-CH₃-3-CF₃-4-Pyrazolyl | H | H | CH₃ | H | -CH₂-CH₂-S-CH₂-CH₂- | | 381 | 223 |
| 2.104 | 1-CH₃-3-CF₃-4-Pyrazolyl | H | H | CH₃ | (CO)N(CH₃)₂ | -CH₂-CH₂-S-CH₂-CH₂- | | 452 | 215 |
| 2.105 | 1-CH₃-3-CF₃-4-Pyrazolyl | H | H | CH₃ | (CO)N(CH₃)₂ | -CH₂-CH₂-S(O)-CH₂-CH₂- | | 468 | 225 |
| 2.106 | 1-CH₃-3-CF₃-4-Pyrazolyl | H | H | CH₃ | (CO)N(CH₃)₂ | -CH₂-CH₂-S(O)₂-CH₂-CH₂- | | 484 | |
| 2.107 | 1-CH₃-3-CF₃-4-Pyrazolyl | H | H | CH₃ | H | -CH₂-CH₂-CH₂-CH₂-CH₂- | | 363 | 211 |
| 2.108 | 1-CH₃-3-CF₃-4-Pyrazolyl | H | H | CH₃ | H | -CH₂-CH₂-CH₂-CH₂- | | 349 | 204 |
| 2.109 | 1-CH₃-3-CF₃-4-Pyrazolyl | H | H | CH₃ | (CO)N(CH₃)₂ | -CH₂-CH₂-CH₂-CH₂-CH₂- | | 434 | 195 |
| 2.110 | 1-CH₃-3-CF₃-4-Pyrazolyl | H | H | CH₃ | (CO)NH₂ | -CH₂-CH₂-CH₂-CH₂-CH₂- | | 406 | 205 |
| 2.111 | 1-CH₃-3-CF₃-4-Pyrazolyl | H | H | CH₃ | H | -CH₂-CH₂-O-CH(CH₃)- | | 365 | 210 |
| 2.112 | 1-CH₃-3-CF₃-4-Pyrazolyl | H | H | CH₃ | (CO)N(CH₃)₂ | -CH₂-CH₂-S-CH₂- | | 438 | 205 |
| 2.113 | 1-CH₃-3-CF₃-4-Pyrazolyl | H | H | CH₃ | H | -CH₂-CH₂-S-CH₂- | | 367 | 215 |
| 2.114 | 1-CH₃-3-CF₃-4-Pyrazolyl | H | H | CH₃ | H | -CH₂-CH₂-CH₂-CH(CH₂CH₂CONHCH₃)- | | 434 | 156 |
| 2.115 | 1-CH₃-3-CF₃-4-Pyrazolyl | H | H | CH₃ | H | -CH₂-CH₂-N(COOC(CH₃)₃)-CH₂- | | 450 | 215 |
| 2.116 | 1-CH₃-3-CF₃-4-Pyrazolyl | H | H | CH₃ | (CO)CH₃ | -CH₂-CH₂-CH₂-CH₂-CH₂- | | 405 | 182 |
| 2.117 | 1-CH₃-3-CF₃-4-Pyrazolyl | H | H | CH₃ | (CO)NH(CH₃) | -CH₂-CH₂-CH₂-CH₂-CH₂- | | 420 | 145 |
| 2.118 | 1-CH₃-3-CF₃-4-Pyrazolyl | H | H | CH₃ | H | -CH₂-CH₂-C(-O-CH₂-CH₂-O-)-CH₂-CH₂- | | 421 | 214 |
| 2.119 | 1-CH₃-3-CF₃-4-Pyrazolyl | H | H | CH₃ | (CO)N(CH₃)₂ | -CH₂-CH₂-C(-O-CH₂-CH₂-O-)-CH₂-CH₂- | | 492 | 220 |
| 2.120 | 1-CH₃-3-CF₃-4-Pyrazolyl | H | H | CH₃ | H | -CH₂-CH₂-CH(OSi(CH₃)₂C(CH₃)₃)-CH₂-CH₂- | | 493 | 174 |
| 2.121 | 1-CH₃-3-CF₃-4-Pyrazolyl | H | H | CH₃ | (CO)NH(CH₃) | -CH₂-CH₂-O-CH(CH₃)- | | 422 | 200 |
| 2.122 | 1-CH₃-3-CF₃-4-Pyrazolyl | H | H | CH₃ | H | -CH₂-CH₂-CH₂-CH(CH₃)- | | 363 | 203 |
| 2.123 | 1-CH₃-3-CF₃-4-Pyrazolyl | H | H | CH₃ | H | -CH(CH₃)-CH₂-CH₂-CH(CH₃)- | | 377 | 194 |
| 2.124 | 1-CH₃-3-CF₃-4-Pyrazolyl | H | H | CH₃ | (CO)NH(CH₃) | -CH(CH₃)-CH₂-CH₂-CH(CH₃)- | | 434 | 192 |
| 2.125 | 1-CH₃-3-CF₃-4-Pyrazolyl | H | H | CH₃ | (CO)NH(CH₃) | -CH₂-CH₂-N(COOC(CH₃)₃)-CH₂- | | 507 | |
| 2.126 | 1-CH₃-3-CF₃-4-Pyrazolyl | H | H | CH₃ | H | -CH₂-CH₂-CH₂-CH₂-CH₂-CH₂-CH₂- | | 391 | 175 |
| 2.127 | 1-CH₃-3-CF₃-4-Pyrazolyl | H | H | CH₃ | H | -CH₂-CH₂-CH₂-CH₂-CH₂-CH₂- | | 377 | 185 |
| 2.128 | 1-CH₃-3-CF₃-4-Pyrazolyl | H | H | CH₃ | (CO)NH(CH₃) | -CH₂-CH₂-CH₂-CH₂-CH₂-CH₂- | | 434 | 177 |
| 2.129 | 1-CH₃-3-CF₃-4-Pyrazolyl | H | H | CH₃ | (CO)N(CH₃)₂ | -CH₂-CH₂-N(COO(CH₃)₃)-CH₂-CH₂- | | 535 | 224 |
| 2.130 | 1-CH₃-3-CF₃-4-Pyrazolyl | H | H | CH₃ | H | -CH₂-CH₂-CH₂- | | 335 | 210 |
| 2.131 | 1-CH₃-3-CF₃-4-Pyrazolyl | H | H | CH₃ | (CO)N(CH₃)₂ | -CH₂-CH₂-CH₂- | | 406 | 190 |
| 2.132 | 1-CH₃-3-CF₃-4-Pyrazolyl | H | H | CH₃ | (CO)CH₃ | -CH₂-CH₂-CH₂-CH₂- | | 391 | 170 |
| 2.133 | 1 -CH₃-3-CF₃-4-Pyrazolyl | H | H | CH₃ | (CO)N(CH₃)₂ | -CH₂-CH₂-CH₂-CH₂- | | 420 | 228 |
| 2.134 | 1-CH₃-3-CF₃-4-Pyrazolyl | H | H | CH₃ | (CO)NH(CH₃) | -CH₂-CH₂-CH₂-CH₂- | | 406 | |
| 2.135 | 1-CH₃-3-CF₃-4-Pyrazolyl | H | H | CH₃ | (CO)NH₂ | -CH₂-CH₂-CH₂-CH₂- | | 392 | 218 |
| 2.136 | 1-CH₃-3-CF₃-4-Pyrazolyl | H | H | CH₃ | (CO)NH(CH₃) | -CH₂-CH₂-CH₂- | | 392 | 200 |
| 2.137 | 1-CH₃-3-CF₃-4-Pyrazolyl 1-CH₃-3-CF₃-4-Pyrazolyl | H | H | CH₃ | H | 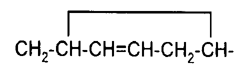 | | 373 | 132 |
| 2.138 | | H | H | CH₃ | H | 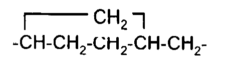 | | 375 | 210 |
| 2.139 | 1-CH₃-3-CF₃-4-Pyrazolyl | H | H | CH₃ | H | 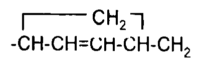 | | 373 | 192 |
| 2.140 | 1-CH₃-3-CF₃-4-Pyrazolyl 1-CH₃-3-CF₃-4-Pyrazolyl | H | H | CH₃ | (CO)NH(CH₃) | 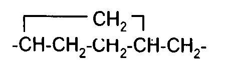 | | 431 | 194 |
| 2.141 | 1-CH₃-3-CF₃-4-Pyrazolyl | H | H | CH₃ | (CO)N(CH₃)₂ | 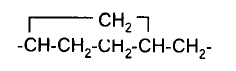 | | 446 | 207 |
| 2.142 | | H | H | CH₃ | (CO)NH₂ | 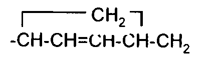 | | 416 | 190 |
| 2.143 | 1-CH₃-3-CF₃-4-Pyrazolyl | H | H | CH₃ | (CO)N(CH₃)₂ | -CH₂-CH₂-CH(OSi(CH₃)₂C(CH₃)₃)-CH₂-CH₂- | | 537 | 175 |
| 2.144 | 1-CH₃-3-CF₃-4-Pyrazolyl | H | H | CH₃ | (CO)NH₂ | 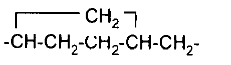 | | 418 | 182 |
| 2.145 | 1-CH₃-3-CF₃-4-Pyrazolyl 1-CH₃-3-CF₃-4-Pyrazolyl | H | H | CH₃ | (CO)N(CH₃)₂ | 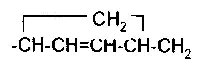 | | 444 | 210 |
| 2.146 | | H | H | CH₃ | (CO)NH(CH₃) | 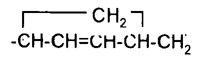 | | 429 | 95 |
| 2.147 | 1-CH₃-3-CF₃-4-Pprazolyl | H | H | CH₃ | (CO)N(CH₃)₂ | 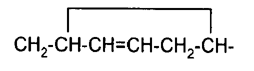 | | 444 | 156 |
| 2.148 | 1-CH₃-3-CF₃-4-Pyrazolyl | H | H | CH₃ | (CO)NH(CH₃) | 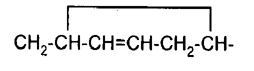 | | 429 | 182 |
| 2.149 | 1-CH₃-3-CF₃-4-Pyrazolyl | H | H | CH₃ | (CO)CH₃ | -CH₂-CH₂-CH₂- | | 377 | 190 |
| 2.150 | 1-CH₃-3-CF₃-4-Pyrazolyl | H | H | CH₃ | (CO)N(CH₃)₂ | -CH₂-CH₂-CH(OH)-CH₂-CH₂- | | 450 | |
| 2.151 | 1-CH₃-3-CF₃-4-Pyrazolyl | H | H | CH₃ | H | -CH₂-CH₂-CH(OH)-CH₂-CH₂- | | 379 | 80 |
| 2.152 | 1-CH₃-3-CF₃-4-Pyrazolyl | H | H | CH₃ | (CO)N(CH₃)₂ | -CH₂-CH₂-C(O)-CH₂-CH₂- | | 448 | 188 |
| 2.153 | 1-CH₃-3-CF₃-4-Pyrazolyl | H | H | CH₃ | H | -CH₂-CH₂-CH₂-O-CH₂- | | 365 | |
| 2.154 | 1-CH₃-3-CF₃-4-Pyrazolyl | H | H | CH₃ | H | -CH₂-CH₂-N(CON(CH₃)₂)-CH₂-CH₂- | | 435 | 222 |
| 2.155 | 1-CH₃-3-CF₃-4-Pyrazolyl | H | H | CH₃ | H | -CH₂-CH₂-N(COCH₃)-CH₂-CH₂- | | 406 | |
| 2.156 | 1-CH₃-3-CF₃-4-Pyrazolyl | H | H | CH₃ | H | -CH₂-CH₂-NH-CH₂-CH₂- | HCl | 364 | |
| 2.157 | 1-CH₃-3-CF₃-4-Pyrazolyl | H | H | CH₃ | (CO)N(CH₃)₂ | -CH₂-CH₂-N(CON(CH₃)₂)-CH₂-CH₂- | | 596 | 184 |
| 2.158 | 1-CH₃-3-CF₃-4-Pyrazolyl | H | H | CH₃ | (CO)N(CH₃)₂ | -CH₂-CH₂-N(COCH₃)-CH₂-CH₂- | | 477 | 88 |
| 2.159 | 1-CH₃-3-CF₃-4-Pyrazolyl | H | H | CH₃ | (CO)N(CH₃)₂ | -CH₂-CH₂-N(CH₃)-CH₂-CH₂- | | 449 | 212 |
| 2.160 | 1-CH₃-3-CF₃-4-Pyrazolyl | H | H | CH₃ | (CO)N(CH₃)₂ | -CH₂-CH₂-NH-CH₂-CH₂- | HCl | 471 | 175 |
| 2.161 | 1-CH₃-3-CF₃-4-Pyrazolyl | H | H | CH₃ | (CO)N(CH₃)₂ | -CH₂-CH₂-N(CH₂CN)-CH₂-CH₂- | | 474 | 197 |
| 2.162 | 1-CH₃-3-CF₃-4-Pyrazolyl | H | H | CH₃ | H | -CH₂-CH₂-O-CH₂- | | 351 | |
| 2.163 | 1-CH₃-3-CF₃-4-Pyrazolyl | H | H | CH₃ | H | -CH₂-O-CH₂- | | 337 | |
| 2.164 | 1-CH₃-3-CF₃-4-Pyrazolyl | H | H | CH₃ | (CO)CH₃ | 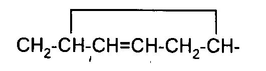 | | 415 | |
| 2.165 | 1-CH₃-3-CF₃-4-Pyrazolyl | H | H | CH₃ | (CO)N(CH₃)₂ | -CH₂-O-CH₂- | | 408 | |
| 2.166 | 1-CH₃-3-CF₃-4-Pyrazolyl | H | H | CH₃ | (CO)NH(CH₃) | -CH₂-CH₂-O-CH₂- | | 408 | |
| 2.167 | 1-CH₃-3-CF₃-4-Pyrazolyl | H | H | CH₃ | (CO)N(CH₃)₂ | -CH₂-CH₂-O-CH₂- | | 422 | |
| 2.168 | 1-CH₃-3-CF₃-4-Pyrazolyl | H | H | CH₃ | (CO)N(CH₃)₂ | -CH₂-CH₂-O-CH(CH₃)- | | 436 | 152 |
| 2.169 | 1-CH₃-3-CF₃-4-Pyrazolyl | H | H | CH₃ | (CO)N(CH₃)₂ | -CH₂-CH₂-CH₂-CH(CH₃)- | | 434 | 198 |
| 2.170 | 1-CH₃-3-CF₃-4-Pyrazolyl | H | H | CH₃ | (CO)NH(CH₃) | -CH₂-CH₂-CH₂-CH(CH₃)- | | 420 | 160 |
| 2.171 | 1-CH₃-3-CF₃-4-Pyrazolyl | H | H | CH₃ | (CO)N(CH₃)₂ | -CH₂-CH₂-N(COOC(CH₃)₃)-CH₂- | | 521 | |
| 2.172 | 1-CH₃-3-CF₃-4-Pyrazolyl | H | H | CH₃ | (CO)N(CH₃)₂ | -CH₂-CH₂-CH₂-CH₂-CH₂-CH₂- | | 448 | 188 |
| 2.173 | 1-CH₃-3-CF₃-4-Pyrazolyl | H | H | CH₃ | H | -C(-NH-CH₃)=C(-O-CH₃)-C(=O)- | | 406 | 120 |
| 2.174 | 1-CH₃-3-CF₃-4-Pyrazolyl | H | H | CH₃ | H | -C(-O-CH₃)=C(-O-CH₃)-C(=O)- | | 407 | |
| 2.175 | 1-CH₃-3-CF₃-4-Pyrazolyl | H | H | CH₃ | H | -C(-O-CH₃)=C(-OH)-C(=O)- | | 393 | 165 |
| 2.176 | 1-CH₃-3-CF₃-4-Pyrazolyl | H | H | CH₃ | (CO)N(CH₃)₂ | -CH₂-CH₂-NH-CH₂- | CF₃CO₂H | 420 | |
| 2.177 | 1-CH₃-3-CF₃-4-Pyrazolyl | H | H | CH₃ | (CO)N(CH₃)₂ | -CH₂-CH₂-N(COO(CH₃))-CH₂- | | 479 | |
| 2.178 | 1-CH₃-3-CF₃-4-Pyrazolyl | H | H | CH₃ | (CO)N(CH₃)₂ | -CH₂-CH₂-CH₂-O-CH₂- | | 436 | 198 |
| 2.179 | 1-CH₃-3-CF₃-4-Pyrazolyl | H | H | CH₃ | (CO)N(CH₃)₂ | -CH₂-CH₂-C(=N-N-CO-CH₃)-CH₂-CH₂- | | 520 | |
| 2.180 | 1-CH₃-3-CF₃-4-Pyrazolyl | H | H | CH₃ | (CO)N(CH₃)₂ | -CH₂-CH₂-C(=N-N-SO₂-CH₃)-CH₂-CH₂- | | 540 | |
| 2.181 | 1-CH₃-3-CF₃-4-Pyrazolyl | H | H | CH₃ | (CO)N(CH₃)₂ | -CH₂-CH₂-C(=N-OH)-CH₂-CH₂- | | 463 | |
| 2.182 | 1-CH₃-3-CF₃-4-Pyrazolyl | H | H | CH₃ | (CO)N(CH₃)₂ | -CH₂-CH₂-C(=N-O-CH₃)-CH₂-CH₂- | | 477 | |
| 2.183 | 1-CH₃-3-CF₃-4-Pyrazolyl | H | H | CH₃ | H | -CH₂-CH₂-CF₂-CH₂-CH₂- | | 399 | |
| 2.184 | 1-CH₃-3-CF₃-4-Pyrazolyl | H | H | CH₃ | (CO)N(CH₃)₂ | -CH₂-CH₂-CF₂-CH₂-CH₂- | | 470 | |
| 2.185 | 1-CH₃-3-CF₃-4-Pyrazolyl | H | H | CH₃ | (CO)NH(CH₃) | -CH₂-CH₂-CF₂-CH₂-CH₂- | | 456 | |
| 2.186 | 1-CH₃-3-CF₃-4-Pyrazolyl | H | H | CH₃ | (CO)CH₃ | 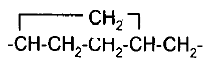 | | 417 | 95 |
| 2.187 | 1-CH₃-3-CF₃-4-Pyrazolyl | H | H | CH₃ | (CO)CH₃ | 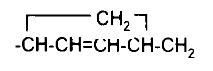 | | 415 | |
| 2.188 | 1-CH₃-3-CF₃-4-Pyrazolyl | H | H | CH₃ | H | 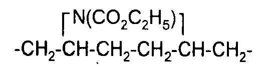 | | 462 | |
| 2.189 | 1-CH₃-3-CF₃-4-Pyrazolyl | H | H | CH₃ | (CO)NH(CH₃) | 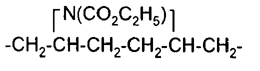 | | 519 | 134 |
| 2.190 | 1-CH₃-3-CF₃-4-Pyrazolyl | H | H | CH₃ | H | -CH₂-CH₂-CH₂-CH(CH₂-CH₂-CH₂-CH₂-CH₃)- | | 419 | 148 |

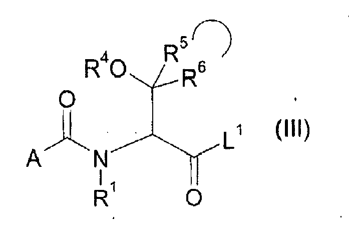

**Tabelle 3 (Zwischenprodukte der Formel III)**

| Nr. | A | R¹ | L¹ | R⁴ | R⁵ + R⁶ | MS (m⁺ + H) | Fp (°C) |
|---|---|---|---|---|---|---|---|
| 3.1 | 1-CH₃-3-CF₃-4-Pyrazolyl | H | -O-CH₂-CH₃ | H | -CH₂-CH₂-CH₂- | 350 | |
| 3.2 | 1-CH₃-3-CF₃-4-Pyrazolyl | H | -O-CH₂-CH₃ | H | -CH₂-CH(O-CH₂CH(CH₃)₂)-CH2- | 422 | |
| 3.3 | 1-CH₃-3-CF₃-4-Pyrazolyl | H | -O-CH₂-CH₃ | H | -CH₂-CH₂-CH(CH₃)-CH₂-CH₂- | 392 | |
| 3.4 | 1-CH₃-3-CF₃-4-Pyrazolyl | H | -O-CH₂-CH₃ | H | -CH₂-CH₂-N(O-CH₂-CH₃)-CH₂-CH₂- | 423 | 120 |
| 3.4 | 1-CH₃-3-CF₃-4-Pyrazolyl | H | -O-CH₂-CH₃ | H | -CH₂-CH₂-CH(CF₃)-CH₂-CH₂- | 446 | 140 |

### Biologische Wirksamkeit

Die Verbindungen der Formel (I) und deren landwirtschaftlich brauchbaren Salze eignen sich - sowohl als Isomerengemische als auch in Form der reinen Isomeren - als Herbizide. Die Verbindungen der Formel (I) enthaltenden herbiziden Mittel bekämpfen Pflanzenwuchs auf Nichtkulturflächen sehr gut, besonders bei hohen Aufwandmengen. In Kulturen wie Weizen, Reis, Mais, Soja und Baumwolle wirken sie gegen Unkräuter und Schadgräser, ohne die Kulturpflanzen nennenswert zu schädigen. Dieser Effekt tritt vor allem bei niedrigen Aufwandmengen auf.

In Abhängigkeit von der jeweiligen Applikationsmethode können die Verbindungen der Formel (I) bzw. sie enthaltenden herbiziden Mittel noch in einer weiteren Zahl von Kulturpflanzen zur Beseitigung unerwünschter Pflanzen eingesetzt werden. In Betracht kommen beispielsweise folgende Kulturen:
Allium cepa, Ananas comosus, Arachis hypogaea, Asparagus officinalis, Avena sativa Beta vulgaris spec. altissima, Beta vulgaris spec. rapa, Brassica napus var. napus, Brassica napus var. napobrassica, Brassica rapa var. silvestris, Brassica oleracea, Brassica nigra, Camellia sinensis, Carthamus tinctorius, Carya illinoinensis, Citrus limon, Citrus sinensis, Coffea arabica (Coffea canephora, Coffea liberica), Cucumis sativus, Cynodon dactylon, Daucus carota, Elaeis guineensis, Fragaria vesca, Glycine max, Gossypium hirsutum, (Gossypium arboreum, Gossypium herbaceum, Gossypium vitifolium), Helianthus annuus, Hevea brasiliensis, Hordeum vulgare, Humulus lupulus, Ipomoea batatas, Juglans regia, Lens culinaris, Linum usitatissimum, Lycopersicon lycopersicum, Malus spec., Manihot esculenta, Medicago sativa, Musa spec., Nicotiana tabacum (N.rustica), Olea europaea, Oryza sativa, Phaseolus lunatus, Phaseolus vulgaris, Picea abies, Pinus spec., Pistacia vera, Pisum sativum, Prunus armeniaca, Prunus avium, Prunus cerasus, Prunus dulcis, Prunus domestica, Prunus persica, Pyrus communis, Ribes sylvestre, Ricinus communis, Saccharum officinarum, Secale cereale, Sinapis alba, Solanum tuberosum, Sorghum bicolor (s. vulgare), Theobroma cacao, Trifolium pratense, Triticale, Triticum aestivum, Triticum durum, Vicia faba, Vitis vinifera und Zea mays.

Darüber hinaus können die Verbindungen der Formel (I) auch in Kulturen, die durch Züchtung einschließlich gentechnischer Methoden gegen die Wirkung von Herbiziden tolerant sind, verwendet werden.

Darüber hinaus können die Verbindungen der Formel (I) auch in Kulturen, die durch Züchtung einschließlich gentechnischer Methoden gegen Insekten- oder Pilzbefall tolerant sind, verwendet werden.

Die Verbindungen der Formel (I) bzw. die sie enthaltenden herbiziden Mittel können beispielsweise in Form von direkt versprühbaren wässrigen Lösungen, Pulvern, Suspensionen, auch hochprozentigen wässrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die herbiziden Mittel enthalten eine herbizid wirksame Menge mindestens einer Verbindung der Formel (I) oder eines landwirtschaftlich brauchbaren Salzes von (I) und für die Formulierung von Pflanzenschutzmitteln übliche Hilfsmittel.

Beispiele für die Formulierung von Pflanzenschutzmitteln übliche Hilfsmittel sind inerte Hilfsstoffe, feste Trägerstoffe, oberflächenaktive Stoffe (wie Dispergiermittel, Schutzkolloide, eEmulgatoren, Netzmittel und Haftmittel), organische und anorganische Verdicker, Bakterizide, Frostschutzmittel, Entschäumer ggf. Farbstoffe und für Saatgutformulierungen Kleber.

Beispiele für Verdicker (d.h. Verbindungen, die der Formulierung ein modifiziertes Fließverhalten verleihen, d.h. hohe Viskosität im Ruhezustand und niedrige Viskosität im bewegten Zustand) sind Polysaccharide sowie organische und anorganische Schichtmineralien wie Xanthan Gum (Kelzan® der Fa. Kelco), Rhodopol® 23 (Rhone Poulenc) oder Veegum® (Firma R.T. Vanderbilt) oder Attaclay® (Firma Engelhardt).

Beispiele für Antischaummittel sind Silikonemulsionen (wie z.B. Silikon® SRE, Firma Wacker oder Rhodorsil® der Firma Rhodia), langkettige Alkohole, Fettsäuren, Salze von Fettsäuren, fluororganische Verbindungen und deren Gemische.

Bakterizide können zur Stabilisierung der wässrigen Herbizid-Formulierung zugesetzt werden. Beispiele für Bakterizide sind Bakterizide basierend auf Diclorophen und Benzylalkoholhemiformal (Proxel® der Fa. ICI oder Acticide® RS der Fa. Thor Chemie und Kathon® MK der Firma Rohm & Haas) sowie Isothiazolinonderivaten wie Alkylisothiazolinonen und Benzisothiazolinonen (Acticide MBS der Fa. Thor Chemie)

Beispiele für Frostschutzmittel sind Ethylenglycol, Propylenglycol, Harnstoff oder Glycerin.

Beispiele für Farbmittel sind sowohl in Wasser wenig lösliche Pigmente als auch in Wasser lösliche Farbstoffe. Als Beispiele genannt seien die unter den Bezeichnungen Rhodamin B, C.I. Pigment Red 112 und C.I. Solvent Red 1 bekannten Farbstoffe, sowie pigment blue 15:4, pigment blue 15:3, pigment blue 15:2, pigment blue 15:1, pigment blue 80, pigment yellow 1, pigment yellow 13, pigment red 112, pigment red 48:2, pigment red 48:1, pigment red 57:1, pigment red 53:1, pigment orange 43, pigment orange 34, pigment orange 5, pigment green 36, pigment green 7, pigment white 6, pigment brown 25, basic violet 10, basic violet 49, acid red 51, acid red 52, acid red 14, acid blue 9, acid yellow 23, basic red 10, basic red 108

Beispiele für Kleber sind Polyvinylpyrrolidon, Polyvinylacetat, Polyvinylalkohol und Tylose.

Als inerte Hilfsstoffe kommen im Wesentlichen in Betracht:
Mineralölfraktionen von mittlerem bis hohem Siedepunkt wie Kerosin und Dieselöl, ferner Kohlenteeröle sowie Öle pflanzlichen oder tierischen Ursprungs, aliphatische, cyclische und aromatische Kohlenwasserstoffe, z.B. Paraffine, Tetrahydronaphthalin, alkylierte Naphthaline und deren Derivate, alkylierte Benzole und deren Derivate, Alkohole wie Methanol, Ethanol, Propanol, Butanol und Cyclohexanol, Ketone wie Cyclohexanon, stark polare Lösungsmittel, z.B. Amine wie N-Methylpyrrolidon und Wasser.

Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nussschalenmehl, Cellulosepulver oder andere feste Trägerstoffe.

Als oberflächenaktive Stoffe (Adjuvantien, Netz-, Haft-, Dispergier- oder Emulgiermittel) kommen die Alkali-, Erdalkali-, Ammoniumsalze von aromatischen Sulfonsäuren, z.B. Lignin- (Borresperse typen Borregaard), Phenol-, Naphthalin-(Morwet typen, Akzo Nobel) und Dibutylnaphthalinsulfonsäure (Nekal typen BASF), sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenyl-, Tributylphenylpolyglykolether, Alkylarylpolyetheralkohole, Isotridecylalkohol, Alkoxylate wie zum Beispiel, Fettalkoholethylenoxid-Kondensate, ethoxyliertes Rizinusöl, Polyoxyethylen- oder Polyoxypropylenalkylether, Laurylalkoholpolyglykoletheracetat, Sorbitester, Lignin-Sulfitablaugen oder Methylcellulose in Betracht sowie Proteine, denaturierte Proteine, Polysaccharide (z.B. Methylcellulose), hydrophob modifizierte Stärken, Polyvinylalkohol (Mowiol typen Clariant), Polycarboxylate (BASF Sokalan typen), Polyalkoxylate, Polyvinylamin (BASF Lupamin typen), Polyethylenimin (BASF Lupasol typen), Polyvinylpyrrolidon und deren Copolymere in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte wie Getreidemehl, Baumrinden-, Holz- und Nussschalenmehl, Cellulosepulver oder andere feste Trägerstoffe.

Wässrige Anwendungsformen können aus Emulsionskonzentraten, Suspensionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Die Konzentrationen der Verbindungen der Formel (I) in den anwendungsfertigen Zubereitungen können in weiten Bereichen variiert werden. Im Allgemeinen enthalten die Formulierungen etwa von 0,001 bis 98 Gew.-%, vorzugsweise 0,01 bis 95 Gew.-%, mindestens eines Wirkstoffs. Die Wirkstoffe werden dabei in einer Reinheit von 90% bis 100%, vorzugsweise 95% bis 100% (nach NMR-Spektrum) eingesetzt.

Beispiele für Formulierungen sind:

### 1. Produkte zur Verdünnung in Wasser

### A Wasserlösliche Konzentrate

10 Gew.-Teile der Wirkstoffe werden mit 90 Gew.-Teilen Wasser oder einem wasserlöslichen Lösungsmittel gelöst. Alternativ werden Netzmittel oder andere Hilfsmittel zugefügt. Bei der Verdünnung in Wasser löst sich der Wirkstoff. Man erhält auf diese Weise eine Formulierung mit 10 Gew.-% Wirkstoffgehalt.

### B Dispergierbare Konzentrate

20 Gew.-Teile der Wirkstoffe werden in 70 Gew.-Teilen Cyclohexanon unter Zusatz von 10 Gew.-Teilen eines Dispergiermittels z.B. Polyvinylpyrrolidon gelöst. Bei Verdünnung in Wasser ergibt sich eine Dispersion. Der Wirkstoffgehalt beträgt 20 Gew.-%

### C Emulgierbare Konzentrate

15 Gew.-Teile der Wirkstoffe werden in 75 Gew.-Teilen eines organischen Lösungsmittels unter Zusatz von Ca-Dodecylbenzolsulfonat und Ricinusölethoxylat (jeweils 5 Gew.-Teile) gelöst. Bei der Verdünnung in Wasser ergibt sich eine Emulsion. Die Formulierung hat 15 Gew.-% Wirkstoffgehalt.

### D Emulsionen

25 Gew.-Teile der Wirkstoffe werden in 35 Gew.-Teilen eines organischen Lösungsmittels unter Zusatz von Ca-Dodecylbenzolsulfonat und Ricinusölethoxylat (jeweils 5 Gew.-Teile) gelöst. Diese Mischung wird mittels einer Emulgiermaschine (z.B. Ultraturax) in 30 Gew.Teile Wasser gegeben und zu einer homogenen Emulsion gebracht. Bei der Verdünnung in Wasser ergibt sich eine Emulsion. Die Formulierung hat einen Wirkstoffgehalt von 25 Gew.-%.

### E Suspensionen

20 Gew.-Teile der Wirkstoffe werden unter Zusatz von 10 Gew.-Teilen Dispergier- und Netzmitteln und 70 Gew.-Teilen Wasser oder einem organischen Lösungsmittel in einer Rührwerkskugelmühle zu einer feinen Wirkstoffsuspension zerkleinert. Bei der Verdünnung in Wasser ergibt sich eine stabile Suspension des Wirkstoffs. Der Wirkstoffgehalt in der Formulierung beträgt 20 Gew.-%.

### F Wasserdispergierbare und wasserlösliche Granulate

50 Gew.-Teile der Wirkstoffe werden unter Zusatz von 50 Gew-Teilen Dispergier- und Netzmitteln fein gemahlen und mittels technischer Geräte (z.B. Extrusion, Sprühturm, Wirbelschicht) als wasserdispergierbare oder wasserlösliche Granulate hergestellt. Bei der Verdünnung in Wasser ergibt sich eine stabile Dispersion oder Lösung des Wirkstoffs. Die Formulierung hat einen Wirkstoffgehalt von 50 Gew.-%.

### G Wasserdispergierbare und wasserlösliche Pulver

75 Gew.-Teile der Wirkstoffe werden unter Zusatz von 25 Gew.-Teilen Dispergier- und Netzmitteln sowie Kieselsäuregel in einer Rotor-Strator Mühle vermahlen. Bei der Verdünnung in Wasser ergibt sich eine stabile Dispersion oder Lösung des Wirkstoffs. Der Wirkstoffgehalt der Formulierung beträgt 75 Gew.-%.

### H Gelformulierungen

In einer Kugelmühle werden 20 Gew.-Teile der Wirkstoffe, 10 Gew.-Teile Dispergiermittel, 1Gew.-Teil Geliermittel und 70 Gew.-Teile Wasser oder eines organischen Lösungsmittels zu einer feinen Suspension vermahlen. Bei der Verdünnung mit Wasser ergibt sich eine stabile Suspension mit 20 Gew.-% Wirkstoffgehalt.

### 2. Produkte für die Direktapplikation

### I Stäube

5 Gew.-Teile der Wirkstoffe werden fein gemahlen und mit 95 Gew.-Teilen feinteiligem Kaolin innig vermischt. Man erhält dadurch ein Stäubemittel mit 5 Gew.-% Wirkstoffgehalt.
J Granulate (GR, FG, GG, MG)
0,5 Gew-Teile der Wirkstoffe werden fein gemahlen und mit 99,5 Gewichtsteilen Trägerstoffe verbunden. Gängige Verfahren sind dabei die Extrusion, die Sprühtrocknung oder die Wirbelschicht. Man erhält dadurch ein Granulat für die Direktapplikation mit 0,5 Gew.-% Wirkstoffgehalt.
K ULV- Lösungen (UL)
10 Gew.-Teile der Wirkstoffe werden in 90 Gew.-Teilen eines organischen Lösungsmittels z.B. Xylol gelöst. Dadurch erhält man ein Produkt für die Direktapplikation mit 10 Gew.-% Wirkstoffgehalt.

Die Applikation der Verbindungen der Formel (I) bzw. der herbiziden Mittel kann im Vorauflauf- oder im Nachauflaufverfahren erfolgen. Sind die Wirkstoffe für gewisse Kulturpflanzen weniger verträglich, so können Ausbringungstechniken angewandt werden, bei welchen die herbiziden Mittel mit Hilfe der Spritzgeräte so gespritzt werden, dass die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

In einer weiteren Ausführungsform kann die Applikation der Verbindungen der Formel (I) bzw. der herbiziden Mittel durch Behandlung von Saatgut erfolgen.

Behandlung von Saatgut umfasst im Wesentlichen alle dem Fachmann geläufigen Techniken (seed dressing, seed coating, seed dusting, seed soaking, seed film coating, seed multilayer coating, seed encrusting, seed dripping, und seed pelleting") basierend auf den erfindungsgemäßen Verbindungen der Formel (I) bzw. daraus hergestellten Mitteln. Hierbei können die herbiziden Mittel verdünnt oder unverdünnt aufgetragen werden.

Der Begriff Saatgut umfasst Saatgut aller Arten, wie z.B. Körner, Samen, Früchte, Knollen, Stecklinge und ähnliche Formen. Bevorzugt beschreibt der Begriff Saatgut hier Körner und Samen.

Als Saatgut kann Saatgut der oben erwähnten Nutzpflanzen aber auch das Saatgut transgener oder durch herkömmliche Züchtungsmethoden erhaltener Pflanzen eingesetzt werden.

Die Aufwandmengen an Verbindung der Formel (I) betragen je nach Bekämpfungsziel, Jahreszeit, Zielpflanzen und Wachstumsstadium 0,001 bis 3,0, vorzugsweise 0,01 bis 1,0 kg/ha aktive Substanz (a.S.).

Zur Saatgutbehandlung werden üblicherweise Mengen von 0,001 bis 10kg pro 100kg Saatgut eingesetzt.

Zur Verbreiterung des Wirkungsspektrums und zur Erzielung synergistischer Effekte können die 3-(Heterocyclyl)-substituierten BenzoylpyrazoleVerbindungen der Formel (I) mit zahlreichen Vertretern anderer herbizider oder wachstumsregulierender Wirkstoffgruppen gemischt und gemeinsam ausgebracht werden. Beispielsweise kommen als Mischungspartner 1,2,4-Thiadiazole, 1,3,4-Thiadiazole, Amide, Aminophosphorsäure und deren Derivate, Aminotriazole, Anilide, Aryloxy-/Heteroaryloxyalkansäuren und deren Derivate, Benzoesäure und deren Derivate, Benzothiadiazinone, 2-(Hetaroyl/Aroyl)-1,3-cyclohexandione, Heteroaryl-Aryl-Ketone, Benzylisoxazolidinone, meta-CF₃-Phenylderivate, Carbamate, Chinolincarbonsäure und deren Derivate, Chloracetanilide, Cyclohexenonoximetherderivate, Diazine, Dichlorpropionsäure und deren Derivate, Dihydrobenzofurane, Dihydrofuran-3-one, Dinitroaniline, Dinitrophenole, Diphenylether, Dipyridyle, Halogencarbonsäuren und deren Derivate, Harnstoffe, 3-Phenyluracile, Imidazole, Imidazolinone, N-Phenyl-3,4,5,6-tetrahydrophthalimide, Oxadiazole, Oxirane, Phenole, Arylöxy- und Heteroaryloxyphenoxypropionsäureester, Phenylessigsäure und deren Derivate, 2-Phenylpropionsäure und deren Derivate, Pyrazole, Phenylpyrazole, Pyridazine, Pyridincarbonsäure und deren Derivate, Pyrimidylether, Sulfonamide, Sulfonylharnstoffe, Triazine, Triazinone, Triazolinone, Triazolcarboxamide und Uracile sowie Phenylpyrazoline, Isoxazoline und deren Derivate in Betracht.

Außerdem kann es von Nutzen sein, die Verbindungen der Formel (I) allein oder in Kombination mit anderen Herbiziden auch noch mit weiteren Pflanzenschutzmitteln gemischt, gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- und Spurenelementmängeln eingesetzt werden. Es können auch weitere Additive wie nichtphytotoxische Öle und Ölkonzentrate zugesetzt werden.

### Anwendungsbeispiele

Die herbizide Wirkung der heteroaroylsubstituierten Serin-Amide der Formel (I) ließ sich durch die folgenden Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern, und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zweck der Nachauflaufbehandlung wurden die Testpflanzen je nach Wuchsform erst bis zu einer Wuchshöhe von 3 bis 15 cm angezogen und erst dann mit den in Wasser suspendierten oder emulgierten Wirkstoffen behandelt. Die Testpflanzen wurden dafür entweder direkt gesät und in den gleichen Gefäßen aufgezogen oder sie wurden erst als Keimpflanzen getrennt angezogen und einige Tage vor der Behandlung in die Versuchsgefäße verpflanzt. Die Aufwandmenge für die Nachauflaufbehandlung betrug 1,0 kg/ha a.S. (aktive Substanz).

Die Pflanzen wurden artenspezifisch bei Temperaturen von 10 bis 25°C bzw. 20 bis 35°C gehalten. Die Versuchsperiode erstreckte sich über 2 bis 4 Wochen. Während dieser Zeit wurden die Pflanzen gepflegt, und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| | |
|---|---|
| Lateinischer Name | Deutscher Name |
| Amaranthus retroflexus | Fuchsschwanz |
| Chenopodium album | Weißer Gänsefuß |
| Setaria viridis | Grüne Borstenhirse |

Bei Aufwandmengen von 1 kg/ha zeigten die Verbindungen 2.2, 2.7, 2.9, 2.12, 2.14, 2.15, 2.19, 2.21, 2.22, 2.24, 2.27, 2.28, 2.31, 2.34, 2.35, 2.36, 2.38, 2.41, 2.43, 2.44, 2.46, 2.48, 2.50, 2.51, 2.53, 2.55, 2.56, 2.57, 2.60, 2.61, 2.71, 2.72, 2.74, 2.80, 2.82, 2.83, 2.85, 2.86, 2.87, 2.88, 2.100, 2.101, 2.102, 2.104, 2.105, 2.106, 2.107, 2.109, 2.111, 2.112, 2.116, 2.117, 2.118, 2.119, 2.121, 2.124, 2.125, 2.128, 2.131, 2.132, 2.133, 2.135, 2.136, 2.137, 2.138, 2.140, 2.141, 2.143, 2.145, 2.147, 2.148, 2.149, 2.150, 2.152, 2.153, 2.154, 2.157, 2.158, 2.164, 2.168, 2.169, 2.170, 2.171, 2.177, 2.179, 2.180, 2.181, 2.182, 2.183, 2.184, 2.185, 2.186, 2.187, 2.188, 2.189 und 2.190 im Nachauflauf eine sehr gute Wirkung (> 80%) gegen die unerwünschten Pflanzen Amaranthus retroflexus, Chenopodium album und Setaria viridis.

## Patentansprüche

1. Heteroaroylsubstituierte Serin-Amide der Formel (I), 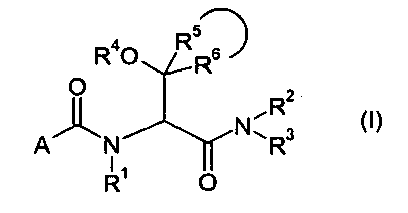 in der die Variablen die folgenden Bedeutungen haben:
A 5- oder 6-gliedriges Heteroaryl mit ein bis vier Stickstoffatomen, oder mit ein bis drei Stickstoffatomen und einem Sauerstoff- oder Schwefelatom, oder mit einem Sauerstoff- oder Schwefelatom, welches partiell oder vollständig halogeniert sein kann und/oder 1 bis 3 Reste aus der Gruppe Cyano, C₁-C₆-Alkyl, C₃-C₆- Cycloalkyl, C₁-C₆-Halogerlalkyl, C₁-C₆-Alkoxy, C₁-C₆-Halogenalkoxy und C₁-C₆- Alkoxy-C₁-C₄-alkyl tragen kann;
R¹, R² Wasserstoff, Hydroxy oder C₁-C₆-Alkoxy;
R³ C₁-C₆-Alkyl, C₁-C₄-Cyanoalkyl oder C₁-C₆-Halogenalkyl;
R⁴ Wasserstoff, C₁-C₆-Alkyl, C₃-C₆-Cycloalkyl, C₃-C₆-Alkenyl, C₃-C₆-Alkinyl, C₃-C₆- Halogenalkenyl, C₃-C₆-Halogenalkinyl, Formyl, C₁-C₆-Alkylcarbonyl, C₃-C₆-Cyclo- alkylcarbonyl, C₂-C₆-Alkenylcarbonyl, C₂-C₆-Alkinylcarbonyl, C₁-C₆-Alkoxycarbo- nyl, C₃-C₆-Alkenyloxycarbonyl, C₃-C₆-Alkinyloxycarbonyl, Aminocarbonyl, C₁-C₆- Alkylaminocarbonyl, C₃-C₆-Alkenylaminocarbonyl, C₃-C₆-Alkinylaminocarbonyl, C₁-C₆-Alkylsulfonylaminocarbonyl, Di-(C₁-C₆-alkyl)-aminocarbonyl, N-(C₃-C₆- Alkenyl)-N-(C₁-C₆-alkyl)-aminocarbonyl, N-(C₃-C₆-Alkinyl)-N-(C₁-C₆-alkyl)- aminocarbonyl, N-(C₁-C₆-Alkoxy)-N-(C₁-C₆-alkyl)-amino-carbonyl, N-(C₃-C₆- Alkenyl)-N-(C₁-C₆-alkoxy)-aminocarbonyl, N-(C₃-C₆-Alkinyl)-N-(C₁-C₆-alkoxy)- aminocarbonyl, (C₁-C₆)Alkylaminothiocarbonyl, Di-(C₁-C₆-alkyl)- aminothiocarbonyl, (C₁-C₆-Alkyl)cyanoimino, (Amino)cyanoimino, [(C₁-C₆- Alkyl)amino]cyanoimino, [Di(C₁-C₆-Alkyl)amino]cyanoimino, C₁-C₆-Alkylcarbonyl- C₁-C₆-alkyl, C₁-C₆-Alkoxyimino-C₁-C₆-alkyl, N-(C₁-C₆-Alkylamino)-imino-C₁-C₆- alkyl, N-(Di-C₁-C₈-alkylamino)-imino-C₁-C₆-alkyl oder Tri-C₁-C₄-alkylsilyl,
wobei die genannten Alkyl-, Cycloalkyl- und Alkoxyreste partiell oder voll- ständig halogeniert sein können und/oder eine bis drei der folgenden Grup- pen tragen können: Cyano, Hydroxy, C₃-C₆-Cycloalkyl, C₁-C₆-Alkoxy-C₁-C₄- alkyl, C₁-C₄-Alkoxy-C₁-C₄-alkoxy-C₁-C₄-alkyl, C₁-C₄-Alkoxy, C₁-C₄-Alkylthio, Di-(C₁-C₄-alkyl)-amino, C₁-C₄-Alkyl-C₁-C₄-alkoxycarbonyl-amino, C₁-C₄- Alkylcarbonyl, Hydroxycarbonyl, C₁-C₄-Alkoxycarbonyl, Aminocarbonyl, C₁- C₄-Alkylamino-carbonyl, Di-(C₁-C₄-alkyl)-aminocarbonyl oder C₁-C₄- Alkylcarbonyloxy;
Phenyl, Phenyl-C₁-C₆-alkyl, Phenylcarbonyl, Phenylcarbonyl-C₁-C₆-alkyl, Pheno- xycarbonyl, Phenylaminocarbonyl, Phenylsulfonylaminocarbonyl, N-(C₁-C₆- Alkyl)-N-(phenyl)-aminocarbonyl, Phenyl-C₁-C₆-alkylcarbonyl,
wobei der Phenylrest partiell oder vollständig halogeniert sein kann und/oder eine bis drei der folgenden Gruppen tragen kann: Nitro, Cyano, C₁-C₄-Alkyl, C₁-C₄-Halogenalkyl, C₁-C₄-Alkoxy oder C₁-C₄-Halogenalkoxy; oder
SO₂R⁷;
R⁵ und R⁶ zusammen mit dem Kohlenstoffatom, an das sie gebunden sind, einen 3- 12-gliedrigen, gesättigten oder partiell ungesättigten Ring, der carbocyclisch ist oder 1 bis 3 N-Atome, 0 bis 3 N-Atome und 1 O- oder S-Atom, 0 bis 2 N-Atome und 2 O- oder S-Atome, 0 oder 1 N-Atom und 1 O- und 1 S-Atom, 3 O- oder S- Atome, 2 O-Atome und 1 S-Atom, oder 1 O-Atom und 2 S-Atome enthält,
wobei der Ring unsubstituiert ist oder substituiert durch 1 bis 3, im Fall von Halogen auch bis zur maximal möglichen Anzahl, Substituenten aus der Gruppe Halogen, Cyano, Nitro, C₁-C₆-Alkyl, C₃-C₆-Cycloalkyl, C₃-C₆- Alkenyl, C₃-C₆-Alkinyl, C₃-C₆-Halogenalkenyl, C₃-C₆-Halogenalkinyl, Hydroxy, C₁-C₆-Alkoxy, C₃-C₆-Alkenyloxy, C₃-C₆-Alkinyloxy, Trialkylsilyloxy, Formyl, C₁-C₆-Alkyl-carbonyl, C₃-C₆-Cycloalkylcarbonyl, C₂-C₆- Alkenylcarbonyl, C₂-C₆-Alkinylcarbonyl, C₁-C₆-Alkoxycarbonyl, C₃-C₆- Alkenyloxycarbonyl, C₃-C₆-Alkinyloxycarbonyl, Aminocarbonyl, C₁-C₆- Alkylaminocarbonyl, C₃-C₆-Alkenylamino-carbonyl, C₃-C₆- Alkinylaminocarbonyl, Di-(C₁-C₆-alkyl)-aminocarbonyl, N-(C₃-C₆-Alkenyl)- N-(C₁-C₆-alkyl)-aminocarbonyl, N-(C₃-C₆-Alkinyl)-N-(C₁-C₆-alkyl)- aminocarbonyl, N-(C₁-C₆-Alkoxy)-N-(C₁-C₆-alkyl)-amino-carbonyl, N-(C₃- C₆-Alkenyl)-N-(C₁-C₆-alkoxy)-aminocarbonyl, N-(C₃-C₆-Alkinyl)-N-(C₁-C₆- alkoxy)-aminocarbonyl, Di-(C₁-C₆-alkyl)-aminothlocarbonyl, C₁-C₆- Alkoxyimino-C₁-C₆-alkyl, N-(C₁-C₆-Alkylamino)-imino-C₁-C₆-alkyl oder N- (Di-C₁-C₆-alkylamino)-imino-C₁-C₆-alkyl, Amino, Formylamino, C₁-C₆- Alkylcarbonylamino, C₁-C₆-Alkoxycarbonylamino, C₁-C₆-Alkylamino, Formyl-C₁-C₆-Alkylamino, C₁-C₆-Alkylcarbonyl-C₁-C₆-Alkylamino, C₁-C₆- Alkoxycarbonyl-C₁-C₆-Alkylamino, Di-(C₁-C₆-alkyl)amino, Amino- Carbonylamino, C₁-C₆-Alkylamino-Carbonylamino, Di(C₁-C₆-)alkylamino- Carbonylamino, C₁-C₆-Alkylthio, C₁-C₄-Alkylsulfonyl, Alkylsolfonyloxy, Al- kylsulfonylamino, C₁-C₆-Alkylsulfinyl, C₁-C₆-Alkylsulfimino, C₁-C₆-Alkyl- C₁-C₆-Alkylsulfimino, Carbonyl, Thiocarbonyl, Imino, Alkylimino, Hydroxy- imino, Alkoxyimino, Aminoimino, Alkylaminoimino, Di-(Alkyl)aminoimino, Alkylcarbonylaminoimino, Alkylsulfonylaminoimino, C₁-C₆-Vinylidenyl, C₁- C₆-Alkoxyvinyliden, Di-C₁-C₆-Alkylaminovinyliden,
wobei die genannten Alkyl-, Cycloalkyl- und Alkoxyreste partiell oder vollständig halogeniert sein können und/oder eine bis drei der folgen- den Gruppen tragen können: Cyano, Hydroxy, C₃-C₆-Cycloalkyl, C₁- C₄-Alkoxy, C₁-C₄-Alkylthio, Di-(C₁-C₄-alkyl)-amina, C₁-C₄- Alkylcarbonyl, Hydroxycarbonyl, C₁-C₄-Alkoxycarbonyl, Aminocarbo- nyl, C₁-C₄-Alkylaminocarbonyl, Di-(C₁-C₄-alkyl)-aminocarbonyl oder C₁-C₄-Alkylcarbonyloxy,
Phenyl, Phenyl-C₁-C₆-alkyl, Phenylcarbonyl, Phenylcarbonyl-C₁-C₆-alkyl, Phenoxycarbonyl, Phenylaminocarbonyl, Phenylsulfonylaminocarbonyl, N- (C₁-C₆-Alkyl)-N-(phenyl)-aminocarbonyl, Phenyl-C₁-C₆-alkylcarbonyl, Hete- rocyclyl, Heterocyclyl-C₁-C₆-alkyl, Heterocyclylcarbonyl, Heterocyclylsulfo- nylaminocarbonyl, Heterocyclylcarbonyl-C₁-C₆-alkyl, Heterocyclyloxycar- bonyl, Heterocyclylaminocarbonyl, N-(C₁-C₆-Alkyl)-N-(heterocyclyl)- aminocarbonyl oder Heterocyclyl-C₁-C₆-alkylcarbonyl,
wobei der Phenyl- und der Heterocyclyl-Rest der 17 letztgenannten Substituenten partiell oder vollständig halogeniert sein kann und/oder eine bis drei der folgenden Gruppen tragen kann: Nitro, Cyano, C₁- C₄-Alkyl, C₁-C₄-Halogenalkyl, C₁-C₄-Alkoxy oder C₁-C₄- Halogenalkoxy;
und wobei der Ring monocyclisch ist oder anelliert ist mit einem weiteren 3 bis 7- gliedrigen gesättigen, partiell ungesättigten oder vollständig ungesättigten Ring, der carbocyclisch ist oder 1 bis 3 N-Atome, 0 bis 2 N-Atome und 1 O-Atom oder S-Atom, 0 oder 1 N-Atom und 2 O-Atome oder S-Atome, 0 oder 1 N-Atom und 1 O-Atom und 1 S-Atom, 2 O-Atome und 1 S-Atom, oder 1 O-Atom und 2 S-Atome enthält,
wobei der anellierte Ring unsubstituiert ist oder substituiert ist durch 1 bis 3, im Fall von Halogen auch bis zur maximal möglichen Anzahl, Substi- tuenten aus der Gruppe Halogen, Cyano, Nitro, C₁-C₆-Alkyl, C₃-C₆-Alkenyl, C₁-C₆-Halogenalkyl, C₃-C₆-Halogenalkenyl, C₃-C₆-Halogenalkinyl, Hydroxy, C₁-C₆-Alkoxy, C₁-C₆-Halogenalkoxy und C₁-C₆-Alkylsulfonyl,
und wobei der Ring unverbrückt ist oder überbrückt mit einer 1 bis 4-zähligen gesättigten oder ungesättigten Kette, die keine Heteroatome enthält oder 1 bis 2 N-Atome, 0 oder 1 N-Atom und 1 O-Atom oder 1 S-Atom, 0 oder 1 N-Atom und 2 O-Atome oder 2 S-Atome, oder 0 oder 1 N-Atom und 1 O-Atom und 1 S-Atom enthält,
wobei die Brücke unsubstituiert ist oder substituiert mit 1 bis 3, im Fall von Halogen auch bis zur maximal möglichen Anzahl, Substituenten aus Grup- pe Halogen, Cyano, Nitro, C₁-C₆-Alkyl, C₃-C₆-Alkenyl, C₁-C₆-Halogenalkyl, C₃-C₆-Halogenalkenyl, C₃-C₆-Halogenalkinyl, Hydroxy, C₁-C₆-Alkoxy, C₁- C₆-Halogenalkoxy und C₁-C₆-Alkylsulfonyl"
R⁷ C₁-C₆-Alkyl, C₁-C₆-Halogenalkyl oder Phenyl,
wobei der Phenylrest partiell oder vollständig halogeniert sein kann und/oder eine bis drei der folgenden Gruppen tragen kann: C₁-C₆-Alkyl, C₁-C₆-Halogenalkyl oder C₁-C₆-Alkoxy
sowie deren landwirtschaftlich brauchbaren Salze.

2. Heteroaroylsubstituierte Serin-Amide der Formel (I) gemäß Anspruch 1, wobei A für 5- oder 6-gliedriges Heteroaryl ausgewählt aus der Gruppe Pyrrolyl, Thienyl, Furyl, Pyrazolyl, Imidazolyl, Thiazolyl, Oxazolyl, Tetrazolyl, Pyridyl und Pyrimidinyl steht und wobei die genannten Heteroarylreste partiell oder vollständig halogeniert sein können und/oder 1 bis 3 Reste aus der Gruppe Cyano, C₁-C₆-Alkyl, C₃-C₆-Cycloalkyl, C₁-C₆-Halogenalkyl, C₁-C₆-Alkoxy, C₁-C₆-Halogenalkoxy und C₁-C₆-Alkoxy-C₁-C₄-alkyl tragen können.

3. Heteroaroylsubstituierte Serin-Amide der Formel (I) gemäß Anspruch 1 oder 2, wobei R¹, R² und R⁵ für Wasserstoff stehen.

4. Heteroaroylsubstituierte Serin-Amide der Formel (I) gemäß einem der Ansprüche 1 bis 3, wobei
R⁵ und R⁶ zusammen mit dem Kohlenstoffatom, an das sie gebunden sind, einen 3 bis 7-gliedrigen, gesättigten oder partiell ungesättigten Ring, bedeuten, der carbocyclisch ist oder 1 oder 2 N-Atome, 0 oder 1 N-Atom und 1 O-Atom oder S-Atom, 0 oder 1 N-Atom und 1 O- und 1 S-Atom oder 2 O- oder S-Atome enthält,
wobei der Ring unsubstituiert ist oder wie in der Formel (I) angegeben substituiert
und wobei der Ring monocyclisch ist oder anelliert mit einem weiteren 3 bis 6-gliedrigen gesättigten, oder partiell ungesättigten Ring, der carbocyclisch ist oder 1 oder 2 N-Atome, 0 oder 1 N-Atom oder 1 O-Atom oder S-Atom, 2 O-Atome oder S-Atome, 0 oder 1 N-Atom und 1 O-Atom und 1 S-Atome enthält,
wobei der anellierte Ring unsubstituiert ist oder substituiert ist durch 1 bis 3, im Fall von Halogen auch bis zur maximal möglichen Anzahl, Substituenten aus der Gruppe Halogen, C₁-C₆-Alkyl, C₁-C₆-Halogenalkyl und C₁-C₆-Alkoxy,
und wobei der Ring nicht verbrückt ist oder überbrückt mit einer 1 bis 3-zähligen gesättigten oder ungesättigten Kette, die keine. Heteroatome enthält, oder 1 N-Atom, oder 0 oder 1 N-Atom und 1 O-Atom oder 1 S-Atom enthält,
wobei die Brücke unsubstituiert ist oder substituiert mit 1 bis 3, im Fall von Halogen auch bis zur maximal möglichen Anzahl, Substituenten aus der Gruppe Halogen, C₁-C₆-Alkyl, Hydroxy und C₁-C₆-Alkoxy.

5. Verfahren zur Herstellung von heteroaroylsubstituierten Serin-Amide der Formel (I) gemäß einem der Ansprüche 1 bis 4, wobei Serinderivate der Formel (V), 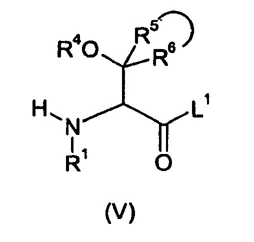 wobei R¹, R⁴, R⁵ und R⁶ die in Formel (I) genannten Bedeutungen haben und L¹ für Hydroxy oder C₁-C₆-Alkoxy steht,
mit Heteroarylsäure(derivate)n der Formel (IV), 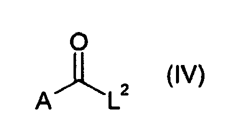 wobei A die in Formel (I) genannte Bedeutung hat und L² für Hydroxy, Halogen, C₁-C₆-Alkylcarbonyl, C₁-C₆-Alkoxycarbonyl, C₁-C₄-Alkylsulfonyl, Phosphoryl oder Isoureyl steht,
zu entsprechenden Heteroaroylderivaten der Formel (III), 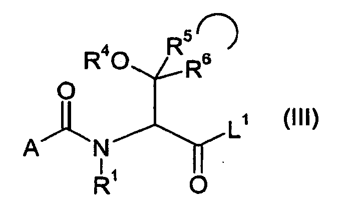 wobei A, R¹, R⁴, R⁵ und R⁶ die in Formel (I) genannten Bedeutungen haben und L¹ für Hydroxy oder C₁-C₆-Alkoxy steht,
und anschließend die erhaltenen Heteroaroylderivate der Formel (III) mit einem
Amin der Formel (II)
HNR²R³ (II)
wobei R² und R³ die in Formel (I) genannten Bedeutungen haben, umgesetzt werden.

6. Heteroaroylderivate der Formel (III), 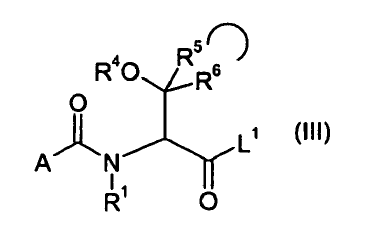 wobei A, R¹, R⁴, R⁵ und R⁶ die in Formel (I) in Anspruch 1 genannten Bedeutungen haben und L¹ für eine nucleophil verdrängbare Abgangsgruppe steht.

7. Mittel, enthaltend eine herbizid wirksame Menge mindestens eines heteroaroyl-substituierten Serin-Amids der Formel (I) oder eines landwirtschaftlich brauchbaren Salzes von (I) gemäß einem der Ansprüche 1 bis 4 und für die Formulierung von Pflanzenschutzmitteln übliche Hilfsmittel.

8. Verfahren zur Herstellung von Mitteln gemäß Anspruch 7, **dadurch gekennzeichnet, dass** man eine herbizid wirksame Menge mindestens eines heteroaroyl-substituierten Serin-Amids der Formel (I) oder eines landwirtschaftlich brauchbaren Salzes von (I) gemäß einem der Ansprüche 1 bis 4 und für die Formulierung von Pflanzenschutzmitteln übliche Hilfsmittel mischt.

9. Verfahren zur Bekämpfung von unerwünschtem Pflanzenwuchs, **dadurch gekennzeichnet, dass** man eine herbizid wirksame Menge mindestens eines heteroaroylsubstituierten Serin-Amids der Formel (I) oder eines landwirtschaftlich brauchbaren Salzes von (I) gemäß einem der Ansprüche 1 bis 4 auf Pflanzen, deren Lebensraum und/oder auf Samen einwirken lässt.

10. Verwendung der heteroaroylsubstituierten Serin-Amide der Formel (I) und deren landwirtschaftlich brauchbaren Salze gemäß einem der Ansprüche 1 bis 4 als Herbizide.

## Claims

1. A heteroaroyl-substituted serineamide of the formula (I) 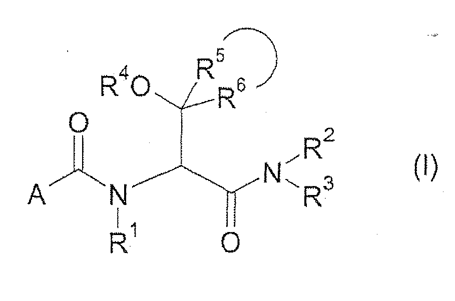 in which the variables are as defined below:
A is 5- or 6-membered heteroaryl having one to four nitrogen atoms or one to three nitrogen atoms and one oxygen or sulfur atom or one oxygen or sulfur atom, which heteroaryl may be partially or fully halogenated and/or may carry 1 to 3 radicals from the group consisting of cyano, C₁-C₆-alkyl, C₃-C₆-cycloalkyl, C₁-C₆- haloalkyl, C₁-C₆-alkoxy, C₁-C₆-haloalkoxy and C₁- C₆-alkoxy-C₁-C₄-alkyl;
R¹, R² are hydrogen, hydroxyl or C₁-C₆-alkoxy;
R³ is C₁-C₆-alkyl, C₁-C₄-cyanoalkyl or C₁-C₆- haloalkyl;
R⁴ is hydrogen, C₁-C₆-alkyl, C₃-C₆-cycloalkyl, C₃-C₆- alkenyl, C₃-C₆-alkynyl, C₃-C₆-haloalkenyl, C₃-C₆- haloalkynyl, formyl, C₁-C₆-alkylcarbonyl, C₃-C₆-cycloalkylcarbonyl, C₂-C₆-alkenylcarbonyl, C₂-C₆-alkynylcarbonyl, C₁-C₆-alkoxycarbonyl, C₃- C₆-alkenyloxycarbonyl, C₃-C₆-alkynyloxycarbonyl, aminocarbonyl, C₁-C₆-alkylaminocarbonyl, C₃-C₆- alkenylaminocarbonyl, C₃-C₆-alkynylamino- carbonyl, C₁-C₆-alkylsulfonylaminocarbonyl, di-(C₁-C₆-alkyl)aminocarbonyl, N-(C₃-C₆- alkenyl)-N-(C₁-C₆-alkyl)aminocarbonyl, N-(C₃-C₆- alkynyl)-N-(C₁-C₆-alkyl)aminocarbonyl, N-(C₁-C₆- alkoxy)-N-(C₁-C₆-alkyl)aminocarbonyl, N-(C₃-C₆- alkenyl)-N-(C₁-C₆-alkoxy)aminocarbonyl, N-(C₃-C₆-alkynyl)-N-(C₁-C₆-alkoxy)aminocarbonyl, (C₁-C₆) alkylaminothiocarbonyl, di-(C₁-C₆- alkyl)aminothiocarbonyl, (C₁-C₆-alkyl)- cyanoimino, (amino)cyanoimino, [(C₁-C₆- alkyl)amino]cyanoimino, [di(C₁-C₆-alkyl)- amino]cyanoimino, C₁-C₆-alkylcarbonyl-C₁-C₆- alkyl, C₁-C₆-alkoxyimino-C₁-C₆-alkyl, N-(C₁-C₆- alkylamino)imino-C₁-C₆-alkyl, N-(di-C₁-C₆- alkylamino)imino-C₁-C₆-alkyl or tri-C₁-C₄- alkylsilyl,
where the alkyl, cycloalkyl and alkoxy radicals mentioned may be partially or fully halogenated and/or may carry one to three of the following groups: cyano, hydroxyl, C₃-C₆-cycloalkyl, C₁-C₆-alkoxy- C₁-C₄-alkyl, C₁-C₄-alkoxy-C₁-C₄-alkoxy-C₁-C₄- alkyl, C₁-C₄-alkoxy, C₁-C₄-alkylthio, di-(C₁- C₄-alkyl)amino, C₁-C₄-alkyl-C₁-C₄-alkoxy- carbonylamino, C₁-C₄-alkylcarbonyl, hydroxycarbonyl, C₁-C₄-alkoxycarbonyl, aminocarbonyl, C₁-C₄-alkylaminocarbonyl, di- (C₁-C₄-alkyl)aminocarbonyl or C₁-C₄- alkylcarbonyloxy;
phenyl, phenyl-C₁-C₆-alkyl, phenylcarbonyl, phenylcarbonyl-C₁-C₆-alkyl, phenoxycarbonyl, phenylaminocarbonyl,
phenylsulfonylaminocarbonyl, N-(C₁-C₆-alkyl)-N- (phenyl)aminocarbonyl, phenyl-C₁-C₆- alkylcarbonyl,
where the phenyl radical may be partially or fully halogenated and/or may carry one to three of the following groups: nitro, cyano, C₁-C₄-alkyl, C₁-C₄-haloalkyl, C₁-C₄- alkoxy or C₁-C₄-haloalkoxy; or
SO₂R⁷ ;
R⁵ and R⁶ together with the carbon atom to which they are attached are a 3-12-membered saturated or partially unsaturated ring which is carbocyclic or contains 1 to 3 nitrogen atoms, 0 to 3 nitrogen atoms and 1 oxygen or sulfur atom, 0 to 2 nitrogen atoms and 2 oxygen or sulfur atoms, 0 or 1 nitrogen atom and 1 oxygen and 1 sulfur atom, 3 oxygen or sulfur atoms, 2 oxygen atoms and 1 sulfur atom, or 1 oxygen atom and 2 sulfur atoms,
where the ring is unsubstituted or substituted by 1 to 3, in the case of halogen also up to the maximum possible number, substituents from the group consisting of halogen, cyano, nitro, C₁-C₆-alkyl, C₃-C₆-cycloalkyl, C₃-C₆-alkenyl, C₃-C₆-alkynyl, C₃-C₆-haloalkenyl, C₃-C₆- haloalkynyl, hydroxyl, C₁-C₆-alkoxy, C₃-C₆- alkenyloxy, C₃-C₆-alkynyloxy, trialkylsilyloxy, formyl , C₁-C₆-alkyl-carbonyl, C₃-C₆- cycloalkylcarbonyl, C₂-C₆-alkenylcarbonyl, C₂-C₆- alkynylcarbonyl, C₁-C₆-alkoxycarbonyl, C₃-C₆- alkenyloxycarbonyl, C₃-C₆-alkynyloxycarbonyl, aminocarbonyl, C₁-C₆-alkylaminocarbonyl, C₃-C₆- alkenylamino-carbonyl, C₃-C₆- alkynylaminocarbonyl, di-(C₁-C₆-alkyl)- aminocarbonyl, N-(C₃-C₆-alkenyl)-N-(C₁-C₆-alkyl)- aminocarbonyl, N-(C₃-C₆-alkynyl)-N-(C₁-C₆-alkyl)- aminocarbonyl, N-(C₁-C₆-alkoxy)-N-(C₁-C₆-alkyl)- amino-carbonyl, N-(C₃-C₆-alkenyl)-N-(C₁-C₆- alkoxy)-aminocarbonyl, N-(C₃-C₆-alkynyl)- N-(C₁-C₆-alkoxy) -aminocarbonyl, di-(C₁-C₆-alkyl)- aminothiocarbonyl, C₁-C₆-alkoxyimino-C₁-C₅-alkyl, N-(C₁-C₆-alkylamino)-imino-C₁-C₆-alkyl or N-(di- C₁-C₆-alkylamino)-imino-C₁-C₆-alkyl, amino, formylamino, C₁-C₆-alkylcarbonylamino, C₁-C₆- alkoxycarbonylamino, C₁-C₆-alkylamino, formyl- C₁-C₆-alkylamino, C₁-C₆-alkylcarbonyl-C₁-C₆- alkylamino, C₁-C₆-alkoxycarbonyl-C₁-C₆- alkylamino, di-(C₁-C₆-alkyl)amino, amino- carbonylamino, C₁-C₆-alkylamino-carbonylamino, di (C₁-C₆-)alkylamino-carbonylamino, C₁-C₆- alkylthio, C₁-C₆alkylsulfonyl, alkylsulfonyloxy, alkylsulfonylamino, C₁-C₆-alkylsulfinyl, C₁-C₆- alkylsulfimino, C₁-C₆-alkyl-C₁-C₆-alkylsulfimino, carbonyl, thiocarbonyl, imino, alkylimino, hydroxyimino, alkoxyimino, aminoimino, alkylaminoimino, di-(alkyl)aminoimino, alkylcarbonylaminoimino, alkylsulfonylaminoimino, C₁-C₆-vinylidenyl, C₁-C₆-alkoxyvinylidene, di-C₁-C₆- alkylaminovinylidene,
where the alkyl, cycloalkyl and alkoxy radicals mentioned may be partially or fully halogenated and/or may carry one to three of the following groups: cyano, hydroxyl, C₃-C₆-cycloalkyl, C₁-C₄-alkoxy, C₁-C₄-alkylthio, di- (C₁-C₄-alkyl) -amino, C₁-C₄-alkylcarbonyl, hydroxycarbonyl, C₁-C₄- alkoxycarbonyl, aminocarbonyl, C₁-C₄- alkylaminocarbonyl, di-(C₁-C₄-alkyl)- aminocarbonyl or C₁-C₄-alkylcarbonyloxy,
phenyl, phenyl-C₁-C₆-alkyl, phenylcarbonyl, phenylcarbonyl-C₁-C₆-alkyl, phenoxycarbonyl, phenylaminocarbonyl, phenylsulfonylaminocarbonyl, N-(C₁-C₆-alkyl)- N-(phenyl)-aminocarbonyl, phenyl-C₁-C₆- alkylcarbonyl, heterocyclyl, heterocyclyl-C₁-C₆- alkyl, heterocyclylcarbonyl, heterocyclyl- sulfonylaminocarbonyl; heterocyclylcarbonyl- C₁-C₆-alkyl, heterocyclyloxycarbonyl, heterocyclylaminocarbonyl, N-(C₁-C₆-alkyl)- N-(heterocyclyl)-aminocarbonyl or heterocyclyl- C₁-C₆-alkylcarbonyl,
where the phenyl and the heterocyclyl radical of the 17 last-mentioned substituents may be partially or fully halogenated and/or may carry one to three of the following groups: nitro, cyano, C₁-C₄-alkyl, C₁-C₄-haloalkyl, C₁-C₄-alkoxy or C₁-C₄-haloalkoxy;
and where the ring is monocyclic or fused to a further 3- to 7-membered saturated, partially unsaturated or fully unsaturated ring which is carbocyclic or contains 1 to 3 nitrogen atoms, 0 to 2 nitrogen atoms and 1 oxygen atom or sulfur atom, 0 or 1 nitrogen atom and 2 oxygen atoms or sulfur atoms, 0 or 1 nitrogen atom and 1 oxygen atom and 1 sulfur atom, 2 oxygen atoms and 1 sulfur atom, or 1 oxygen atom and 2 sulfur atoms,
where the fused ring is unsubstituted or substituted by 1 to 3, in the case of halogen also up to the maximum possible number, substituents from the group consisting of halogen, cyano, nitro, C₁-C₆-alkyl, C₃-C₆- alkenyl, C₁-C₆-haloalkyl, C₃-C₆-haloalkenyl, C₃-C₆-haloalkynyl, hydroxyl, C₁-C₆-alkoxy, C₁-C₆- haloalkoxy and C₁-C₆-alkylsulfonyl,
and where the ring is not bridged or bridged by a 1- to 4-membered saturated or unsaturated chain which contains no heteroatoms or contains 1 to 2 nitrogen atoms, 0 or 1 nitrogen atom and 1 oxygen atom or 1 sulfur atom, 0 or 1 nitrogen atom and 2 oxygen atoms or 2 sulfur atoms, or 0 or 1 nitrogen atom and 1 oxygen atom and 1 sulfur atom,
where the bridge is unsubstituted or substituted by 1 to 3, in the case of halogen also up to the maximum possible number, substituents from the group consisting of halogen, cyano, nitro, C₁-C₆-alkyl, C₃-C₆-alkenyl, C₁-C₆-haloalkyl, C₃-C₆-haloalkenyl, C₃-C₆-haloalkynyl, hydroxyl, C₁-C₆-alkoxy, C₁-C₆-haloalkoxy and C₁-C₆-alkylsulfonyl ;
R⁷ C₁-C₆-alkoxy, C₁-C₆-haloalkoxy and C₁-C₆- alkylsulfonyl; is C₁-C₆-alkyl, C₁-C₆-haloalkyl or phenyl,
where the phenyl radical may be partially or fully halogenated and/or may carry one to three of the following groups: C₁-C₆-alkyl, C₁-C₆-haloalkyl or C₁- C₆-alkoxy
or an agriculturally useful salt thereof.

2. The heteroaroyl-substituted serineamide of the formula (I) according to claim 1 where A is 5- or 6-membered heteroaryl selected from the group consisting of pyrrolyl, thienyl, furyl, pyrazolyl, imidazolyl, thiazolyl, oxazolyl, tetrazolyl, pyridyl and pyrimidinyl and where the heteroaryl radicals mentioned may be partially or fully halogenated and/or may carry 1 to 3 radicals from the group consisting of cyano, C₁-C₆-alkyl, C₃-C₆-cycloalkyl, C₁-C₆-haloalkyl, C₁-C₆-alkoxy, C₁-C₆-haloalkoxy and C₁-C₆-alkoxy-C₁-C₄-alkyl.

3. The heteroaroyl-substituted serineamide of the formula (I) according to claim 1 or 2 where R¹, R² and R⁵ are hydrogen.

4. The heteroaroyl-substituted serineamide of the formula (I) according to any of claims 1 to 3, where
R⁵ and R⁶ together with the carbon atom to which they are attached are a 3- to 7-membered saturated or partially unsaturated ring which is carbocylic or contains 1 or 2 nitrogen atoms, 0 or 1 nitrogen atom and 1 oxygen atom or sulfur atom, 0 or 1 nitrogen atom and 1 oxygen and 1 sulfur atom or 2 oxygen or sulfur atoms,
where the ring is unsubstituted or substituted as indicated in formula (I),
and where the ring is monocyclic or fused to a further 3- to 6-membered saturated or partially unsaturated ring, which is carbocyclic or contains 1 or 2 nitrogen atoms, 0 or 1 nitrogen atom or 1 oxygen atom or sulfur atom, 2 oxygen atoms or sulfur atoms, 0 or 1 nitrogen atom and 1 oxygen atom and 1 sulfur atom,
where the fused ring is unsubstituted or substituted by 1 to 3, in the case of halogen also up to the maximum possible number, substituents from the group consisting of halogen, C₁-C₆-alkyl, C₁-C₆-haloalkyl and C₁-C₆-alkoxy,
and where the ring is not bridged or bridged by a 1- to 3-membered saturated or unsaturated chain which contains no heteroatoms, or contains 1 nitrogen atom, or 0 or 1 nitrogen atom and 1 oxygen atom or 1 sulfur atom,
where the bridge is unsubstituted or substituted by 1 to 3, in the case of halogen also up to the maximum possible number, substituents from the group consisting of halogen, C₁-C₆-alkyl, hydroxy and C₁-C₆-alkoxy.

5. A process for preparing heteroaroyl-substituted serineamides of the formula (I) according to any of claims 1 to 4, wherein serine derivatives of the formula (V) 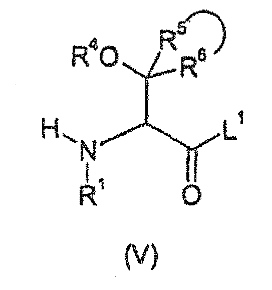 where R¹, R⁴, R⁵ and R⁶ are as defined in formula (I) and L¹ is hydroxyl or C₁-C₆-alkoxy,
are reacted with heteroaryl acid (derivatives) of the formula (IV) 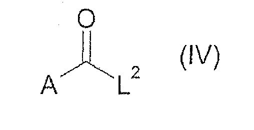 where A is as defined in formula (I) and L² is hydroxyl, halogen, C₁-C₆-alkylcarbonyl, C₁-C₆-alkoxycarbonyl, C₁-C₄-alkylsulfonyl, phosphoryl or isoureyl,
to give the corresponding heteroaroyl derivatives of the formula (III) 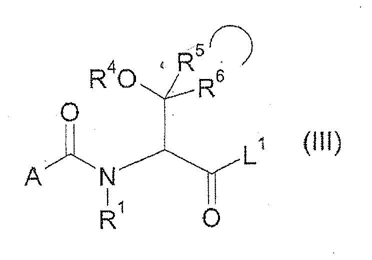 where A, R¹, R⁴, R⁵ and R⁶ are as defined in formula (I) and L¹ is hydroxyl or C₁-C₆-alkoxy,
and the resulting heteroaroyl derivatives of the formula (III) are then reacted with an amine of the formula (II)
HNR²R³ (II)
where R² and R³ are as defined in formula (I).

6. A heteroaroyl derivative of the formula (III) 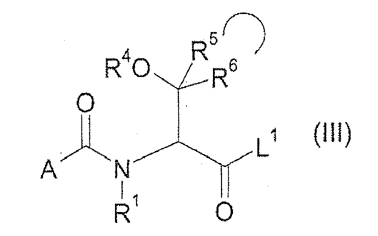 where A, R¹, R⁴, R⁵ and R⁶ are as defined in formula (I) and L¹ is a nucleophilically displaceable leaving group.

7. A composition, comprising a herbicidally effective amount of at least one heteroaroyl-substituted serineamide of the formula (I) or an agriculturally useful salt of (I) according to any of claims 1 to 4 and auxiliaries customary for formulating crop protection agents.

8. A process for preparing compositions according to claim 7, wherein a herbicidally effective amount of at least one heteroaroyl-substituted serineamide of the formula (I) or an agriculturally useful salt of (I) according to any of claims 1 to 4 and auxiliaries customary for formulating crop protection agents are mixed.

9. A method for controlling unwanted vegetation, wherein a herbicidally effective amount of at least one heteroaroyl-substituted serineamide of the formula (I) or an agriculturally useful salt of (I) according to any of claims 1 to 4 is allowed to act on plants, their habitat and/or on seed.

10. The use of a heteroaroyl-substituted serineamide of the formula (I) or an agriculturally useful salt thereof according to any of claims 1 to 4 as a herbicide.

## Revendications

1. Amides de sérine substitués par hétéroaroyle de formule (I), 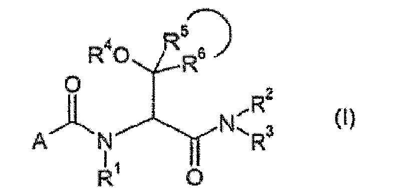 dans laquelle les variables ont les significations suivantes :
A hétéroaryle de 5 ou 6 chaînons présentant un à quatre atomes d'azote ou présentant un à trois atomes d'azote et un atome d'oxygène ou de soufre ou présentant un atome d'oxygène ou de soufre qui peut être halogéné partiellement ou complètement et/ou qui peut porter 1 à 3 radicaux du groupe cyano, C₁-C₆-alkyle, C₃-C₆-cycloalkyle, C₁-C₆- halogénoalkyle, C₁-C₆-alcoxy, C₁-C₆-halogénoalcoxy et C₁-C₆-alcoxy-C₁-C₄-alkyle ;
R¹, R² hydrogène, hydroxy ou C₁-C₆-alcoxy ;
R³ C₁-C₆-alkyle, C₁-C₄-cyanoalkyle ou C₁-C₆- halogénoalkyle ;
R⁴ hydrogène, C₁-C₆-alkyle, C₃-C₆-cycloalkyle, C₃-C₆- alcényle, C₃-C₆-alcynyle, C₃-C₆-halogénoalcényle, C₃-C₆-halogénoalcynyle, formyle, C₁-C₆- alkylcarbonyle, C₃-C₆-cycloalkylcarbonyle, C₂-C₆- alcénylcarbonyle, C₂-C₆-alcynylcarbonyle, C₁-C₆- alcoxycarbonyle, C₃-C₆-alcényloxycarbonyle, C₃-C₆- alcynyloxycarbonyle, aminocarbonyle, C₁-C₆- alkylaminocarbonyle, C₃-C₆-alcénylaminocarbonyle, C₃-C₆-alcynylaminocarbonyle, C₁-C₆- alkylsulfonylaminocarbonyle, di-(C₁-C₆-alkyl)- aminocarbonyle, N-(C₃-C₆-alcényl)-N-(C₁-C₆-alkyl)- aminocarbonyle, N-(C₃-C₆-alcynyl)-N-(C₁-C₆-alkyl)- aminocarbonyle, N-(C₁-C₆-alcoxy)-N-(C₁-C₆-alkyl)- aminocarbonyle, N-(C₃-C₆-alcényl)-N-(C₁-C₆-alcoxy)- aminocarbonyle, N-(C₃-C₆-alcynyl)-N-(C₁-C₆-alcoxy)- aminocarbonyle, (C₁-C₆)alkylaminothiocarbonyle, di- (C₁-C₆-alkyl) -aminothiocarbonyle, (C₁-C₆- alkyl)cyano-imino, (amino)cyano-imino, [(C₁-C₆- alkyl)amino]cyano-imino, [di(C₁-C₆- alkyl)amino]cyano-imino, C₁-C₆-alkylcarbonyl-C₁-C₆- alkyle, C₁-C₆-alcoxyimino-C₁-C₆-alkyle, N-(C₁-C₆)- alkylamino)-imino-C₁-C₆-alkyle, N-(di-C₁-C₆- alkylamino)-imino-C₁-C₆-alkyle ou tri-C₁-C₄- alkylsilyle,
où les radicaux alkyle, cycloalkyle et alcoxy mentionnés peuvent être partiellement ou totalement halogénés et/ou peuvent porter un à trois des groupes suivants : cyano, hydroxy, C₃-C₆-cycloalkyle, C₁-C₆-alcoxy-C₁-C₄- alkyle, C₁-C₄-alcoxy-C₁-C₄-alcoxy-C₁-C₄-alkyle, C₁-C₄-alcoxy, C₁-C₄-alkylthio, di-(C₁-C₄- alkyl)-amino, C₁-C₄-alkyl-C₁-C₄- alcoxycarbonylamino, C₁-C₄-alkylcarbonyle, hydroxycarbonyle, C₁-C₄-alcoxycarbonyle, aminocarbonyle, C₁-C₄-alkylaminocarbonyle, di- (C₁-C₄-alkyl)-aminocarbonyle ou C₁-C₄- alkylcarbonyloxy ;
phényle, phényl-C₁-C₆-alkyle, phénylcarbonyle, phénylcarbonyl-C₁-C₆-alkyle, phénoxycarbonyle, phénylaminocarbonyle, phénylsulfonylaminocarbonyle, N-(C₁-C₆-alkyl)-N- (phényl)-aminocarbonyle, phényl-C₁-C₆- alkylcarbonyle, où le radical phényle peut être partiellement ou complètement halogéné et/ou peut porter un à trois des groupes suivants : nitro, cyano, C₁-C₄-alkyle, C₁-C₄-halogénoalkyle, C₁-C₄- alcoxy ou C₁-C₄-halogénoalcoxy ; ou
SO₂R⁷ ;
R⁵ et R⁶ ensemble avec l'atome de carbone auquel ils sont liés, un cycle de 3 à 12 chaînons, saturé ou partiellement insaturé, qui est carbocyclique ou qui contient 1 à 3 atomes de N, 0 à 3 atomes de N et 1 atome d'O ou de S, 0 à 2 atomes de N et 2 atomes d'O ou de S, 0 ou 1 atome de N et 1 atome d'O et 1 atome de S, 3 atomes d'O ou de S, 2 atomes d'O et 1 atome de S, ou 1 atome d'O et 2 atomes de S,
où le cycle est non substitué ou substitué par 1 à 3, dans le cas d'halogène également jusqu'au nombre maximal possible, substituants du groupe formé par halogène, cyano, nitro, C₁-C₆-alkyle, C₃-C₆-cycloalkyle, C₃-C₆-alcényle, C₃-C₆-alcynyle, C₃-C₆- halogénoalcényle, C₃-C₆-halogénoalcynyle, hydroxy, C₁-C₆-alcoxy, C₃-C₆-alcényloxy, C₃-C₆- alcynyloxy, trialkylsilyloxy, formyle, C₁-C₆- alkylcarbonyle, C₃-C₆-cycloalkylcarbonyle, C₂- C₆-alcénylcarbonyle, C₂-C₆-alcynylcarbonyle, C₁-C₆-alcoxycarbonyle, C₃-C₆- alcényloxycarbonyle, C₃-C₆- alcynyloxycarbonyle, aminocarbonyle, C₁-C₆- alkylaminocarbonyle, C₃-C₆- alcénylaminocarbonyle, C₃-C₆- alcynylaminocarbonyle, di-(C₁-C₆-alkyl)- aminocarbonyle, N-(C₃-C₆-alcényl)-N-(C₁-C₆- alkyl)-aminocarbonyle, N-(C₃-C₆-alcynyl)-N- (C₁-C₆-alkyl)-aminocarbonyle, N-(C₁-C₆- alcoxy)-N-(C₁-C₆-alkyl)-aminocarbonyle, N- (C₃- C₆-alcényl)-N-(C₁-C₆-alcoxy)-aminocarbonyle, N-(C₃-C₆-alcynyl)-N-(C₁-C₆-alcoxy)- aminocarbonyle, di-(C₁-C₆-alkyl)- aminothiocarbonyle, C₁-C₆-alcoxyimino-C₁-C₆- alkyle, N-(C₁-C₆-alkylamino)-imino-C₁-C₆- alkyle ou N-(di-C₁-C₆-alkylamino)-imino-C₁-C₆- alkyle, amino, formylamino, C₁-C₆- alkylcarbonylamino, C₁-C₆-alcoxycarbonylamino, C₁-C₆-alkylamino, formyl-C₁-C₆-alkylamino, C₁- C₆-alkylcarbonyl-C₁-C₆-alkylamino, C₁-C₆- alcoxycarbonyl-C₁-C₆-alkylamino, di- (C₁-C₅- alkyl) amino, amino-carbonylamino, C₁-C₆- alkylamino-carbonylamino, di(C₁-C₆- alkyl)amino-carbonylamino, C₁-C₆-alkylthio, C₁-C₆-alkylsulfonyle, alkylsulfonyloxy, alkylsulfonylamino, C₁-C₆-alkylsulfinyle, C₁- C₆-alkylsulfimino, C₁-C₆-alkyl-C₁-C₆- alkylsulfimino, carbonyle, thiocarbonyle, imino, alkylimino, hydroxyimino, alcoxyimino, amino-imino, alkylamino-imino, di- (alkyl)amino-imino, alkylcarbonylamino-imino, alkylsulfonylamino-imino, C₁-C₆-vinylidényle, C₁-C₆-alcoxyvinylidène, di-C₁-C₆- alkylaminovinylidène,
où les radicaux alkyle, cycloalkyle et alcoxy mentionnés peuvent être partiellement ou totalement halogénés et/ou peuvent porter un à trois des groupes suivants : cyano, hydroxy, C₃-C₆- cycloalkyle, C₁-C₄-alcoxy, C₁-C₄-alkylthio, di-(C₁-C₄-alkyl)-amino, C₁-C₄- alkylcarbonyle, hydroxycarbonyle, C₁-C₄- alcoxycarbonyle, aminocarbonyle, C₁-C₄- alkylaminocarbonyle, di-(C₁-C₄-alkyl)- aminocarbonyle ou C₁-C₄-alkylcarbonyloxy ; phényle, phényl-C₁-C₆-alkyle, phénylcarbonyle, phénylcarbonyl-C₁-C₆-alkyle, phénoxycarbonyle, phénylaminocarbonyle, phénylsulfonylaminocarbonyle, N-(C₁-C₆-alkyl)- N-(phényl)-aminocarbonyle, phényl-C₁-C₆- alkylcarbonyle, hétérocyclyle, hétérocyclyl- C₁-C₆-alkyle, hétérocyclylcarbonyle, hétérocyclylsulfonylaminocarbonyle, hétérocyclylcarbonyl-C₁-C₆-alkyle, hétérocyclyloxycarbonyle, hétérocyclylaminocarbonyle, N-(C₁-C₆-alkyl)-N- (hétérocyclyl)-aminocarbonyle ou hétérocyclyl-C₁-C₆-alkylcarbonyle,
où le radical phényle et hétérocyclyle des 17 derniers substituants mentionnés peut être partiellement ou complètement halogéné et/ou peut porter un à trois des groupes suivants : nitro, cyano, C₁-C₄- alkyle, C₁-C₄-halogénoalkyle, C₁-C₄-alcoxy ou C₁-C₄-halogénoalcoxy ;
et où le cycle est monocyclique ou annelé avec un autre cycle de 3 à 7 chaînons, saturé, partiellement insaturé ou totalement insaturé, qui est carbocyclique ou qui contient 1 à 3 atomes de N, 0 à 2 atomes de N et 1 atome d'O ou atome de S, 0 ou 1 atome de N et 2 atomes d'O ou atomes de S, 0 ou 1 atome de N et 1 atome d'O et 1 atome de S, 2 atomes d'O et 1 atome de S, ou 1 atome d'O et 2 atomes de S,
où le cycle annelé est non substitué ou substitué par 1 à 3, dans le cas d'halogène également jusqu'au nombre maximal possible, substituants du groupe formé par halogène, cyano, nitro, C₁-C₆-alkyle, C₃-C₆-alcényle, C₁- C₆-halogénoalkyle, C₃-C₆-halogénoalcényle, C₃- C₆-halogénoalcynyle, hydroxy, C₁-C₆-alcoxy, C₁-C₆-halogénoalcoxy et C₁-C₆-alkylsulfonyle,
et où le cycle est non ponté ou ponté avec une chaîne saturée ou insaturée de 1 à 4 chaînons, qui ne contient pas d'hétéroatomes ou qui contient 1 à 2 atomes de N, 0 à 1 atome de N et 1 atome d'O ou atome de S, 0 ou 1 atome de N et 2 atomes d'O ou atomes de S, ou 0 ou 1 atome de N et 1 atome d'O et 1 atome de S,
où le pont est non substitué ou substitué par 1 à 3, dans le cas d'halogène également jusqu'au nombre maximal possible, substituants du groupe formé par halogène, cyano, nitro, C₁-C₆-alkyle, C₃-C₆-alcényle, C₁- C₆-halogénoalkyle, C₃-C₆-halogénoalcényle, C₃- C₆-halogénoalcynyle, hydroxy, C₁-C₆-alcoxy, C₁-C₆-halogénoalcoxy et C₁-C₆-alkylsulfonyle ;
R⁷ représente C₁-C₆-alkyle, C₁-C₆-halogénoalkyle ou phényle,
où le radical phényle peut être partiellement ou complètement halogéné et/ou peut porter un à trois des groupes suivants : C₁-C₆-alkyle, C₁-C₆-halogénoalkyle ou C₁-C₆-alcoxy ;
ainsi que leurs sels utilisables en agriculture.

2. Amides de sérine substitués par hétéroaroyle de formule (I) selon la revendication 1, où A représente un hétéroaryle de 5 ou 6 chaînons choisi parmi le groupe pyrrolyle, thiényle, furyle, pyrazolyle, imidazolyle, thiazolyle, oxazolyle, tétrazolyle, pyridyle et pyrimidinyle et où les radicaux hétéroaryle mentionnés peuvent être partiellement ou complètement halogénés et/ou peuvent porter 1 à 3 radicaux du groupe formé par cyano, C₁-C₆-alkyle, C₃-C₆-cycloalkyle, C₁-C₆-halogénoalkyle, C₁-C₆-alcoxy, C₁-C₆-halogénoalcoxy et C₁-C₆-alcoxy-C₁-C₄-alkyle .

3. Amides de sérine substitués par hétéroaroyle de formule (I) selon la revendication 1 ou 2, où R¹, R² et R⁵ représentent hydrogène.

4. Amides de sérine substitués par hétéroaroyle de formule (I) selon l'une quelconque des revendications 1 à 3, où
R⁵ et R⁶ signifient, ensemble avec l'atome de carbone auquel ils sont liés, un cycle de 3 à 7 chaînons, saturé ou partiellement insaturé, qui est carbocyclique ou qui contient 1 ou 2 atomes de N, 0 ou 1 atome de N et 1 atome d'O ou de S, 0 à 1 atome de N et 1 atome d'O et de S, ou 2 atomes d'O ou de S,
où le cycle est non substitué ou substitué comme indiqué dans la formule (I) et où le cycle est monocyclique ou annelé avec un autre cycle de 3 à 6 chaînons, saturé ou partiellement insaturé, qui est carbocyclique ou qui contient 1 ou 2 atomes de N, 0 ou 1 atome de N ou 1 atome d'O ou de S, 2 atomes d'O ou de S, 0 ou 1 atome de N et 1 atome d'O et 1 atome de S,
où le cycle annelé est non substitué ou substitué par 1 à 3, dans le cas d'halogène également jusqu'au nombre maximal possible, substituants du groupe formé par halogène, C₁-C₆-alkyle, C₁-C₆-halogénoalkyle et C₁-C₆-alcoxy,
et où le cycle est non ponté ou ponté avec une chaîne saturée ou insaturée de 1 à 3 chaînons, qui ne contient pas d'hétéroatomes ou qui contient 1 atome de N ou 0 ou 1 atome de N et 1 atome d'O ou 1 atome de S,
où le pont est non substitué ou substitué par 1 à 3, dans le cas d'halogène également jusqu'au nombre maximal possible, substituants du groupe formé par halogène, C₁-C₆-alkyle, hydroxy et C₁-C₆-alcoxy.

5. Procédé pour préparer des amides de sérine substitués par hétéroaroyle de formule (I) selon l'une quelconque des revendications 1 à 4, où des dérivés de sérine de formule (V), 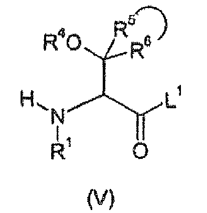 où R¹, R⁴, R⁵ et R⁶ ont les significations mentionnées dans la formule (I) et L¹ représente hydroxy ou C₁-C₆-alcoxy,
sont transformés avec (des dérivés) des acides hétéroaryliques de formule (IV), 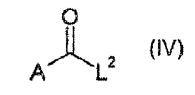 où A a la signification mentionnée dans la formule (I) et L² représente hydroxy, halogène, C₁-C₆-alkylcarbonyle, C₁-C₆-alcoxycarbonyle, C₁-C₄-alkylsulfonyle, phosphoryle ou iso-uréyle,
en dérivés d'hétéroaroyle correspondants de formule (III), 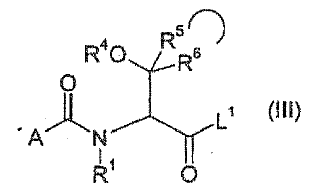 où A, R¹ R⁴, R⁵ et R⁶ ont les significations mentionnées dans la formule (I) et L¹ représente hydroxy ou C₁-C₆-alcoxy,
et les dérivés d'hétéroaroyle de formule (III) obtenus sont ensuite transformés avec une amine de formule (II)
HNR²R³ (II)
où R² et R³ ont les significations mentionnées dans la formule (I).

6. Dérivés d'hétéroaroyle de formule (III), 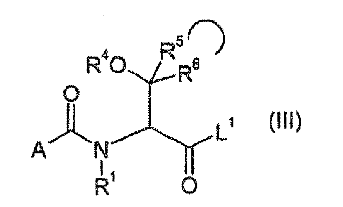 où A, R¹, R⁴, R⁵ et R⁶ ont les significations mentionnées dans la formule (I) dans la revendication 1 et L¹ représente un groupe partant pouvant être repoussé de manière nucléophile.

7. Agents, contenant une quantité active en tant qu'herbicide d'au moins un amide de sérine substitué par hétéroaroyle de formule (I) ou d'un sel utilisable en agriculture de (I) selon l'une quelconque des revendications 1 à 4 et des adjuvants usuels pour la formulation d'agents de phytoprotection.

8. Procédé pour la préparation d'agents selon la revendication 7, **caractérisé en qu'**on mélange une quantité active en tant qu'herbicide d'au moins un amide de sérine substitué par hétéroaroyle de formule (I) ou d'un sel utilisable en agriculture de (I) selon l'une quelconque des revendications 1 à 4 et des adjuvants usuels pour la formulation d'agents de phytoprotection.

9. Procédé pour lutter contre une croissance non souhaitée de plantes, **caractérisé en ce qu'**on laisse agir une quantité active en tant qu'herbicide d'au moins un amide de sérine substitué par hétéroaroyle de formule (I) ou d'un sel utilisable en agriculture de (I) selon l'une quelconque des revendications 1 à 4 sur des plantes, leur espace de vie et/ou sur des semences.

10. Utilisation des amides de sérine substitués par hétéroaroyle de formule (I) et de leurs sels utilisables en agriculture selon l'une quelconque des revendications 1 à 4 comme herbicides.
